# EUROPEAN PATENT APPLICATION

(11) **EP 4 008 785 A1**
(43) Date of publication of application: **08.06.2022**
(21) Application number: 20211415.3
(22) Date of filing: 03.12.2020
(51) Int. Cl.: C12N 15/113, A61K 31/7105

(54) **CIRCULAR NUCLEIC ACIDS AND USES THEREOF FOR INTERFERING WITH GENOME EXPRESSION AND PROLIFERATION OF CORONAVIRUSES**

(71) Applicant: Justus-Liebig-Universität Gießen, 35390 Gießen (DE)
(72) Inventor: BINDEREIF, Albrecht, 35394 Gießen (DE); MÜLLER, Christin, 35753 Greifenstein (DE); PFAFENROT, Christina, 35440 Linden (DE); SCHNEIDER, Tim, 35440 Linden (DE); ZIEBUHR, John, 35614 Aßlar (DE)
(74) Representative: Stumpf, Peter

(57) **Abstract**

The present invention relates to polynucleotides, preferably circular polynucleotides, pharmaceutical compositions and the use thereof in methods for modulating gene expression and/or inhibiting proliferation of pathogens. The polynucleotides comprise a nucleotide sequence, which sequence comprises a targeting region having a nucleotide sequence complementary to a 5'-UTR of a Coronavirus genome or to a portion thereof. In particular, the polynucleotides are capable of inhibiting proliferation of Coronaviruses.

## Description

### INTRODUCTION

The present invention relates to polynucleotides, *e.g.,* circular polynucleotides, compositions and the use thereof in methods for modulating gene expression and/or inhibiting proliferation of pathogens. The polynucleotides comprise a nucleotide sequence, which sequence comprises a targeting region having a nucleotide sequence complementary to a 5'-untranslated region of the genome of a Coronavirus or a portion thereof. In particular, the polynucleotides are capable of inhibiting proliferation of Coronaviruses.

### BACKGROUND OF THE INVENTION

Coronaviruses are positive-strand RNA viruses with large polycistronic genomes of around 30 kb, which have been extensively studied since the 2003 SARS outbreak (severe acute respiratory syndrome, reviewed by Perlman and Netland, Nat Rev Microbiol. 7 (2009): 439-450). Following receptor-mediated coronavirus entry into susceptible host cells, the two large open reading frames (ORFs), 1a and 1b, located in the 5'-terminal two-thirds of the capped and polyadenylated coronavirus genome are translated, resulting in two polyproteins that are processed by viral proteases to produce nonstructural proteins that direct viral RNA synthesis. Subsequently, the viral genome RNA serves as a template for negative-strand RNA synthesis. Two types of minus-strand RNAs are produced: first, full-length copies of the plus-strand RNA that are used as templates for the production of new genome RNAs, and second, a set of 5'-coterminal subgenomic (sg) minus-strand RNAs of varying length. The vast majority of sg minus-strands RNAs carry at their 3'-end an 'antileader' sequence, *i.e.,* a complement of the leader sequence located at the 5'-end of the genome, which they acquire in a process called 'discontinuous extension' of minus strands. The sg minus strands serve as templates for the production of a nested set of sg-mRNAs of varying length that share a common 5'-leader sequence. The unusual process of discontinuous (minus-strand) RNA synthesis is guided by pairing between complements of the conserved transcription regulatory sequences (TRS) located upstream of the various ORFs in the 3'-region of the genome ("body-TRS") and the TRS located immediately downstream of the 5' leader ("leader-TRS", reviewed by Sola I., et al., Annu Rev Virol. 2 (2015): 265-288, and studied by transcriptomics for SARS-CoV-2 by Kim D., et al., Cell 181 (2020): 914-921).

The 5'-UTRs of subgenomic mRNAs (sgRNAs) contain the 5'-leader (75 nucleotides in SARS-CoV-2) plus a few nucleotides upstream of the translation start codon of the first ORF in the respective mRNA. It is followed by ORFs encoding the viral structural and several accessory proteins. With only few exceptions, only the first ORF (that is not present on the next smaller mRNA of this nested set of mRNAs) is translated. The SARS-CoV-2 genome RNA contains a 5'-UTR comprising nucleotides 1 to 265 and serves as an mRNA for the translation of ORFs 1a and 1b, resulting in polyproteins (pp) 1a and 1ab, with production of pp1ab requiring a (-1) ribosomal frameshift upstream of the ORF1a stop codon (for secondary structure models of the 5'-UTR regions, see Madhugiri R., et al., Virology 517 (2018): 44-55; Miao Z., et al., RNA Biol 23 (2020): 1-10.

The current COVID-19 pandemic, with its dramatic worldwide impact on human health and economy, is caused by SARS-CoV-2, which emerged in late 2019 in China. The SARS-CoV-2 genome was sequenced in early 2020 (Zhou P., et al., Nature 579 (2020):270-273; Zhu N., et al., N Engl J Med. 382 (2020): 727-733), and currently there are intense worldwide efforts to develop and apply new therapeutic strategies to fight this life-threatening disease. Most of these approaches focus on, first, drugs targeting viral enzymes (nucleoside analogs, protease inhibitors and others), and second, on antibodies interfering with virus entry, in particular virus receptor interactions. In addition, immunomodulatory agents are being used and a large number of SARS-CoV-vaccines (including virus vector- and mRNA-based vaccines) are being developed and tested, many of which providing promising new approaches to prevent or treat COVID-19 more effectively.

Antisense approaches have been investigated for the last 30 years (for reviews, see Bennet and Swayze, 2010; Crooke et al. 2018; Bennet et al. 2019). By targeting specific RNA sequences, antisense approaches aim to modulate RNA structure and activity, mRNA splicing, translation, or stability. Best known examples are antisense oligonucleotides (ASO), such as locked nucleic acids (LNA), so-called gapmers, morpholino or other modifications that are introduced to increase RNA base-pairing and/or metabolic stability. As a result of antisense research over several decades, ASO-based therapies have been advanced to the stage of approved drugs used in certain genetic diseases.

Circular RNAs are a novel class of RNAs, which are generated from pre-mRNAs by circular splicing (also termed "back splicing") of one or several adjacent exons (Wilusz et al., 2018, Wiley Interdiscip Rev RNA 9(4):e1478). Circular RNAs are expressed in a cell-type specific manner, are much more stable than the corresponding linear mRNA, evolutionarily conserved, and are mostly cytoplasmic. Although present in all eukaryotes investigated so far, circular RNAs are still largely undefined in functional terms, except for few validated miRNA sponges (Hansen TB., et al., Nature 495 (2013): 384-388; Memczak S., et al., Nature 495 (2013): 333-338), which are embedded in regulatory RNA networks (Piwecka M., et al., Science 357 (2017); Kleaveland B., et al., Cell 174 (2018): 350-362).

Despite impressive benefits associated with antisense approaches, there is still a need for the development of improved antisense polynucleotides capable of modulating the gene expression and/or proliferation of pathogens, in particular of Coronaviruses.

### SUMMARY OF THE INVENTION

The present inventors surprisingly found that polynucleotides targeting a 5'-untranslated region (also referred to as 5'-UTR herein) of the genome or a subgenomic part of a Coronavirus, in particular polynucleotides targeting a stem-loop 2 and/or a stem-loop 3 within the 5'-UTR, are particularly suitable for improved antisense approaches. Preferably, the targeting region comprising or consisting of a nucleotide sequence complementary to the 5'-UTR or to a portion thereof is presented by a circular polypeptide, such as a circular RNA (also referred to as circRNA herein), thereby exploiting the unusual metabolic stability of circular RNAs.

In one aspect, the invention therefore relates to a polynucleotide comprising a nucleotide sequence, which sequence comprises a targeting region comprising or consisting of a nucleotide sequence complementary to a 5'-UTR of a Coronavirus or to a continuous or discontinuous portion of said 5'-UTR.

In one embodiment, the polynucleotide is a linear, preferably circular polynucleotide.

In one embodiment, the polynucleotide comprises at least one ribonucleotide. In one embodiment, the polynucleotide consists of ribonucleotides. In one embodiment, the polynucleotide comprises at least one deoxyribonucleotide. In one embodiment, the polynucleotide comprises a mixture of ribonucleotides and deoxyribonucleotides. For example, the polynucleotide of the present invention may be a DNA polynucleotide, a cDNA polynucleotide, an RNA polynucleotide or an DNA/RNA hybrid polynucleotide. A DNA/RNA hybrid polynucleotide comprises at least one deoxyribonucleotide and at least one ribonucleotide, *e.g.,* such a hybrid may comprise one or more portions being DNA and one or more portions being RNA. In a further example or preferred embodiment, the polynucleotide of the invention may be a circular DNA polynucleotide, a circular cDNA polynucleotide, a circular RNA polynucleotide or a circular DNA/RNA hybrid polynucleotide.

In one embodiment, the polynucleotide comprises at least one modified nucleotide. For example, a nucleotide may be modified with regard to the nucleobase and/or the sugar compound. Within the present invention such modifications may further comprise or be backbone modifications.

In one embodiment of the polynucleotide, the nucleotide sequence of the targeting region is complementary to at least one region of the 5'-UTR capable of forming a stem-loop. Such regions in the 5'-UTR may be capable of forming a secondary structure including a predominantly stem portion and a predominantly single-stranded loop portion, which is linked to the stem portion. In one embodiment, the region of the 5'-UTR capable of forming a stem-loop is selected from the group consisting of stem-loop 1 (SL1), stem-loop 2 (SL2) and stem-loop 3 (SL3). In one embodiment, the nucleotide sequence of the targeting region of the polynucleotide comprises or consists of a nucleotide sequence complementary to the SL2 and/or the SL3. In one embodiment, the nucleotide sequence of the targeting region of the polynucleotide does not comprise a nucleotide sequence complementary to the SL1. In one embodiment, the nucleotide sequence of the targeting region of the polynucleotide comprises or consists of a nucleotide sequence complementary to the SL1. For example, the nucleotide sequence of the targeting region may be complementary to the SL2 and/or the SL3, but may not comprise a nucleotide sequence complementary to (and thus targeting) the SL1. Nevertheless, such a nucleotide sequence may in addition comprise one or more nucleotide sequences complementary to the 5'-UTR portions flanking the SL1 5' and/or 3'.

In one embodiment, the nucleotide sequence of the 5'-untranslated region is depicted in SEQ ID NO: 1, which sequence shows the 5'-UTR of the SARS-CoV-2. In one embodiment, the 5'-UTR as a variant of the sequence depicted in SEQ ID NO: 1.

In one embodiment of the polynucleotide, the nucleotide sequence of the targeting region is complementary to nucleotides 1 to 85 of the sequence depicted in SEQ ID NO: 1 or to a continuous or discontinuous portion of said sequence. In one embodiment, the nucleotide sequence of the targeting region is complementary to nucleotides 1 to 75 of the sequence depicted in SEQ ID NO: 1 or to a continuous or discontinuous portion of said sequence. In one embodiment, the nucleotide sequence of the targeting region is complementary to nucleotides 247 to 265 of the sequence depicted in SEQ ID NO: 1 or to a continuous or discontinuous portion of said sequence.

In one embodiment of the polynucleotide, the nucleotide sequence of the targeting region is complementary to at least one continuous portion of the 5'-UTR, the continuous portion being selected from the group consisting of:
(i) nucleotides 1 to 75 of the sequence depicted in SEQ ID NO: 1;
(ii) nucleotides 1 to 65 of the sequence depicted in SEQ ID NO: 1;
(iii) nucleotides 1 to 40 of the sequence depicted in SEQ ID NO: 1;
(iv) nucleotides 36 to 75 of the sequence depicted in SEQ ID NO: 1;
(v) nucleotides 45 to 75 of the sequence depicted in SEQ ID NO: 1;
(vi) nucleotides 58 to 75 of the sequence depicted in SEQ ID NO: 1
(vii) nucleotides 61 to 75 of the sequence depicted in SEQ ID NO: 1;
(viii) nucleotides 70 to 75 of the sequence depicted in SEQ ID NO: 1;
(ix) nucleotides 45 to 59 of the sequence depicted in SEQ ID NO: 1;
(x) nucleotides 1 to 44 of the sequence depicted in SEQ ID NO: 1;
(xi) nucleotides 34 to 44 of the sequence depicted in SEQ ID NO: 1;
(xii) nucleotides 1 to 6 of the sequence depicted in SEQ ID NO: 1; and
(xiii) nucleotides 7 to 33 of the sequence depicted in SEQ ID NO: 1.

In one embodiment, the continuous portion of the 5'-UTR is variant of any one of these portions under (i) to (xiii).

The nucleotide sequence of the targeting region may comprise at least two segments, each segment being complementary to a nucleotide sequence of the 5'-UTR. The two segments can be continuous of each other or discontinuous if one or more nucleotides are comprised between the discontinuous segments. In one embodiment of the polynucleotide, the nucleotide sequence of the targeting region comprises at least two segments, each segment complementary to a nucleotide sequence of the 5'-UTR, wherein the complementary nucleotide sequences of the 5'-UTR are not continuous. For example, the nucleotide sequence of the targeting region may comprise two or three segments, each segment complementary to a nucleotide sequence of the 5'-UTR, wherein the complementary nucleotide sequences of the 5'-UTR are not continuous. In one embodiment, the first segment of the nucleotide sequence of the targeting region is complementary to nucleotides 1 to 6 and the second segment is complementary to nucleotides 34 to 44 of the sequence depicted in SEQ ID NO: 1. In one embodiment, the first segment of the nucleotide sequence of the targeting region is complementary to nucleotides 45 to 59 and the second segment is complementary to nucleotides 61 to 75 of the sequence depicted in SEQ ID NO: 1. In one embodiment, the first segment of the nucleotide sequence of the targeting region is complementary to nucleotides 36 to 59 and the second segment is complementary to nucleotides 61 to 75 of the sequence depicted in SEQ ID NO: 1. In one embodiment, the first segment of the nucleotide sequence of the targeting region is complementary to nucleotides 1 to 6 and the second segment is complementary to nucleotides 34 to 59 of the sequence depicted in SEQ ID NO: 1. In one embodiment, the first segment of the nucleotide sequence of the targeting region is complementary to nucleotides 1 to 6, the second segment is complementary to nucleotides 34 to 59 and the third segment is complementary to nucleotides 61 to 75 of the sequence depicted in SEQ ID NO: 1.

In one embodiment of the polynucleotide, the nucleotide sequence of the targeting region comprises one or more nucleotides between the at least two segments of a discontinuous portion of the 5'-UTR. In one embodiment, the one or more nucleotides between the two segments are two nucleotides. In one embodiment, the two nucleotides are two adenine nucleotides. In one embodiment, the nucleotide between the two segments is a single nucleotide.

In one embodiment of the polynucleotide, two nucleotide are inserted between (and linked to) the nucleotides of the first segment complementary to nucleotides 1 to 6 and the second segment complementary to nucleotides 34 to 44 of the sequence depicted in SEQ ID NO: 1. These inserted two nucleotides may be non-complementary to nucleotides 7 and 33, respectively, of the sequence depicted in SEQ ID NO: 1. For example, the inserted dinucleotide may be a dinucleotide of two adenine nucleotides.

In one embodiment of the polynucleotide, one nucleotide is inserted between (and linked to) the nucleotides of the first segment complementary to nucleotides 45 to 59 and the second segment complementary to nucleotides 61 to 75 of the sequence depicted in SEQ ID NO: 1. The inserted nucleotide may be complementary or non-complementary to nucleotide 60 of the sequence depicted in SEQ ID NO: 1.

In one embodiment of the polynucleotide, the nucleotide sequence of the targeting region is complementary to at least nucleotides 45 to 59 of the sequence depicted in SEQ ID NO: 1. The nucleotides 45 to 59 of the sequence depicted in SEQ ID NO: 1 define a SL2 of SARS-CoV-2. In one embodiment, the nucleotide sequence of the targeting region of the polynucleotide is complementary to at least nucleotides 61 to 75 of the sequence depicted in SEQ ID NO: 1. The nucleotides 45 to 59 of the sequence depicted in SEQ ID NO: 1 define a SL3 of SARS-CoV-2.

For increasing specificity, efficiency and/or flexibility of the polynucleotide, in one embodiment, the targeting region of the polynucleotide may be flanked on one or both ends by a flanking region. Each flanking region may comprise one or more additional nucleotides, wherein each additional nucleotide may be complementary or non-complementary to the corresponding nucleotide of the nucleotides flanking the portion of the 5'-UTR to which the nucleotide sequence of the targeting region is complementary. Flanking on both ends means 3' and 5', relative to the 3' terminal nucleotide or the 5' terminal nucleotide, respectively, of the nucleotide sequence of the targeting region.

In one embodiment of the polynucleotide, the nucleotide sequence of the targeting region complementary to at least nucleotides 45 to 59 of the sequence depicted in SEQ ID NO: 1 is flanked 3' by one or more additional nucleotides for targeting the nucleotide at position 44 up to at most to the nucleotide at position 1 of the sequence depicted in SEQ ID NO: 1. Each nucleotide added 3' may be complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 45 to 59 of the sequence depicted in SEQ ID NO: 1 is flanked 3' by one or more additional nucleotides for targeting the nucleotide at position 44 up to at least to the nucleotide at position 34 of the sequence depicted in SEQ ID NO: 1. Each nucleotide added 3' may be complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and one or more nucleotides at position 6 up to at most the nucleotide at position 1 of the sequence depicted in SEQ ID NO: 1. Each nucleotide added 3' may be complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 2 of the sequence depicted in SEQ ID NO: 1.

Each nucleotide added 3' may be complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 3 of the sequence depicted in SEQ ID NO: 1. Each nucleotide added 3' may be complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 4 of the sequence depicted in SEQ ID NO: 1. Each nucleotide added 3' may be complementary or non-complementary to the corresponding nucleotide of the nucleotides flanking the portion of the 5'-UTR to which the nucleotide sequence of the targeting region is complementary. In one embodiment, the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 5 of the sequence depicted in SEQ ID NO: 1. Each nucleotide added 3' may be complementary or non-complementary to the corresponding nucleotide of the nucleotides flanking the portion of the 5'-UTR to which the nucleotide sequence of the targeting region is complementary. In one embodiment, a non-complementary sequence of two or more nucleotides is inserted between the nucleotides for targeting the nucleotides at positions 44 to 34 and the nucleotides for targeting the nucleotides at position 6 up to at most the nucleotide at position 1 of the sequence depicted in SEQ ID NO: 1. These two or more nucleotides are to be inserted for bridging a SL1. Nucleotides 7 to 33 of the sequence depicted in SEQ ID NO: 1 define a SL1 of SARS-CoV-2. In one embodiment, two nucleotides are inserted in between the said nucleotide sequences. In one embodiment, two adenine nucleotides are inserted. In one embodiment, an oligonucleotide, *e.g.,* an oligonucleotide being composed of 3, 4, 5, 6, 7, 8, 9, 10, or more nucleotides, is inserted. The nucleotide sequence of the said inserted oligonucleotide is non-complementary to the SL1 or a portion thereof.

In one embodiment of the polynucleotide, the nucleotide sequence of the targeting region is depicted in any one of SEQ ID NO: 2 to SEQ ID: 9. In one embodiment, the nucleotide sequence of the targeting region of the polynucleotide is a variant of any of the sequences depicted in any one of SEQ ID NO: 2 to SEQ ID: 9.

In one embodiment of the polynucleotide, the nucleotide sequence of the targeting region complementary to at least nucleotides 61 to 75 of the sequence depicted in SEQ ID NO: 1 is flanked 3' by one or more additional nucleotides for targeting the nucleotide at position 60 up to at most to the nucleotide at position 45 of the sequence depicted in SEQ ID NO: 1. In one embodiment, the nucleotide sequence of the targeting region complementary to at least nucleotides 61 to 75 of the sequence depicted in SEQ ID NO: 1 is flanked 3' by one or more additional nucleotides for targeting the nucleotides at position 60 to 58 of the sequence depicted in SEQ ID NO: 1.

In one embodiment of the polynucleotide, the nucleotide sequence of the targeting region complementary to at least nucleotides 45 to 59 of the sequence depicted in SEQ ID NO: 1 is flanked 5' by one or more additional nucleotides for targeting the nucleotide at position 60 up to at most to the nucleotide at position 85 of the sequence depicted in SEQ ID NO: 1. In one embodiment, the nucleotide sequence of the targeting region complementary to at least nucleotides 45 to 59 of the sequence depicted in SEQ ID NO: 1 is flanked 5' by one or more additional nucleotides for targeting the nucleotide at position 60 up to at most to the nucleotide at position 75 of the sequence depicted in SEQ ID NO: 1. Each nucleotide added 5' may be complementary or non-complementary to the corresponding nucleotide of the nucleotides flanking the portion of the 5'-UTR to which the nucleotide sequence of the targeting region is complementary. In one embodiment, the nucleotide sequence of the targeting region is depicted in any one of SEQ ID NO: 10 to SEQ ID: 20. In one embodiment, the nucleotide sequence of the targeting region may be a variant of any of the sequences depicted in any one of SEQ ID NO: 10 to SEQ ID: 20.

In one embodiment of the polynucleotide, the targeting region may comprise or consist of a nucleotide sequence being any combination between the above mentioned 5' elongations, for example as depicted in any one of SEQ ID NO: 10 to 20, and the above mentioned 3' elongations, for example as depicted in any one of SEQ ID NO: 3 to 9, including variants of these combined sequences. Preferred embodiments encompass any combinations between the mentioned 5' elongations according to any one of SEQ ID NO: 10 to SEQ ID NO: 20 and the mentioned 3' elongations according to any one of SEQ ID NO: 3 to SEQ ID NO: 9, or respective variants thereof. For flanking the nucleotide sequence complementary to the SL2 (SEQ ID NO: 2), for example, the nucleotide sequence for 5' elongation comprised in SEQ ID NO: 10 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 11 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 12 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 13 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 14 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 15 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 16 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 17 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 18 as shown in Table 4, below, can be combined with any one of the nucleotide sequences for 3' elongation of the targeting region as shown for SEQ ID NO: 3 to 9 in Table 2 or 3, below. In another example, the nucleotide sequence for 5' elongation comprised in SEQ ID NO: 19 as shown in Table 4, below, can be combined with any one of the nucleotide sequences for 3' elongation of the targeting region as shown for SEQ ID NO: 3 to 9 in Table 2 or 3, below. In a still further example, the nucleotide sequence for 5' elongation comprised in SEQ ID NO: 20 as shown in Table 4, below, can be combined with any one of the nucleotide sequences for 3' elongation of the targeting region as shown for SEQ ID NO: 3 to 9 in Table 2 or 3, below.

Alternatively, or in addition to the above embodiments of the polynucleotide, in a further embodiment the targeting region, being it already elongated or not, is flanked on one or both ends by a flanking linker, wherein each linker nucleotide sequence may be independently at least 1, at least 2 or at least 3 nucleotides in length, and/or may be independently at most 20, at most 15, at most 12, at most 10 or at most 8 nucleotides in length. For example, each linker nucleotide sequence may be 1 to 20 or 2 to 10 nucleotides in length. The linker nucleotide sequence may be a polyA sequence, *i.e.,* a nucleotide sequence composed of adenine nucleotides or modified adenine nucleotides.

In one embodiment of the polynucleotide, the nucleotide sequence of the targeting region is complementary to nucleotides 1 to 40 of the sequence depicted in SEQ ID NO: 1. In one embodiment, the nucleotide sequence of the targeting region is depicted in SEQ ID NO: 24. In one embodiment, the nucleotide sequence of the targeting region of the polynucleotide is complementary to nucleotides 1 to 6 and 34 to 65 of the sequence depicted in SEQ ID NO: 1. In one embodiment, the nucleotide sequence of the targeting region is depicted in SEQ ID NO: 21. In one embodiment, the nucleotide sequence of the targeting region of the polynucleotide is complementary to nucleotides 1 to 6 and 34 to 75 of the sequence depicted in SEQ ID NO: 1. In one embodiment, the nucleotide sequence of the targeting region is depicted in SEQ ID NO: 22. In one embodiment, the nucleotide sequence of the targeting region of the polynucleotide is complementary to nucleotides 36 to 75 of the sequence depicted in SEQ ID NO: 1. In one embodiment, the nucleotide sequence of the targeting region is depicted in SEQ ID NO: 23. In one embodiment, the nucleotide sequence of the targeting region of the polynucleotide is complementary to nucleotides 58 to 75 of the sequence depicted in SEQ ID NO: 1. In one embodiment, the nucleotide sequence of the targeting region is depicted in SEQ ID NO: 26. In one embodiment, the nucleotide sequence of the targeting region is depicted in SEQ ID NO: 25. In one embodiment, the nucleotide sequence of the targeting region of the polynucleotide has at least 70%, at least 75%, at least 80%, at least 85%, at least 90%, at least 95%, at least 97%, or at least 99% identity to the sequence depicted in SEQ ID NO: 21 to 26, respectively.

In one embodiment of the polynucleotide, the nucleotide sequence of the targeting region is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% complementary to the 5'-UTR or to a continuous or discontinuous portion thereof. In one embodiment, the nucleotide sequence of the targeting region is fully complementary, *i.e.,* 100% complementary, to the 5'-UTR or to a continuous or discontinuous portion thereof.

In one embodiment of the polynucleotide, the nucleotide sequence of the targeting region is at least 15 nucleotides, at least 20 nucleotides, at least 25 nucleotides, at least 30 nucleotides, at least 35 nucleotides, at least 40 nucleotides, or at least 45 nucleotides in length. In one embodiment, the nucleotide sequence of the targeting region is at most 300 nucleotides, at most 265 nucleotides, at most 250 nucleotides, at most 200 nucleotides, at most 150 nucleotides, at most 125 nucleotides, at most 100 nucleotides, at most 90 nucleotides, at most 80 nucleotides, at most 70 nucleotides, or at most 60 nucleotides in length. For example, the nucleotide sequence of the targeting region may be 15 to 300 or may be 20 to 250 or may be 15 to 265 nucleotides in length. As will be easily understood by a person skilled in the art any of the lower limits disclosed can be combined with any of the upper limits mentioned.

In one embodiment, the polynucleotide of the invention comprises in addition to (i) the targeting region, preferably a targeting region as specified above, (ii) a region comprising or consisting of a nucleotide sequence capable of forming a stem-loop, which region is linked to the targeting region of (i), and (iii) two linker nucleotide sequences flanking the nucleotide sequence of the targeting region at both sides, *i.e.,* 5' and 3'. That is, when the polynucleotide is in a circularized form, the targeting region is on both sides separated from region capable of forming a stem-loop of (ii) by the one linker sequence. The linkers allow a more flexible presentation of the targeting region. In one embodiment, the nucleotide sequence capable of forming a stem-loop comprises at least 2, at least 3, at least 4, at least 5 or at least 6, and/or at most 15, at most 14, at most 13, at most 12, at most 11, at most 10, at most 9 or at most 8 perfect base-pairs with two overhanging ends. For example, the nucleotide sequence capable of forming a stem-loop may comprise 4 to 10 or 5 to 8 perfect base-pairs. As will be easily understood by a person skilled in the art any of the lower limits disclosed can be combined with any of the upper limits mentioned. The overhanging ends may be each at least 2, at least 3, at least 4, at least 5, at least 6, at least 7, at least 8, at least 9 or at least 10 nucleotides in length, and/or the overhanging ends may be each at most 40, at most 35, at most 30, at most 20 or at most 15 nucleotides in length. For example, the overhanging ends may be each 2 to 30 or 3 to 20 nucleotides in length. As will be easily understood by a person skilled in the art any of the lower limits disclosed can be combined with any of the upper limits mentioned. In one embodiment, each linker nucleotide sequence may be independently at least 1, at least 2 or at least 3 nucleotides in length, and/or may be independently at most 20, at most 15, at most 12, at most 10 or at most 8 nucleotides in length. For example, each linker may be 1 to 20 or 2 to 10 nucleotides in length. As will be easily understood by a person skilled in the art any of the lower limits disclosed can be combined with any of the upper limits mentioned. In one embodiment, the nucleotide sequence of the polynucleotide is depicted in SEQ ID NO: 27 to 32, respectively. In one embodiment, the nucleotide sequence of the polynucleotide is a variant of any of the sequences depicted in SEQ ID NO: 27 to 32, respectively.

In one embodiment, the entire length of the polynucleotide is at least 30 nucleotides, at least 35 nucleotides, at least 40 nucleotides, at least 45 nucleotides, at least 50 nucleotides, at least 55 nucleotides, or at least 60 nucleotides in length. In one embodiment, the nucleotide sequence of the targeting region is at most 500 nucleotides, at most 400 nucleotides, at most 300 nucleotides, at most 200 nucleotides, at most 150 nucleotides, at most 125 nucleotides, at most 100 nucleotides, at most 90 nucleotides, or at most 80 nucleotides in length. For example, the nucleotide sequence of the targeting region may be 30 to 500 or may be 35 to 300 in length. As will be easily understood by a person skilled in the art any of the lower limits disclosed can be combined with any of the upper limits mentioned.

In a further aspect, the invention relates to a pharmaceutical composition comprising one or more of the polynucleotides of the invention, and at least one pharmaceutically acceptable carrier. The pharmaceutical composition may also comprise one or more excipients and/or one more diluents.

In one embodiment, the composition is an aqueous composition. The aqueous composition may optionally comprise solutes, *e.g.,* salts. In one embodiment, the composition is in the form of a freeze-dried composition. A freeze-dried composition is obtainable by freeze-drying a respective aqueous composition. In one embodiment, the composition is an injectable composition, optionally comprising sodium, potassium and calcium salts, such as Ringer's solution or Ringer's Lactate.

In one embodiment of the pharmaceutical composition, the polynucleotide, *e.g.,* the circular RNA molecule, to be administered is naked, *i.e.,* not complexed with any kind of delivery material or proteins. In one embodiment of the pharmaceutical composition, the polynucleotide, *e.g.,* the RNA molecule, to be administered is formulated in a delivery vehicle. In one embodiment, the delivery vehicle comprises particles. In one embodiment, the delivery vehicle comprises a lipid. In one embodiment, the lipid comprises a cationic lipid. In one embodiment, the lipid forms a complex with and/or encapsulates the polynucleotide, *e.g.,* the RNA molecule. In one embodiment, the polynucleotide, *e.g.,* the RNA molecule, is formulated in liposomes or as lipoplex particles.

The pharmaceutical composition according to the present invention is generally to be applied in a "pharmaceutically effective amount" and in "a pharmaceutically acceptable preparation".

In one embodiment of the pharmaceutical composition, the one or more polynucleotides of the present invention is/are comprised in the pharmaceutical composition in an amount of at least 20 ng, of at least 50 ng, of at least 100 ng, of at least 200 ng, of at least 500 ng, of at least 1,000 ng or of at least 2,000 ng, and/or at most 20 mg, at most 15 mg, at most 10 mg, at most 9 mg, at most 8 mg, at most 7 mg, or at most 6 mg. For example, the one or more polynucleotides may be comprised in an amount of 20 ng to 20 mg or in an amount of 50 ng to 15 mg. As will be easily understood by a person skilled in the art any of the lower limits disclosed can be combined with any of the upper limits mentioned.

In one embodiment of the pharmaceutical composition, the one or more polynucleotides of the present invention is/are comprised in the pharmaceutical composition in a concentration of at least 1 nM, of at least 2 nM, of at least 3 nM, of at least 4 nM, of at least 5 nM, of at least 10 nM, of at least 15 nM, or of at least 20 nM, and/or at most 100 nM, at most 90 nM, at most 80 nM, at most 70 nM, at most 60 nM, at most 50 nM, at most 45 nM, or at most 40 nM. For example, the one or more polynucleotides may be comprised in a concentration of 1 nM to 100 nM or in a concentration of 5 nM to 50 nM As will be easily understood by a person skilled in the art any of the lower limits disclosed can be combined with any of the upper limits mentioned.

In one embodiment, the pharmaceutical composition is formulated to be suitable for local or systemic administration. For example, the pharmaceutical composition can be formulated to be suitable for a parenteral administration.

The pharmaceutical composition of the invention can be used in the field of medicine. Thus, in a further aspect, the invention relates to a pharmaceutical composition of the invention for use as a medicament.

The polynucleotides or the pharmaceutical composition of the invention may be used in a method of treating or preventing an infection with a pathogen. The pathogen may be a virus, e.g., an RNA virus, such as a Coronavirus. The Coronavirus may be SARS-CoV-2.

In a further aspect, the invention relates to a polynucleotide of the invention or a pharmaceutical composition of the invention for use in a method of treating or preventing a Coronavirus infection, said method comprising a step of administering to a patient in need thereof one or more of the polynucleotides or the pharmaceutical composition.

In another aspect, the invention relates to a method of treating or preventing a Coronavirus infection comprising administering to a patient in need thereof one or more of the polynucleotides of the invention or the pharmaceutical composition of the invention.

In one embodiment of these two aspects, (*i.e.,* the medical use and the method of treatment), the one or more polynucleotides or the pharmaceutical composition is administered locally or systemically. In one embodiment, the one or more polynucleotides or the pharmaceutical composition is/are administered parenterally.

In one embodiment of these two aspects, the one or more polynucleotides or the pharmaceutical composition is/are administered alone or in combination with any other therapeutic agent. In one embodiment, the other therapeutic agent is selected from a vaccine, an antiviral drug, a protease inhibitor, lipids or liposomes, or any combination thereof.

In one embodiment of these two aspects, the Coronavirus may be SARS-CoV-2, and/or the patient to be treated may be a human.

In one embodiment of these two aspects, the polynucleotide(s) of the invention or the pharmaceutical composition of the invention is/are applied in a pharmaceutically effective amount and/or in a pharmaceutically acceptable preparation. In one embodiment, the one or more polynucleotides of the present invention are administered per treatment in an amount of at least 20 ng, of at least 50 ng, of at least 100 ng, of at least 200 ng, of at least 500 ng, of at least 1,000 ng or of at least 2,000 ng, and/or at most 20 mg, at most 15 mg, at most 10 mg, at most 9 mg, at most 8 mg, at most 7 mg, or at most 6 mg. For example, the one or more polynucleotides may be administered in an amount of 20 ng to 20 mg per treatment or in an amount of 50 ng to 15 mg per treatment. As will be easily understood by a person skilled in the art any of the lower limits disclosed can be combined with any of the upper limits mentioned. In one embodiment, the one or more polynucleotides of the present invention are administered per treatment with a concentration of at least 1 nM, of at least 2 nM, of at least 3 nM, of at least 4 nM, of at least 5 nM, of at least 10 nM, of at least 15 nM, or of at least 20 nM, and/or at most 100 nM, at most 90 nM, at most 80 nM, at most 70 nM, at most 60 nM, at most 50 nM, at most 45 nM, or at most 40 nM. For example, the one or more polynucleotides may be administered in a concentration of 1 nM to 100 nM or in a concentration of 5 nM to 50 nM. As will be easily understood by a person skilled in the art any of the lower limits disclosed can be combined with any of the upper limits mentioned.

In a further aspect, the invention relates to a method for targeting a nucleic acid comprising contacting the nucleic acid to be targeted with a polynucleotide of the invention. In one embodiment, the nucleic acid to be targeted is DNA, *e.g.,* a portion of a DNA genome or cDNA. In one embodiment, the nucleic acid to be targeted is RNA. The RNA to be targeted may be a portion of an RNA genome, *e.g.,* of a genome of an RNA virus, or may be an RNA derived from a DNA or RNA genome, *e.g.,* an mRNA. For example, the nucleic acid to be targeted may be the genome of a Coronavirus or may be an mRNA (sgRNA) derived from that genome. In one embodiment, the nucleic acid to be targeted is at least partially complementary to the nucleotide sequence of the targeting region of the polynucleotide. In one embodiment, the nucleic acid to be targeted comprises at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence complementarity to the nucleotide sequence of the targeting region of the polynucleotide.

In a further aspect, the invention relates to a method for forming a complex comprising a polynucleotide of the invention, and a single-stranded nucleic acid. The method comprises a step of contacting the polynucleotide with the single-stranded nucleic acid. In one embodiment, the single-stranded nucleic acid is a nucleic acid comprising or consisting of deoxyribonucleotides. For example, the nucleic acid may be a portion of a DNA genome or a cDNA. In one embodiment, the single-stranded nucleic acid is a nucleic acid comprising or consisting of ribonucleotides. In one embodiment, the single-stranded nucleic acid may be the genome or an mRNA of an RNA virus. The RNA virus may be a Coronavirus, such as SARS-CoV-2. In one embodiment, the single-stranded nucleic acid is at least partially complementary to the nucleotide sequence of the targeting region of the polynucleotide. In one embodiment, the single-stranded nucleic acid comprises at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence complementarity to the nucleotide sequence of the targeting region of the polynucleotide.

In a further aspect, the invention relates to a method for interfering with genome expression and/or proliferation of a Coronavirus. The method comprises a step of contacting a polynucleotide of the invention with the nucleic acid of the pathogen. The pathogen may be virus comprising a 5'-UTR. In one embodiment, the Coronavirus may be SARS-CoV-2.

In one embodiment of the methods of the invention, the respective method is an *in vitro* or an *in vivo* method.

In one embodiment of all aspects of the invention, the pathogen or the Coronavirus is SARS-CoV-2.

Other features and advantages of the instant invention will be apparent from the following detailed description and claims.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., (1995) Helvetica Chimica Acta, CH-4010 Basel, Switzerland.

The practice of the present invention will employ, unless otherwise indicated, conventional methods of biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, *i.e.,* the subject-matter consists in the inclusion of a stated member, integer or step or group of members, integers or steps. Thus, the term "comprise", and variations such as "comprises" and "comprising", includes "to comprise at least one", so may be in some embodiments "consisting of". The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (*e.g.,* "such as"), provided herein is intended merely to better illustrate the invention and does not pose a limitation on the scope of the invention otherwise claimed. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether *supra* or *infra*, are hereby incorporated by reference in their entirety. Nothing herein is to be construed as an admission that the invention is not entitled to antedate such disclosure by virtue of prior invention.

Terms such as "reducing" or "inhibiting" relate to the ability to cause an overall decrease, preferably of 5% or greater, 10% or greater, 20% or greater, more preferably of 50% or greater, and most preferably of 75% or greater, in the level. The term "inhibit" or similar phrases can include a complete or essentially complete inhibition, i.e. a reduction to zero or essentially to zero.

Terms such as "increasing", "enhancing", "promoting" or "prolonging" preferably relate to an increase, enhancement, promotion or prolongation by about at least 10%, preferably at least 20%, preferably at least 30%, preferably at least 40%, preferably at least 50%, preferably at least 80%, preferably at least 100%, preferably at least 200% and in particular at least 300%. These terms may also relate to an increase, enhancement, promotion or prolongation from zero or a non-measurable or non-detectable level to a level of more than zero or a level which is measurable or detectable.

The terms "polynucleotide" and "nucleic acid", used interchangeably herein, refer to a polymeric form of nucleotides of any length, either ribonucleotides or/and deoxyribonucleotides. Thus, this term includes, but is not limited to, single-, or double-stranded DNA or RNA, genomic DNA, cDNA, DNA-RNA hybrids, or a polymer comprising purine and pyrimidine bases or other natural, chemically or biochemically modified, non-natural, or derivatized nucleotides. In a polynucleotide preferably the 5' end of one nucleotide and the 3' end of another nucleotide being linked to another. A polynucleotide typically varies in length and the term as used herein is, unless specified otherwise, not intended to be limited to a certain length. Polynucleotides can typically vary in length form 15 to several hundred nucleotides, more typically about 20-300 nucleotides in length or shorter, even more typically about 30-200 nucleotides in length.

As disclosed herein, the terms "nucleotide sequence" and "nucleic acid sequence", used interchangeably herein, refer to the sequence of nucleotides in a polynucleotide, *e.g.,* a ribonucleic acid (RNA) or a deoxyribonucleic acid (DNA). The term may refer to an entire nucleic acid molecule (such as to the single strand of an entire nucleic acid molecule) or to a portion (*e.g.,* a fragment) thereof.

By convention, sequences disclosed herein are set forth in the direction 5' to 3' unless other specified. Moreover, a strand containing the sequence of a SEQ ID NO has that sequence from 5' to 3' unless otherwise specified.

The term "3' ", when used directionally, generally refers to a region or position in a polynucleotide or oligonucleotide 3' (toward the 3' end of the nucleotide) from another region or position in the same polynucleotide or oligonucleotide. The term "3' end" generally refers to the 3' terminal nucleotide of the component oligonucleotides.

The term "5' ", when used directionally, generally refers to a region or position in a polynucleotide or oligonucleotide 5' (toward the 5' end of the nucleotide) from another region or position in the same polynucleotide or oligonucleotide. As used herein, the term "5' end" generally refers to the 5' terminal nucleotide of the component polynucleotide.

All sequences disclosed herein are shown as DNA sequences. A person skilled in the art is well aware of the fact that in respective RNA sequences T/t residues of the DNA sequence are to be replaced with U/u residues.

A polynucleotide of the invention comprises a nucleotide sequence, which sequence comprises a targeting region comprising or consisting of a nucleotide sequence complementary to a 5' -UTR of a Coronavirus genome or to a continuous or discontinuous portion of said 5'-UTR.

The targeting region has a nucleotide sequence that is capable of hybridizing or hybridizes to a corresponding portion of a target nucleic acid, *e.g.,* the 5'-UTR. The targeting region is preferably a single-stranded nucleic acid.

A nucleic acid is "capable of hybridizing" or "hybridizes" to another nucleic acid if the two sequences are complementary with one another. A nucleic acid is "complementary" to another nucleic acid if the two sequences are capable of forming a stable duplex with one another under the appropriate *in vitro* and/or *in vivo* conditions of temperature and solution ionic strength. As is known in the art, standard Watson-Crick base-pairing includes: adenine (A) pairing with thymidine (T), adenine (A) pairing with uracil (U), and guanine (G) pairing with cytosine (C).

Hybridization and washing conditions are also well known. The conditions of temperature and ionic strength determine the "stringency" of the hybridization. Hybridization requires that the two nucleic acids contain complementary sequences, although mismatches between bases are possible. According to the invention, hybridization is preferably carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York.

A person skilled in the art is well aware of the fact that nucleotide sequences need not be 100% complementary to that of its target nucleic acid to be specifically hybridizable or hybridizable. Moreover, a polynucleotide may hybridize over one or more segments such that intervening or adjacent segments are not involved in the hybridization event (*e.g.,* a loop structure or hairpin structure). A nucleotide sequence of a targeting region can comprise at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence complementarity to the nucleotide sequence of a target region, *e.g*., the 5'UTR or a portion thereof. For example, 5, 6, 7, 8, 9, 10 nucleotides out of 10 nucleotides being 50%, 60%, 70%, 80%, 90%, and 100% complementary. If the complementary is not 100%, the remaining non-complementary nucleotides may be clustered or interspersed with complementary nucleotides and need not be contiguous to each other or to complementary nucleotides. "Fully complementary" or "100% complementary" means that all the contiguous residues of a nucleotide sequence will hydrogen bond with the same number of contiguous residues in a second nucleotide acid sequence. Preferably, the degree of complementarity according to the invention is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%. Most preferably, the degree of complementarity according to the invention is 100%.

Methods of alignment of sequences for comparison are well-known in the art. Thus, the determination of percent identity between any two sequences can be accomplished using a mathematical algorithm. Sequence comparisons between two nucleotide sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Needleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

"Targeting" or "targeted" means the process of design and selection of a nucleotide sequence that will hybridize, preferably specifically hybridize, to a target sequence, for example a target RNA, such as a 5'UTR portion. A "target sequence", "target nucleic acid" or "target RNA" or "target DNA" all refer to a nucleotide sequence to which a targeting region is targeted.

The polynucleotides of the invention are for targeting a 5'-UTR of a Coronavirus or for targeting a continuous or discontinuous portion of said 5'-UTR. Thus, the 5'-UTR is, *e.g.,* a target sequence.

The term "portion", which is used herein interchangeably with the terms "part" and "fragment", refers to a continuous or discontinuous fraction of a structure. With respect to a particular structure such as an amino acid sequence or protein or a nucleotide sequence the terms "part", "fragment" and "portion" thereof may designate a continuous or a discontinuous fraction of said structure. Preferably, a "part", "fragment" and "portion" of a structure such as an amino acid sequence or a nucleotide sequence preferably comprises, preferably consists of at least 2%, at least 4%, at least 10%, at least 20%, at least 30%, at least 40%, at least 50%, at least 60%, at least 70%, at least 80%, at least 85%, at least 90%, at least 92%, at least 94%, at least 96%, at least 98%, at least 99% of the entire structure or amino acid sequence or nucleotide sequence. A portion, a part or a fragment of a structure preferably comprises one or more functional or structural properties of said structure. For example, a portion, a part or a fragment may be capable of forming a stem-loop in secondary structure. If the portion, part or fragment is a discontinuous fraction said discontinuous fraction is preferably composed of 2, 3, 4, 5, 6, 7, 8, or more parts or segments of a structure, each of these parts or segments being a continuous element of the structure. For example, a discontinuous fraction of a nucleotide sequence may be composed of 2, 3, 4, 5, 6, 7, 8, or more, preferably not more than 4 parts of said nucleotide sequence, wherein each part or segment preferably comprises at least 5 continuous nucleotides, preferably at least 10 continuous nucleotides, more preferably at least 12 continuous nucleotides, more preferably at least 15 continuous nucleotides of the nucleotide sequence.

The terms "continuous" and "contiguous" may be used interchangeably herein and refer preferably to an uninterrupted extension on the nucleotide sequence, *e.g.,* of the 5'-UTR. Two segments are interrupted, if one or more nucleotides are inserted between two segments, *e*.*g*., a first segment and a second segment, thereby connecting these segments, which inserted nucleotides may be any nucleotides and are preferably nucleotides different in number and/or in chemical structure from the respective nucleotides present in the uninterrupted starting sequence. Furthermore, the terms first, second, third and the like in the description and in the claims, are used for distinguishing between similar elements and not necessarily for describing a sequential or chronological order, unless the context indicates otherwise.

A portion of a 5-UTR target sequence may, for example, be a nucleotide sequence of a stem-loop or a subregion of such a stem-loop. A portion of a target sequence may also be sequence which located 5' or 3' to a stem-loop.

A "stem-loop " as used herein, preferably refers to a nucleotide sequence capable of forming a secondary structure that includes a region of nucleotides which are known or predicted to form a double strand (stem portion) that is linked on one side by a region of predominantly single-stranded nucleotides (loop portion). Such structures are well known in the art and these terms are used consistently with their known meanings in the art. As is known in the art, a stem-loop structure does not require exact base-pairing. Thus, the stem may include one or more base mismatches. Alternatively, the base- pairing may be exact, i.e. not include any mismatches.

In one embodiment, the 5'-UTR is the 5'-UTR of the Coronavirus SARS-CoV-2, which comprises nucleotides 1 to 265 of the sequence set forth in NCBI Reference Sequence: NC_045512.2 or a variant of this sequence. The nucleotide sequence of the 5'-UTR is incorporated herein as SEQ ID NO: 1 and the whole genome sequence of the NCBI Reference Sequence is incorporated herein as SEQ ID NO: 35.

The 5'-UTR comprises regions which may in secondary structure from stem loops, wherein for SARS-CoV-2 stem loop 1 (SL1) comprises nucleotides 7 to 33 of the sequences depicted in SEQ ID NO: 1, stem loop 2 (SL2) comprises nucleotides 45 to 59 of the sequences depicted in SEQ ID NO: 1, and stem loop 3 (SL3) comprises nucleotides 61 to 75 of the sequences depicted in SEQ ID NO: 1. The SL3 comprises a region referred to as TRS element comprising nucleotides 70 to 75 of the sequences depicted in SEQ ID NO: 1. The SL1 can be flanked 5' by a sequence, which sequence preferably comprises nucleotides 1 to 6 of the sequence depicted in SEQ ID NO: 1 or is a variant of this sequence. The SL1 and the SL2 can be interspaced by a sequence, which sequence preferably comprises nucleotides 34 to 44 of the sequence depicted in SEQ ID NO: 1 or is a variant of this sequence. The SL2 and the SL3 can be bridged by at least one nucleotide (nucleotide position 66 of the sequence depicted in SEQ ID NO: 1), which nucleotide may contain the nucleobase thymine or uracil, respectively. The SL3 can be flanked 3' by a sequence, which sequence preferably starts with nucleotide 76 of the sequence depicted in SEQ ID NO: 1 or is a variant of this sequence.

As used herein, any reference to a particular nucleotide position in a target sequence should not be construed to that extent that in a corresponding target sequence of another species or subspecies or variant, *e.g.,* in another Coronavirus or another variant of a Coronavirus, the corresponding nucleotide must be present at the same nucleotide position, as insertions or deletions of nucleotides may occur. That is, the corresponding nucleotide positions may vary and can, for example, be determined after performing a sequence alignment of the sequences in questions. Thus, when reference is made herein to a "nucleotide position", *e.g.,* "a nucleotide position of the sequence depicted in SEQ ID NO: 1", or to "a portion comprising nucleotides x to y", *e.g.,* "a portion comprises nucleotides x to y of the sequence depicted in SEQ ID NO: 1", these phrases are intended to comprise also the corresponding nucleotide position or the corresponding portion in another sequence, *e.g.,* in another target sequence.

The target sequence can be the complete 5'-UTR, for example a 5'-UTR having or comprising a sequence depicted in SEQ ID NO: 1 or can be a variant of this depicted nucleotide sequence. Alternatively, the 5'-UTR can be divided into portions and the target sequence comprises one or more of these portions or one or more segments of these portions.

The 5'-UTR can be divided into the following portions:
- a portion comprises nucleotides 1 to 85 of the sequence depicted in SEQ ID NO: 1 or a variant thereof;
- a portion comprises nucleotides 1 to 75 of the sequence depicted in SEQ ID NO: 1 or a variant thereof;
- a portion comprises nucleotides 1 to 65 of the sequence depicted in SEQ ID NO: 1 or a variant thereof;
- a portion comprises nucleotides 1 to 40 of the sequence depicted in SEQ ID NO: 1 or a variant thereof;
- a portion comprises nucleotides 36 to 75 of the sequence depicted in SEQ ID NO: 1 or a variant thereof;
- a portion comprises nucleotides 45 to 75 of the sequence depicted in SEQ ID NO: 1 or a variant thereof;
- a portion comprises nucleotides 58 to 75 of the sequence depicted in SEQ ID NO: 1 or a variant thereof;
- a portion comprises nucleotides 61 to 75 of the sequence depicted in SEQ ID NO: 1 or a variant thereof;
- a portion comprises nucleotides 70 to 75 of the sequence depicted in SEQ ID NO: 1 or a variant thereof;
- a portion comprises nucleotides 45 to 59 of the sequence depicted in SEQ ID NO: 1 or a variant thereof;
- a portion comprises nucleotides 1 to 44 of the sequence depicted in SEQ ID NO: 1 or a variant thereof;
- a portion comprises nucleotides 34 to 44 of the sequence depicted in SEQ ID NO: 1 or a variant thereof;
- a portion comprises nucleotides 1 to 6 of the sequence depicted in SEQ ID NO: 1 or a variant thereof;
- a portion comprises nucleotides 7 to 33 of the sequence depicted in SEQ ID NO: 1 or a variant thereof;
- a portion comprises nucleotides 247 to 265 of the sequence depicted in SEQ ID NO: 1 or a variant thereof.

According to the invention the targeting region of the polynucleotide may comprise or consist of a nucleotide sequence complementary to at least one of these portions or to at least one segment within these portions.

Preferred targeting region comprise or consist of a nucleotide sequence complementary to at least one continuous portion selected form the group consisting of nucleotides 1 to 75, nucleotides 1 to 65, nucleotides 1 to 40, nucleotides 36 to 75, nucleotides 45 to 75, nucleotides 58 to 75, nucleotides 61 to 75, nucleotides 70 to 75, nucleotides 45 to 59, nucleotides 1 to 44, nucleotides 34 to 44, nucleotides 1 to 6, nucleotides 7 to 33, nucleotides 247 to 265, each of the sequence depicted in SEQ ID NO: 1, and any variants of these portions.

According to one embodiment of the invention, the nucleotide sequence of the targeting region may comprise at least two segments, each segment complementary to a nucleotide sequence of the 5' -UTR, wherein the complementary nucleotide sequences of the 5' -UTR are not continuous. Preferred combinations of such segments are disclosed in the summary of the invention and the attached claims. Within the nucleotide sequence of these targeting regions complementary to a discontinuous portion of the 5'-UTR one or more nucleotides, between two consecutive segments can be comprised.

For example, in case that the targeting region comprises a nucleotide sequence complementary to nucleotides 1 to 6 and nucleotides 34 to 44, or respective subregions, of the sequence depicted in SEQ ID NO: 1, but not to nucleotides 7 to 33 (SL1 of the 5'-UTR), the complementary nucleotide sequences in the polynucleotide of the invention are preferably interspaced by two non-complementary nucleotides, for example a dinucleotide of two adenine nucleotides. In case that, for example, the targeting region comprises a nucleotide sequence complementary to nucleotides 45 to 59 (SL2) and nucleotides 61 to 75 (SL3), or respective subregions, of the sequence depicted in SEQ ID NO: 1, the complementary nucleotide sequences in the polynucleotide of the invention are preferably interspaced by one nucleotide, for example an adenine nucleotide.

In addition to comprising a nucleotide sequence complementary to the 5'-UTR or a portion thereof, the targeting region of the polynucleotides of the invention can further comprise a nucleotide sequence complementary to an open reading frame (ORF) of the genome of the Coronavirus, *e.g*., ORF1a or ORF 1ab or an ORF encoding the S protein, also known as spike protein. If present, a nucleotide sequence complementary to an open reading frame is preferably located 3' to the nucleotide sequence complementary to the 5'-UTR or to a portion thereof. The nucleotide sequence complementary to an open reading frame is preferably attached in-frame to the 5'-UTR complementary sequence. The length of the nucleotide sequence complementary to an ORF can be from 1 to 100 nucleotides, preferably 5 to 50 nucleotides, more preferably 10 to 30 nucleotides in length. For example, a targeting region comprising a nucleotide sequence complementary to at least nucleotides 255 to 265 of the sequence depicted in SEQ ID NO: 1, but preferably further elongated 5' to the nucleotide at position 255, may comprise, preferably in-frame, the first 5' nucleotides of ORF1a or ORF 1ab of a Coronavirus. An example of such a polynucleotide is depicted in SEQ ID NO: 31, wherein the nucleotide sequence of the targeting region is from nucleotides 14 to 53 of that sequence. According to another example of a polynucleotide of the present invention, which is capable of hybridizing to subgenomic mRNA of a Coronavirus, a targeting region comprising a nucleotide sequence complementary to at least nucleotides 61 to 75 of the sequence depicted in SEQ ID NO: 1, but preferably further elongated 5' to the nucleotide at position 61, may comprise, preferably in-frame, the first 5' nucleotides of the S-ORF. The length of the respective ORF nucleotide sequence can be from 1 to 100 nucleotides, preferably 5 to 50 nucleotides, more preferably 10 to 30 nucleotides. An example of such a polynucleotide is depicted in SEQ ID NO: 32, wherein the nucleotide sequence of the targeting region is from nucleotides 14 to 53 of that sequence.

A polynucleotide of the invention can comprise a targeting region comprising or consisting of a nucleotide sequence depicted in SEQ ID NO: 2, which sequence is complementary to SL2. Alternatively, a polynucleotide of the invention can comprise a targeting region comprising or consisting of a nucleotide sequence having at least 70%, at least 75%, at least 80%, at least 85%, at least 90%, at least 95%, at least 97%, at least 99%, or 100% identity to the nucleotide sequence depicted in SEQ ID NO: 2.

**Table 1: Nucleotide sequence for targeting SL2:**

| SEQ ID NO: | 5' elongation | SL2 (SEQ ID NO: 2) | 3' elongation |
|---|---|---|---|
| 2 | | GATCTACAAGAGATC | |

As the sequence depicted in SEQ ID NO: 2 is complementary to SL2 of the 5'-UTR, which is present both in the genome RNA and sgRNAs produced *e.g.,* in SARS-CoV-2 infected cells, the said polynucleotide can target both the genomic nucleic acid as well as mRNAs produced thereof.

For increasing specificity and/or efficiency the nucleotide sequence of the targeting region of said polynucleotide can be further elongated by one or more additional nucleotides. The nucleotide sequence depicted in SEQ ID NO: 2 can be elongated in one or both directions, *i.e.,* 3' and/or 5' relative to the said sequence.

Thus, the nucleotide sequence as set forth in SEQ ID NO: 2 can be additionally flanked 3' in a contiguous manner by one or more nucleotides for targeting nucleotide 44 up to at most nucleotide 1 of the sequence depicted in SEQ ID NO:1, respectively, or the respective nucleotides in variant sequences. The first nucleotide added 3' to the sequence depicted in SEQ ID NO: 2 is for targeting the nucleotide at position 44 of the sequence depicted in SEQ ID NO: 1. The second nucleotide added to the elongated sequence (*i.e.,* SEQ ID NO: 2 plus 1 nucleotide) is for targeting the nucleotide at position 43 of the sequence depicted in SEQ ID NO: 1, the third nucleotide added to the elongated sequence (*i.e.,* SEQ ID NO: 2 plus 2 nucleotides) is for targeting the nucleotide at position 42 of the sequence depicted in SEQ ID NO: 1, the forth nucleotide added to the elongated sequence (*i.e.,* SEQ ID NO: 2 plus 3 nucleotide) is for targeting the nucleotide at position 41 of the sequence depicted in SEQ ID NO: 1, and so forth. Thus, the nucleotide sequence as set forth in SEQ ID NO: 2 can be 3' elongated in a contiguous manner by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 or 44 nucleotides. Each nucleotide added 3' can be complementary or not to its respective nucleotide in the sequence depicted in SEQ ID NO: 1. For example, the first nucleotide added at the 3' end of the sequence (5'-gatctacaagagatc-3') depicted in SEQ ID NO: 2 is a nucleotide having the nucleobase guanin for targeting the nucleotide having the nucleobase cytosine at position 44 of the target strand (shown from 5' to 3' in the sequence below), the second nucleotide added at the 3' end of the elongated sequence is a nucleotide having the nucleobase adenine for targeting the nucleotide having the nucleobase tyrosine/uracil at position 43 of the target strand, the third nucleotide added at the 3' end of the elongated sequence is a nucleotide having the nucleobase adenine for targeting the nucleotide having the nucleobase tyrosine/uracil at position 42 of the target strand, the fourth nucleotide added at the 3' end of the elongated sequence is a nucleotide having the nucleobase adenine for targeting the nucleotide having the nucleobase tyrosine/uracil at position 41 of the target strand, and so forth.

(SEQ ID NO: 2)                                   3′-ctagagaacatctag-5′
                                                    |||||||||||||||
5′-attaaaggtttataccttcccaggtaacaaaccaaccaactttcgatctcttgtagatctgttctct
aaacgaactttaaaatctgtgtggctgtcactcggctgcatgcttagtgcactcacgcagtataattaat
aactaattactgtcgttgacaggacacgagtaactcgtctatcttctgcaggctgcttacggtttcgtcc
gtgttgcagccgatcatcagcacatctaggtttcgtccgggtgtgaccgaaaggtaag-3′
(SEQ ID NO: 1)

Even though the nucleotide sequence depicted in SEQ ID NO: 2 can be additionally flanked 3' in a contiguous manner by one or more nucleotides for targeting nucleotide 44 up to at most nucleotide 1 of the nucleotide sequence depicted in SEQ ID NO:1, respectively, in one embodiment nucleotides 33 to 7 of the sequence depicted in SEQ ID NO: 1 (SL1) are not to be targeted. Instead the elongated sequence preferably comprises at the respective portion two nucleotides, which are not complementary to the nucleotides at position 33 and 7 of the sequence depicted in SEQ ID NO: 1. For example, such an elongated targeting region may comprise or consist of a nucleotide sequence depicted in SEQ ID NO: 9 as shown below:

(SEQ ID NO: 9)
3′-taattta                              attggttgaaagctagagaacatctag-5′
   ||||||                                ||||||||||||||||||||||||||||
5′-attaaaggtttataccttcccaggtaacaaaccaaccaactttcgatctcttgtagatctgttctct
aaacgaactttaaaatctgtgtggctgtcactcggctgcatgcttagtgcactcacgcagtataattaat
aactaattactgtcgttgacaggacacgagtaactcgtctatcttctgcaggctgcttacggtttcgtcc
gtgttgcagccgatcatcagcacatctaggtttcgtccgggtgtgaccgaaaggtaag-3′
(SEQ ID NO: 1)

Instead of the AA-dinucleotide (shown in cursive letters) any other dinucleotide sequence may be used, which is not complementary to the sequence "5'-CG-3' " at positions 33 and 7 of the target strand, as is shown below:

(SEQ ID NO: 9)
3′-taatttd                              httggttgaaagctagagaacatctag-5′
   ||||||                               |||||||||||||||||||||||||||
5′-attaaaggtttataccttcccaggtaacaaaccaaccaactttcgatctcttgtagatctgttctct
aaacgaactttaaaatctgtgtggctgtcactcggctgcatgcttagtgcactcacgcagtataattaat
aactaattactgtcgttgacaggacacgagtaactcgtctatcttctgcaggctgcttacggtttcgtcc
gtgttgcagccgatcatcagcacatctaggtttcgtccgggtgtgaccgaaaggtaag-3′
(SEQ ID NO: 1)

In the nucleotide sequence depicted in SEQ ID NO: 9 the nucleotide "h" has the meaning a or c or t/u, but not g, and the nucleotide "d" has the meaning a or g or t/u, but not c. Also encompassed by the present invention are those sequences which comprise instead of the dinucleotide, *e.g.,* the AA-dinucleotide, an oligonucleotide sequence, which is not complementary to SL1 or a portion thereof.

The term "oligonucleotide" preferably refers to nucleic acids of between about at least 3 and about at most 14, about at most 12, or about at most 10 of single- or double-stranded DNA. However, for the purposes of this disclosure, there is no upper limit to the length of an oligonucleotide, and the terms oligonucleotide and polynucleotide may be used interchangeably.

Preferred nucleotide sequences of targeting regions which are elongated in 3' direction comprise the sequences as shown in Table 2:

**Table 2:**

| SEQ ID NO: | 5' elongation | SL2 (SEQ ID NO: 2) | 3' elongation |
|---|---|---|---|
| 3 | | GATCTACAAGAGATC | GAAAGTTGGTT |
| 4 | | GATCTACAAGAGATC | GAAAGTTGGTThdT |
| 5 | | GATCTACAAGAGATC | GAAAGTTGGTThdTT |
| 6 | | GATCTACAAGAGATC | GAAAGTTGGTThdTTT |
| 7 | | GATCTACAAGAGATC | GAAAGTTGGTThdTTTA |
| 8 | | GATCTACAAGAGATC | GAAAGTTGGTThdTTTAA |
| 9 | | GATCTACAAGAGATC | GAAAGTTGGTThdTTTAAT |

Examples of these nucleotide sequences are shown in Table 3.

**Table 3:**

| SEQ ID NO: | 5' elongation | SL2 (SEQ ID NO: 2) | 3' elongation |
|---|---|---|---|
| 3 | | GATCTACAAGAGATC | GAAAGTTGGTT |
| 4 | | GATCTACAAGAGATC | GAAAGTTGGTTaaT |
| 5 | | GATCTACAAGAGATC | GAAAGTTGGTTaaTT |
| 6 | | GATCTACAAGAGATC | GAAAGTTGGTTaaTTT |
| 7 | | GATCTACAAGAGATC | GAAAGTTGGTTaaTTTA |
| 8 | | GATCTACAAGAGATC | GAAAGTTGGTTaaTTTAA |
| 9 | | GATCTACAAGAGATC | GAAAGTTGGTTaaTTTAAT |

These preferred 3' elongations are for targeting the portion flanking the SL1 3' (SEQ ID NO: 3) or for targeting the portions flanking the SL1 3' and 5' (SEQ ID NO: 4 to 9). Also encompassed by the present invention are variants of the sequences depicted in SEQ ID NO: 3 to SEQ ID NO: 9.

Alternatively, or in addition the each possible elongated at 3', the nucleotide sequence depicted in SEQ ID NO: 2 can be flanked 5' in a contiguous manner by one or more nucleotides for targeting nucleotide 60 up to at most nucleotide 85, preferably up to at most nucleotide 75 of the sequence depicted in SEQ ID NO:1, respectively. The first nucleotide added 5' to the sequence as set forth in SEQ ID NO: 2 is for targeting the nucleotide at position 60 of the sequence depicted in SEQ ID NO: 1. The second nucleotide added to the elongated sequence (*i.e.,* SEQ ID NO: 2 plus 1 nucleotide) is for targeting the nucleotide at position 61 of the sequence depicted in SEQ ID NO: 1, the third nucleotide added to the elongated sequence (*i.e.,* SEQ ID NO: 2 plus 2 nucleotides) is for targeting the nucleotide at position 62 of the sequence depicted in SEQ ID NO: 1, the forth nucleotide added to the elongated sequence (*i.e.,* SEQ ID NO: 2 plus 3 nucleotide) is for targeting the nucleotide at position 63 of the sequence depicted in SEQ ID NO: 1, and so forth. Thus, the nucleotide sequence as set forth in SEQ ID NO: 2 can be 5' elongated in a contiguous manner by 1, 2, 3, 4, 5, 6, 7, 8, 9, 10, 11, 12, 13, 14, 15, 16, 17, 18, 19, 20, 21, 22, 23, 24, 25, or 26 nucleotides. Each nucleotide added 5' can be complementary or not to its respective nucleotide in the sequence depicted in SEQ ID NO: 1. For example, the first nucleotide added at the 5' end of the sequence (5'-gatctacaagagatc-3') depicted in SEQ ID NO: 2 is a nucleotide having the nucleobase adenine for targeting the nucleotide having the nucleobase thymine/uracil at position 60 of the target strand (shown from 5' to 3' in the sequence below), the second nucleotide added at the 5' end of the elongated sequence is a nucleotide having the nucleobase cytosine for targeting the nucleotide having the nucleobase guanine at position 61 of the target strand, the third nucleotide added at the 5' end of the elongated sequence is a nucleotide having the nucleobase adenine for targeting the nucleotide having the nucleobase thymine/uracil at position 62 of the target strand, the fourth nucleotide added at the 5' end of the elongated sequence is a nucleotide having the nucleobase adenine for targeting the nucleotide having the nucleobase thymine/uracil at position 63 of the target strand, and so forth.

(SEQ ID NO: 2)                                   3′-ctagagaacatctag-5′
                                                    |||||||||||||||
5′-attaaaggtttataccttcccaggtaacaaaccaaccaactttcgatctcttgtagatctgttctct
aaacgaactttaaaatctgtgtggctgtcactcggctgcatgcttagtgcactcacgcagtataattaat
aactaattactgtcgttgacaggacacgagtaactcgtctatcttctgcaggctgcttacggtttcgtcc
gtgttgcagccgatcatcagcacatctaggtttcgtccgggtgtgaccgaaaggtaag-3′
(SEQ ID NO: 1)

Preferred nucleotide sequences of targeting regions which are elongated in 5' direction comprise the sequences as shown in Table 3:

**Table 4:**

| SEQ ID NO: | 5' elongation | SL2 (SEQ ID NO: 2) | 3' elongation |
|---|---|---|---|
| 10 | AGAACA | GATCTACAAGAGATC | |
| 11 | GAGAACA | GATCTACAAGAGATC | |
| 12 | AGAGAACA | GATCTACAAGAGATC | |
| 13 | TAGAGAACA | GATCTACAAGAGATC | |
| 14 | TTAGAGAACA | GATCTACAAGAGATC | |
| 15 | TTTAGAGAACA | GATCTACAAGAGATC | |
| 16 | GTTTAGAGAACA | GATCTACAAGAGATC | |
| 17 | CGTTTAGAGAACA | GATCTACAAGAGATC | |
| 18 | TCGTTTAGAGAACA | GATCTACAAGAGATC | |
| 19 | TTCGTTTAGAGAACA | GATCTACAAGAGATC | |
| 20 | GTTCGTTTAGAGAACA | GATCTACAAGAGATC | |

Also encompassed by the present invention are variants of the sequences set forth in SEQ ID NO: 10 to SEQ ID NO: 20.

The polynucleotides of the present invention can also comprise a targeting region comprising or consisting of a nucleotide sequence which is the result of any combination between above mentioned 5' elongations and 3' elongations, including variants of these combined nucleotide sequences.

Preferred (combined) targeting regions comprise or consist of a nucleotide sequence depicted in in any of SEQ ID NO: 21 to 23 and/or Table 5, or any variants thereof.

**Table 5:**

| SEQ ID NO: | 5' elongation | SL2 (SEQ ID NO: 2) | 3' elongation |
|---|---|---|---|
| 21 | AGAACA | GATCTACAAGAGATC | GAAAGTTGGTTaaTTTAAT |
| 22 | GTTCGTTTAGAGAACA | GATCTACAAGAGATC | GAAAGTTGGTTaaTTTAAT |
| 23 | GTTCGTTTAGAGAACA | GATCTACAAGAGATC | GAAAGTTGG |

Other preferred targeting regions comprised in a polynucleotide of the invention may comprise or consist of a nucleotide sequence depicted in any of SEQ ID NO: 24 to 26 or any variants thereof.

| | |
|---|---|
| SEQ ID NO: 24 | GTTGGTTGGTTTGTTACCTGGGAAGGTATAAACCTTTAAT |
| SEQ ID NO: 25 | CTTACCTTTCGGTCACACC |
| SEQ ID NO: 26 | GTTCGTTTAGAGAACAGA |

The sequence depicted in SEQ ID NO: 24 is for targeting nucleotide positions 1 to 40 of the sequence depicted in SEQ ID NO: 1, the sequence depicted in SEQ ID NO: 25 is for targeting nucleotide positions 247 to 265 of the sequence depicted in SEQ ID NO: 1, and the sequence depicted in SEQ ID NO: 26 is for targeting nucleotide positions 58 to 75 of the sequence depicted in SEQ ID NO: 1.

In one embodiment, polynucleotide can in addition to (i) the targeting region, comprise (ii) a region comprising or consisting of a nucleotide sequence capable of forming a stem-loop, which region is linked to the targeting region of (i), and (iii) two linker nucleotide sequences flanking the nucleotide sequence of the targeting region at both sides, *i.e.,* 5' and 3'. That is, when the polynucleotide is in a circularized form, the targeting region is on both sides separated from region capable of forming a stem-loop of (ii) by the one linker sequence. The linkers allow a more flexible presentation of the antisense sequence.

The nucleotide sequence of the targeting region may be at least 15 nucleotides, at least 20 nucleotides, or at least 25 nucleotides in length, and/or the nucleotide sequence of the targeting region may be at most 300 nucleotides, at most 265 nucleotides, at most 250 nucleotides, or at most 150 nucleotides in length. For example, the nucleotide sequence of the targeting region may be 15 to 300 nucleotides or 20 to 250 nucleotides or 15 to 265 nucleotides in length. As will be easily understood by a person skilled in the art any of the lower limits disclosed can be combined with any of the upper limits mentioned.

The region capable of forming a stem-loop may comprise at least 2, 3, 4, 5 or 6, and/or at most 15, 14, 13, 12, 11, 10, 9 or 8 perfect base-pairs with two overhanging ends. For example, the region capable of forming a stem-loop may comprise 4 to 10 or 5 to 8 perfect base-pairs. As will be easily understood by a person skilled in the art any of the lower limits disclosed can be combined with any of the upper limits mentioned. The overhanging ends may be each at least 2, 3, 4, 5, 6, 7, 8, 9 or 10 nucleotides in length, and/or the overhanging ends may be each at most 40, 35, 30, 20 or 15 nucleotides in length. For example, the overhanging ends may be each 2 to 30 or 3 to 20 nucleotides in length. As will be easily understood by a person skilled in the art any of the lower limits disclosed can be combined with any of the upper limits mentioned.

Each linker is preferably at least 1, 2 or 3 nucleotides in length, and/or is preferably at most 20, 15, 12, 10 or 8 nucleotides in length. For example, each linker may be 1 to 20 or 2 to 10 nucleotides in length. As will be easily understood by a person skilled in the art any of the lower limits disclosed can be combined with any of the upper limits mentioned.

Preferred embodiments of polynucleotides comprising (i) a targeting region, (ii) a region comprising or consisting of a nucleotide sequence capable of forming a stem-loop, and (iii) two linker nucleotide sequences are represented by the sequences depicted in SEQ ID NO: 27 to 32, or any variants thereof.

A polynucleotide of the invention can in one embodiment comprise at least one ribonucleotide and/or at least one deoxyribonucleotide. In one embodiment, a polynucleotide of the invention consists of ribonucleotides, deoxyribonucleotides or a combination thereof, with the 5' end of one nucleotide and the 3' end of another nucleotide being linked to one another by a phosphodiester bond. These polynucleotides may be synthesized in the conventional manner or produced recombinantly. According to the present invention, the polynucleotide may be or preferably is circular, which means that the polynucleotide, *e.g.,* the RNA has been circularized.

In the context of the present invention, the term "RNA" relates to a molecule which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double stranded RNA, single stranded RNA, isolated RNA such as partially or completely purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as internally, for example, at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from nature, but that may be discovered and/or isolated in the future from a natural source.

The term "recombinant" in the context of the present invention means "made through genetic engineering". Preferably, a "recombinant object" in the context of the present invention is not occurring naturally.

According to the present invention, the term "RNA" includes and preferably relates to "circular RNA" or "circRNA" which means a RNA molecule that has been circularized, for example such that it does not have a 5' and 3' end, *e.g.,* one that results from the ligation of the 5' end to the 3' end or one that results from the action of one or more ribozymes. Preferably, the RNA is produced by *in vitro* transcription using a DNA template and then the ends are ligated together. In one embodiment of the invention, the RNA is obtained by *in vitro* transcription or chemical synthesis. The *in vitro* transcription and ligation methodologies are known to the skilled person. For example, there is a variety of commercially available *in vitro* transcription kits as well as ligation kits.

The RNA according to the invention may be unmodified and/or comprise naturally occurring nucleobases only, i.e., the naturally occurring heterocyclic nucleobases of RNA or DNA: the purine bases adenine (A) and guanine (G), and the pyrimidine bases thymine (T), cytosine (C) (including 5-methyl C), and uracil (U). Alternatively, the RNA according to the invention may contain a modification. As used herein, " modification" means a chemical difference in a compound when compared to a naturally occurring counterpart. In reference to a polynucleotide, chemical modification does not include differences only in nucleobase sequence, *i.e.,* a base modification, but for example, also comprise a backbone modification or a sugar modification. That is, the polynucleotide may be modified in very different ways, without impairing its ability to bind its target, in order to increase, for example, its stability or therapeutic efficacy.

In one embodiment, the RNA has modified ribonucleotides, which can increase its stability and/or decrease cytotoxicity or immunogenicity. For example, in one embodiment, in the RNA used according to the invention, 5-methylcytidine is substituted partially or completely, preferably completely, for cytidine. Alternatively, or additionally, in one embodiment, in the RNA used according to the invention pseudouridine is substituted partially or completely, preferably completely, for uridine.

Other modified ribonucleotides include 1-methyl-adenine, 2-methyladenine, 2-methylthio-N6-isopentenyladenine, N6-methyladenine, N6-isopentenyladenine, 2-thio-cytosine, 3-methylcytosine, 4-acetylcytosine, 5-methylcytosine, 2,6-diaminopurine, 1-methylguanine, 2-methylguanine, 2,2-dimethylguanine, 7-methylguanine, 7-deaza-guanosine, inosine, 1-methylinosine, pseudouracil (5-uracil), dihydrouracil, 2-thiouracil, 4-thiouracil, 5-carboxymethylaminomethyl-2-thiouracil, 5-(carboxyhydroxymethyl)-uracil, 5-fluorouracil, 5-bromouracil, 5-carboxymethylaminomethyluracil, 5- methyl-2-thiouracil, 5-methyluracil, N-uracil-5-oxyacetic acid methyl ester, 5-methylamino-methyluracil, 5-methoxyaminomethyl-2-thiouracil, 5'-methoxycarbonylmethyluracil, 5-methoxyuracil, uracil-5-oxyacetic acid methyl ester, uracil-5-oxyacetic acid, 1-methylpseudouracil, queosine, and β-D-mannosylqueosine.

Backbone modifications typically include, without being limited thereto, modifications from the group consisting of methylphosphonates, methylphosphoramidates, phosphoramidates, phosphorothioates, and boranophosphates.

A sugar modification in connection with the circular RNA described herein is a chemical modification of the sugar of the nucleotides it contains and typically includes, without being limited thereto, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

The term "stability" of an agent, e.g., circular RNA, to the "half-life" of the agent. "Half-life" relates to the period of time which is needed to eliminate half of the maximum (pharmacologic) activity, amount, or number of molecules of an agent, *e.g.,* from the body or blood of a patient to which the agent was administered or as measured in an *in vitro* assay. The maximum pharmacologic activity is defined by the steady state value, where intake equals elimination. Since the kinetics of certain agents, such as pharmaceutical drugs, can be complex, the "half-life" of an agent does not necessarily follow or is limited to first order kinetics.

The nucleic acid, *e*.*g*., the RNA, to be administered according to the invention is preferably non-immunogenic. The term "non-immunogenic nucleic acid" or "non-immunogenic RNA" as used herein refers to a nucleic acid or RNA, respectively, that does not induce a response by the immune system upon administration, *e.g.,* to a mammal, or induces a weaker response than would have been induced by the same nucleic acid, *e.g.,* the same RNA, that differs only in that it has not been subjected to the modifications and treatments that render the immunogenic nucleic acid, *e.g.,* the RNA, non-immunogenic. In one preferred embodiment a non-immunogenic nucleic acid or a non-immunogenic RNA is rendered non-immunogenic by incorporating modified nucleotides suppressing nucleic acid or RNA-mediated activation of innate immune receptors into the nucleic acid or the RNA or by having a sequence that does not form double-stranded structures or by removing any double-stranded nucleic acids or double-stranded RNA (dsRNA) present with the circular RNA.

For rendering the non-immunogenic nucleic acid, *e.g.,* the RNA, non-immunogenic by the incorporation of modified nucleotides, any modified nucleotide may be used as long as it lowers or suppresses immunogenicity of the nucleic acid, *e.g.,* the RNA. Particularly preferred are modified nucleotides that suppress a nucleic acid-mediated activation of innate immune receptors. In one embodiment, the modified nucleotides comprise a replacement of one or more uridines with a nucleoside comprising a modified nucleobase. In one embodiment, the modified nucleobase is a modified uracil. In one embodiment, the nucleoside comprising a modified nucleobase is selected from the group consisting of 3-methyl-uridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho5U), 5-aminoallyl-uridine, 5-halo-uridine (*e.g.,* 5-iodo-uridineor 5-bromo-uridine), uridine 5-oxyacetic acid (cmo5U), uridine 5-oxyacetic acid methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5 s2U), 5-aminomethyl-2-thio-uridine (nm5s2U), 5-methylaminomethyl-uridine (mnm5U), 1-ethyl-pseudouridine, 5-methylaminomethyl-2-thio-uridine (mnm5 s2U), 5 -methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyluridine, 1-propynyl-pseudouridine, 5-taurinomethyl-uridine (τm5U), 1-taurinomethyl-pseudouridine, 5-taurinomethyl-2-thio-uridine (τm5s2U), 1-taurinomethyl-4-thio-pseudouridine, 5-methyl-2-thio-uridine (m5s2U), 1-methyl-4-thio-pseudouridine (m1s4ψ), 4-thio-1-methyl-pseudouridine, 3-methyl-pseudouridine (nψ3), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylamino-methyl)uridine (inm5U), 5-(isopentenylaminomethyl)-2-thio-uridine (inm5s2U), α-thio-uridine, 2'-O-methyl-uridine (Um), 5,2-O-dimethyl-uridine (m5Um), 2'-O-methyl-pseudouridine (ψm), 2-thio-2 '-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,20-dimethyl-uridine (m3Um), 5-(isopentenylaminomethyl)-2'-O-methyl-uridine (inm5Um), 1-thio-uridine, deoxythymidine, 2'-F-ara-uridine, 2-F-uridine, 2-OH-ara-uridine, 5-(2-carbomethoxy-vinyl) uridine, and 5-[3-(1-E-propenylamino)uridine. In one particularly preferred embodiment, the nucleoside comprising a modified nucleobase is pseudouridine (ψ), N1-methyl-pseudouridine (m1ψ) or 5-methyl-uridine (m5U), in particular 1-methyl-pseudouridine.

The structure of an exemplary nucleoside comprising a modified nucleobase is 1-methylpseudouridine m1Ψ:

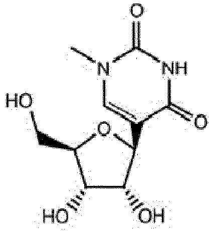

In one embodiment, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of the uridines.

During synthesis of mRNA by *in vitro* transcription (IVT) using T7 RNA polymerase significant amounts of aberrant products, including double-stranded RNA (dsRNA) are produced due to unconventional activity of the enzyme. dsRNA induces inflammatory cytokines and activates effector enzymes leading to protein synthesis inhibition. dsRNA can be removed from RNA such as IVT RNA, for example, by ion-pair reversed phase HPLC using a non-porous or porous C-18 polystyrene-divinylbenzene (PS-DVB) matrix. Alternatively, an enzymatic based method using E. coli RNaseIII that specifically hydrolyzes dsRNA but not ssRNA, thereby eliminating dsRNA contaminants from IVT RNA preparations can be used. Furthermore, dsRNA can be separated from ssRNA by using a cellulose material. In one embodiment, an RNA preparation is contacted with a cellulose material and the ssRNA is separated from the cellulose material under conditions which allow binding of dsRNA to the cellulose material and do not allow binding of ssRNA to the cellulose material. It is to be understood that the unwanted dsRNA can be removed prior to or after the ligation step forming the circular RNA.

According to the invention, "double-stranded RNA" or "dsRNA" means RNA with two partially or completely complementary strands. Although the circRNA described herein preferably is a circularized "single-stranded" RNA molecule, it may nonetheless contain self-complementary sequences that allow parts of the RNA to fold back and to form secondary structure motifs including without limitation base pairs, stems, stem loops and bulges.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances.

The polynucleotides of the invention, *e.g.,* the circular RNAs, and compositions described herein are useful in methods of treating or preventing a disease, for example for treating or preventing an infection with a pathogen, such as, an infection with a Coronavirus. The polynucleotides and compositions described herein are also useful in methods for targeting a nucleic acid, for example for targeting the genome of a pathogen, such as a Coronavirus, for forming a complex with a nucleic acid, for example the genome of a pathogen, such as a Coronavirus, and/or in methods for interfering with genome expression and proliferation of a pathogen, such as a Coronavirus. In all these embodiments, methods and uses the Coronavirus may be SARS-CoV-2 having a nucleotide sequence depicted in NCBI Reference Sequence: NC_045512.2 (incorporated herein as SEQ ID NO: 35). According to the invention preferably circular DNA or circular RNA, more preferably non-immunogenic circular DNA or circular RNA, is administered to the patient/subject.

The expression "for use in treating or preventing a disease, for example for treating or preventing an infection with a pathogen, *e.g.,* an infection with a Coronavirus" is used herein also replaceable with "for use as a medicament, especially in a method of treating or preventing an infection with a pathogen, *e.g.,* an infection with a Coronavirus"; or the use of said products in the preparation of a pharmaceutical formulation for use in said method of treatment in humans (or more generically a subject in need thereof).

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as cancer. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality. According to the invention, the term "disease" preferably includes an infection with a pathogen, such as an infection with an RNA virus. The RNA virus may be a Coronavirus, such as SARS-CoV-2.

The term "treatment" or "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual, in particular an individual being at risk for the disease. The terms "prophylactic treatment" or "preventive treatment" are used herein interchangeably.

The terms "protect", "prevent", "prophylactic", "preventive", or "protective" relate to the prevention and/or treatment of the occurrence and/or the propagation of a disease, e.g., an infection with a Coronavirus, in an individual. For example, a prophylactic administration of a circular RNA, *e.g.,* by administering a composition of the present invention, can protect the receiving individual from the development of infection symptoms. For example, by administering a composition of the present invention, the development of a disease can be stopped, e.g., leading to inhibition of the progress of an infection. This comprises the deceleration of the progress of the infection, in particular a disruption of the progression of the infection, which preferably leads to elimination of the pathogen.

By "being at risk" is meant a subject, *i.e.,* a patient, that is identified as having a higher than normal chance of developing a disease compared to the general population.

The term *"in vivo"* relates to the situation in a subject.

The term "individual" or "subject" or "patient" relates to vertebrates, particularly mammals. For example, mammals in the context of the present invention are humans, non-human primates, domesticated mammals such as dogs, cats, sheep, cattle, goats, pigs, horses *etc.,* laboratory animals such as mice, rats, rabbits, guinea pigs, *etc.* as well as animals in captivity such as animals of zoos. The term "subject" also relates to non-mammalian vertebrates such as birds (particularly domesticated birds such as chicken, ducks, geese, turkeys) and to fish (particularly farmed fish, *e.g.,* salmon or catfish). The term "animal" as used herein also includes humans.

The agents, *e*.*g*., the circular RNA molecules described herein, may be administered in the form of any suitable pharmaceutical composition. The term "pharmaceutical composition" relates to a formulation comprising a therapeutically effective agent or a salt thereof, preferably together with pharmaceutical excipients such as buffers, preservatives and tonicity modifiers. Said pharmaceutical composition is useful for treating or preventing a disease or disorder by administration of said pharmaceutical composition to an individual. A pharmaceutical composition is also known in the art as a pharmaceutical formulation. The pharmaceutical composition can be administered locally or systemically, alone or in combination with any other agent.

The term "systemic administration" refers to the administration of a therapeutically effective agent such that the agent becomes widely distributed in the body of an individual in significant amounts and develops a biological effect. According to the present invention, it is preferred that administration is by parenteral administration, wherein the polynucleotide or the pharmaceutical composition of the invention can be administered alone or in combination with any other agent.

The term "parenteral administration" refers to administration of a therapeutically effective agent such that the agent does not pass the intestine. The term "parenteral administration" includes intravenous administration, subcutaneous administration, intradermal administration, inhalation or intraarterial administration, but is not limited thereto.

The pharmaceutical composition according to the present invention is generally applied in a "pharmaceutically effective amount" and in "a pharmaceutically acceptable preparation".

The term "pharmaceutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severity of the disease, the individual parameters of the patient, including age, physiological condition, size and weight, the duration of treatment, the type of an accompanying therapy (if present), the specific route of administration and similar factors. Accordingly, the doses administered of the compositions described herein may depend on several of such parameters. In the case that a reaction in a patient is insufficient with an initial dose, higher doses (or effectively higher doses achieved by a different, more localized route of administration) may be used.

The term "pharmaceutically acceptable" refers to the non-toxicity of a material which does not interact with the action of the active component of the pharmaceutical composition.

The pharmaceutical compositions of the present invention may contain salts, buffers, preserving agents, carriers and optionally other therapeutic agents. Preferably, the pharmaceutical compositions of the present invention comprise one or more pharmaceutically acceptable carriers, diluents and/or excipients.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient. Possible carrier substances include, e.g., sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxypropylene copolymers, oils, including those which are derived from mineral oil, animals, or plants, such as peanut oil, soy bean oil, sesame oil, sunflower oil, *etc.* Salt solutions and aqueous dextrose and glycerin solutions may also be used as aqueous carrier compounds.

The term "excipient" when used herein is intended to indicate all substances which may be present in a pharmaceutical composition and which are not active ingredients such as, *e.g.,* carriers, binders, lubricants, thickeners, surface active agents, preservatives, emulsifiers, buffers, flavoring agents, or colorants.

The term "diluent" relates a diluting and/or thinning agent. Moreover, the term "diluent" includes any one or more of fluid, liquid or solid suspension and/or mixing media.

Pharmaceutically acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include, for example, magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. Examples of suitable diluents include ethanol, glycerol and water.

Pharmaceutical carriers, excipients or diluents can be selected with regard to the intended route of administration and standard pharmaceutical practice. The pharmaceutical compositions of the present invention may comprise as, or in addition to, the carrier(s), excipient(s) or diluent(s) any suitable binder(s), lubricant(s), suspending agent(s), coating agent(s), and/or solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include sodium benzoate, benzalkonium chloride, chlorobutanol, paraben, thimersol, sorbic acid and esters of p-hydroxybenzoic acid. Antioxidants and suspending agents may be also used.

In one embodiment, the composition is an aqueous composition. The aqueous composition may optionally comprise solutes, *e.g.,* salts. In one embodiment, the composition is in the form of a freeze-dried composition. A freeze-dried composition is obtainable by freeze-drying a respective aqueous composition.

In one embodiment, the composition is an injectable composition, optionally comprising sodium, potassium and calcium salts, such as Ringer's solution or Ringer's Lactate.

Terms such as "transferring", "introducing", "transfecting" or "transducing" are used interchangeably herein and relate to the introduction of nucleic acids, in particular exogenous or heterologous nucleic acids, such as circular RNA into a cell. According to the present invention, the cell can be present *in vitro* or *in vivo, e.g.,* the cell can form part of an organ, a tissue and/or an organism. According to the invention, transfection can be transient or stable. For some applications of transfection, it is sufficient if the transfected genetic material is only transiently present. Since the nucleic acid introduced in the transfection process is usually not integrated into the nuclear genome, the foreign nucleic acid will be diluted through mitosis or degraded.

In one embodiment, the polynucleotide, *e.g.,* the circular RNA molecule, to be administered is naked, *i.e.,* not complexed with any kind of delivery material or proteins. In one embodiment, the polynucleotide, *e.g.,* the RNA molecule, to be administered is formulated in a delivery vehicle. In one embodiment, the delivery vehicle comprises particles. In one embodiment, the delivery vehicle comprises a lipid. In one embodiment, the lipid comprises a cationic lipid. In one embodiment, the lipid forms a complex with and/or encapsulates the polynucleotide molecule. In one embodiment, the polynucleotide molecule is formulated in liposomes.

The pharmaceutical compositions of the invention are preferably sterile and contain an effective amount of the agents described herein and optionally of further agents as discussed herein to generate the desired reaction or the desired effect.

Pharmaceutical compositions are usually provided in a uniform dosage form and may be prepared in a manner known *per se.* A pharmaceutical composition may, *e.g.,* be in the form of a solution or suspension.

In one embodiment, if the pharmaceutical composition comprises nucleic acids, it can comprise at least one cationic entity. In general, cationic lipids, cationic polymers and other substances with positive charges may form complexes with negatively charged nucleic acids. It is possible to stabilize in particular the RNA according to the invention by complexation with cationic compounds, preferably polycationic compounds such as for example a cationic or polycationic peptide or protein. In one embodiment, the pharmaceutical composition according to the present invention comprises at least one cationic molecule selected from the group consisting protamine, polyethylene imine, a poly-L-lysine, a poly-L-arginine, a histone or a cationic lipid.

According to the present invention, a cationic lipid is a cationic amphiphilic molecule, *e.g.,* a molecule which comprises at least one hydrophilic and lipophilic moiety. The cationic lipid can be monocationic or polycationic. Cationic lipids typically have a lipophilic moiety, such as a sterol, an acyl or diacyl chain, and have an overall net positive charge. The head group of the lipid typically carries the positive charge. The cationic lipid preferably has a positive charge of 1 to 10 valences, more preferably a positive charge of 1 to 3 valences, and more preferably a positive charge of 1 valence. Examples of cationic lipids include, but are not limited to 1,2-di-O-octadecenyl-3-trimethylammonium propane (DOTMA); dimethyldioctadecylammonium (DDAB); 1,2-dioleoyl-3-trimethylammonium-propane (DOTAP); 1,2-dioleoyl-3-dimethylammonium-propane (DODAP); 1,2-diacyloxy-3-dimethylammonium propane; 1,2-dialkyloxy-3-dimethylammonium propane; dioctadecyldimethyl ammonium chloride (DODAC), 1,2-dimyristoyl-oxypropyl-1,3-dimethylhydroxyethyl ammonium (DMRIE), and 2,3-dioleoyloxy-N-[2(spermine carboxamide)ethyl]-N,N-dimethyl-1-propanamium trifluoroacetate (DOSPA). Cationic lipids also include lipids with a tertiary amine group, including 1,2-dilinoleyloxy-N,N-dimethyl-3-aminopropane (DLinDMA). Cationic lipids are suitable for formulating RNA in lipid formulations as described herein, such as liposomes, emulsions and lipoplexes. Typically, positive charges are contributed by at least one cationic lipid and negative charges are contributed by the RNA. In one embodiment, the pharmaceutical composition comprises at least one helper lipid, in addition to a cationic lipid. The helper lipid may be a neutral or an anionic lipid. The helper lipid may be a natural lipid, such as a phospholipid, or an analogue of a natural lipid, or a fully synthetic lipid, or lipid-like molecule, with no similarities with natural lipids. In the case where a pharmaceutical composition includes both a cationic lipid and a helper lipid, the molar ratio of the cationic lipid to the neutral lipid can be appropriately determined in view of stability of the formulation and the like.

In one embodiment, the pharmaceutical composition according to the present invention comprises protamine. According to the invention, protamine is useful as cationic carrier agent. The term "protamine" refers to any of various strongly basic proteins of relatively low molecular weight that are rich in arginine and are found associated especially with DNA in place of somatic histones in the sperm cells of animals such as fish. In particular, the term "protamine" refers to proteins found in fish sperm that are strongly basic, are soluble in water, are not coagulated by heat, and comprise multiple arginine monomers. According to the invention, the term "protamine" as used herein is meant to comprise any protamine amino acid sequence obtained or derived from native or biological sources including fragments thereof and multimeric forms of said amino acid sequence or fragment thereof. Furthermore, the term encompasses (synthesized) polypeptides which are artificial and specifically designed for specific purposes and cannot be isolated from native or biological sources.

The pharmaceutical composition according to the invention can be buffered, (e.g., with an acetate buffer, a citrate buffer, a succinate buffer, a Tris buffer, a phosphate buffer).

In some embodiments, the polynucleotides, *e.g.,* the circular RNA molecules, of the present invention are provided in complexed or encapsulated form. Respective pharmaceutical compositions are provided in the present invention. In particular, in some embodiments, the pharmaceutical composition of the present invention comprises nucleic acid-containing particles, preferably RNA-containing particles. Respective pharmaceutical compositions are referred to as particulate formulations. In particulate formulations according to the present invention, a particle comprises a polynucleotide according to the invention and a pharmaceutically acceptable carrier or a pharmaceutically acceptable vehicle that is suitable for delivery of the nucleic acid. The nucleic acid-containing particles may be, for example, in the form of proteinaceous particles or in the form of lipid-containing particles. Suitable proteins or lipids are referred to as particle forming agents. Proteinaceous particles and lipid-containing particles have been described previously to be suitable for delivery of RNA in particulate form (*e.g.,* Strauss & Strauss, 1994, Microbiol. Rev. 58:491-562).

In one embodiment, the particulate formulation of the present invention is a nanoparticulate formulation. In that embodiment, the composition according to the present invention comprises a polynucleotide according to the invention in the form of nanoparticles. Nanoparticulate formulations can be obtained by various protocols and with various complexing compounds. Lipids, polymers, oligomers, or amphiphiles are typical constituents of nanoparticulate formulations.

As used herein, the term "nanoparticle" refers to any particle having a diameter making the particle suitable for systemic, in particular parenteral, administration, of, in particular, nucleic acids, typically a diameter of 1000 nanometers (nm) or less. In one embodiment, the nanoparticles have an average diameter in the range of from about 50 nm to about 1000 nm, preferably from about 50 nm to about 400 nm, preferably about 100 nm to about 300 nm such as about 150 nm to about 200 nm. In one embodiment, the nanoparticles have a diameter in the range of about 200 to about 700 nm, about 200 to about 600 nm, preferably about 250 to about 550 nm, in particular about 300 to about 500 nm or about 200 to about 400 nm.

In one embodiment, the polydispersity index (PI) of the nanoparticles described herein, as measured by dynamic light scattering, is 0.5 or less, preferably 0.4 or less or even more preferably 0.3 or less. The "polydispersity index" (PI) is a measurement of homogeneous or heterogeneous size distribution of the individual particles (such as liposomes) in a particle mixture and indicates the breadth of the particle distribution in a mixture. The PI can be determined, for example, as described in WO 2013/143555 A1.

As used herein, the term "nanoparticulate formulation" or similar terms refer to any particulate formulation that contains at least one nanoparticle. In some embodiments, a nanoparticulate composition is a uniform collection of nanoparticles. In some embodiments, a nanoparticulate composition is a lipid-containing pharmaceutical formulation, such as a liposome formulation or an emulsion.

In one embodiment, the pharmaceutical composition according to the invention comprises circular RNA encapsulated in a vesicle, *e.g.,* in a liposome. In one embodiment, the pharmaceutical composition according to the invention comprises circRNA in the form of an emulsion. In one embodiment, the pharmaceutical composition according to the invention comprises circular RNA in a complex with a cationic compound, thereby forming, e.g., so-called lipoplexes or polyplexes. Encapsulation of RNA within vesicles such as liposomes is distinct from, for instance, lipid/RNA complexes. Lipid/RNA complexes are obtainable, *e.g.,* when RNA is, *e.g.,* mixed with pre-formed liposomes.

In one embodiment, the pharmaceutical composition according to the invention comprises circular RNA encapsulated in a vesicle. Such formulation is a particular particulate formulation according to the invention. A vesicle is a lipid bilayer rolled up into a spherical shell, enclosing a small space and separating that space from the space outside the vesicle. Typically, the space inside the vesicle is an aqueous space, *i.e.,* comprises water. Typically, the space outside the vesicle is an aqueous space, *i.e.,* comprises water. The lipid bilayer is formed by one or more lipids (vesicle-forming lipids). The membrane enclosing the vesicle is a lamellar phase, similar to that of the plasma membrane. The vesicle according to the present invention may be a multilamellar vesicle, a unilamellar vesicle, or a mixture thereof. When encapsulated in a vesicle, the circular RNA is typically separated from any external medium. Suitable vesicles are particles, particularly nanoparticles, as described herein.

For example, circular RNA may be encapsulated in a liposome. In that embodiment, the pharmaceutical composition is or comprises a liposome formulation. Encapsulation within a liposome will typically protect RNA from RNase digestion. It is possible that the liposomes include some external RNA (*e.g.,* on their surface), but at least half of the RNA (and ideally all of it) is encapsulated within the core of the liposome.

Liposomes are microscopic lipidic vesicles often having one or more bilayers of a vesicle-forming lipid, such as a phospholipid, and are capable of encapsulating a drug, *e.g.,* circular RNA. Different types of liposomes may be employed in the context of the present invention, including, without being limited thereto, multilamellar vesicles (MLV), small unilamellar vesicles (SUV), large unilamellar vesicles (LUV), sterically stabilized liposomes (SSL), multivesicular vesicles (MV), and large multivesicular vesicles (LMV) as well as other bilayered forms known in the art. The size and lamellarity of the liposome will depend on the manner of preparation. There are several other forms of supramolecular organization in which lipids may be present in an aqueous medium, comprising lamellar phases, hexagonal and inverse hexagonal phases, cubic phases, micelles, reverse micelles composed of monolayers.

Liposomes may be formed using standard methods known to the skilled person. Respective methods include the reverse evaporation method, the ethanol injection method, the dehydration-rehydration method, sonication or other suitable methods. Following liposome formation, the liposomes can be sized to obtain a population of liposomes having a substantially homogeneous size range.

In one embodiment, the circular RNA is present in a liposome which includes at least one cationic lipid. Respective liposomes can be formed from a single lipid or from a mixture of lipids, provided that at least one cationic lipid is used. Preferred cationic lipids have a nitrogen atom which is capable of being protonated; preferably, such cationic lipids are lipids with a tertiary amine group. A particularly suitable lipid with a tertiary amine group is 1,2-dilinoleyloxy-N,N-dimethyl-3-aminopropane (DLinDMA). In one embodiment, the RNA according to the present invention is present in a liposome formulation as described in WO 2012/006378 A1: a liposome having a lipid bilayer encapsulating an aqueous core including RNA, wherein the lipid bilayer comprises a lipid with a pKa in the range of 5.0 to 7.6, which preferably has a tertiary amine group. Preferred cationic lipids with a tertiary amine group include DLinDMA (pKa 5.8) and are generally described in WO 2012/031046 A2. According to WO 2012/031046 A2, liposomes comprising a respective compound are particularly suitable for encapsulation of RNA and thus liposomal delivery of RNA. In one embodiment, the circRNA according to the present invention is present in a liposome formulation, wherein the liposome includes at least one cationic lipid whose head group includes at least one nitrogen atom (N) which is capable of being protonated, wherein the liposome and the RNA have a N:P ratio of between 1:1 and 20:1. According to the present invention, "N:P ratio" refers to the molar ratio of nitrogen atoms (N) in the cationic lipid to phosphate atoms (P) in the RNA comprised in a lipid containing particle (*e.g.,* liposome), as described in WO 2013/006825 A1. The N:P ratio of between 1:1 and 20:1 is implicated in the net charge of the liposome and in efficiency of delivery of RNA to a vertebrate cell.

In one embodiment, the polynucleotide, *e.g.,* the circular RNA, according to the present invention is present in a liposome formulation that comprises at least one lipid which includes a polyethylene glycol (PEG) moiety, wherein RNA is encapsulated within a PEGylated liposome such that the PEG moiety is present on the liposome's exterior, as described in WO 2012/031043 A1 and WO 2013/033563 A1.

In one embodiment, the polynucleotide, *e.g.,* the circular RNA, according to the present invention is present in a liposome formulation, wherein the liposome has a diameter in the range of 60-180 nm, as described in WO 2012/030901 A1.

In one embodiment, the polynucleotide, *e.g.,* the circular RNA, according to the present invention is present in a liposome formulation, wherein the RNA-containing liposomes have a net charge close to zero or negative, as disclosed in WO 2013/143555 A1.

In other embodiments, the polynucleotide, *e.g.,* the circular RNA, according to the present invention is present in the form of an emulsion. Emulsions have been previously described to be used for delivery of nucleic acid molecules, such as RNA molecules, to cells. Preferred herein are oil-in-water emulsions. The respective emulsion particles comprise an oil core and a cationic lipid. More preferred are cationic oil-in-water emulsions in which the polynucleotide, *e.g.,* the circular RNA, according to the present invention is complexed to the emulsion particles. The emulsion particles comprise an oil core and a cationic lipid. The cationic lipid can interact with the negatively charged RNA, thereby anchoring the circular RNA to the emulsion particles. In an oil-in-water emulsion, emulsion particles are dispersed in an aqueous continuous phase. For example, the average diameter of the emulsion particles may typically be from about 80 nm to 180 nm. In one embodiment, the pharmaceutical composition of the present invention is a cationic oil-in-water emulsion, wherein the emulsion particles comprise an oil core and a cationic lipid, as described in WO 2012/006380 A2. The polynucleotide, *e.g.,* the RNA, according to the present invention may be present in the form of an emulsion comprising a cationic lipid wherein the N:P ratio of the emulsion is at least 4:1, as described in WO 2013/006834 A1. The polynucleotide, *e.g.,* the RNA according to the present invention may be present in the form of a cationic lipid emulsion, as described in WO 2013/006837 A1. In particular, the composition may comprise RNA complexed with a particle of a cationic oil-in-water emulsion, wherein the ratio of oil/lipid is at least about 8:1 (mole:mole).

In other embodiments, the pharmaceutical composition according to the invention comprises circular RNA in the format of a lipoplex. The term, "lipoplex" or "RNA lipoplex" refers to a complex of lipids and nucleic acids such as RNA. Lipoplexes can be formed of cationic (positively charged) liposomes and the anionic (negatively charged) nucleic acid. The cationic liposomes can also include a neutral "helper" lipid. In the simplest case, the lipoplexes form spontaneously by mixing the nucleic acid with the liposomes with a certain mixing protocol, however various other protocols may be applied. It is understood that electrostatic interactions between positively charged liposomes and negatively charged nucleic acid are the driving force for the lipoplex formation (WO 2013/143555 A1). In one embodiment of the present invention, the net charge of the RNA lipoplex particles is close to zero or negative. It is known that electroneutral or negatively charged lipoplexes of RNA and liposomes lead to substantial RNA expression in spleen dendritic cells (DCs) after systemic administration and are not associated with the elevated toxicity that has been reported for positively charged liposomes and lipoplexes (see WO 2013/143555 A1). Therefore, in one embodiment of the present invention, the pharmaceutical composition according to the invention comprises RNA in the format of nanoparticles, preferably lipoplex nanoparticles, in which (i) the number of positive charges in the nanoparticles does not exceed the number of negative charges in the nanoparticles and/or (ii) the nanoparticles have a neutral or net negative charge and/or (iii) the charge ratio of positive charges to negative charges in the nanoparticles is 1.4:1 or less and/or (iv) the zeta potential of the nanoparticles is 0 or less. As described in WO 2013/143555 A1, zeta potential is a scientific term for electrokinetic potential in colloidal systems. In the present invention, (a) the zeta potential and (b) the charge ratio of the cationic lipid to the RNA in the nanoparticles can both be calculated as disclosed in WO 2013/143555 A1. In summary, pharmaceutical compositions which are nanoparticulate lipoplex formulations with a defined particle size, wherein the net charge of the particles is close to zero or negative, as disclosed in WO 2013/143555 A1, are preferred pharmaceutical compositions in the context of the present invention.

The polynucleotides provided herein, *e.g.,* the circular RNA molecules, and compositions comprising the polynucleotides provided herein, may be used alone or in combination with conventional therapeutic regimens, for example with any other agent or therapy. Such agent or therapy may be useful for treating or preventing the disease, *e.g.,* a viral infection, such as an infection with a Coronavirus. Agent(s) useful for treating or preventing the disease or condition includes, but is not limited to, vaccines, such as DNA vaccines and/or protein vaccines, antigens, antibodies, preferably monoclonal antibodies, antibiotics, antiviral drugs, protease inhibitors, lipids or liposomes, or any combination thereof.

The present inventors surprisingly found that the inhibitory potency of circular versions of antisense sequences surpasses that of traditional antisense strategies based on linear polynucleotides, such as linear RNA polynucleotides. These circular polynucleotides, *e.g.,* circRNAs, can be used as a new class of therapeutics and agents, for example in the context of antiviral therapeutic applications, for targeting a nucleic acid, for forming a complex with a single-stranded nucleic acid, and/or for interfering with genome expression and proliferation of a pathogen. The circular polynucleotides can in addition to (i) a targeting region, which region comprises or consists of a nucleotide sequence complementary to a 5'-UTR, comprise (ii) a region comprising or consisting of a nucleotide sequence capable of forming a stem-loop, which region is linked to the targeting region of (i), and (iii) two linker nucleotide sequences flanking the nucleotide sequence of the targeting region at both sides, *i.e.,* 5' and 3'. These regions and sequences are preferably as described above. These circular polynucleotides can be used in medicine, e.g., in an antiviral therapy.

As used herein, with respect to polynucleotides or nucleic acid molecules, the term "variant" includes degenerate nucleotide sequences, wherein a degenerate nucleotide sequence according to the invention is a nucleotide sequence that differs from a reference nucleotide sequence in codon sequence due to the degeneracy of the genetic code.

Furthermore, a "variant" of a given nucleotide sequence according to the invention includes nucleotide sequences comprising single or multiple such as at least 2, at least 4, or at least 6 and preferably up to 3, up to 4, up to 5, up to 6, up to 10, up to 15, or up to 20 nucleotide substitutions, deletions and/or additions.

Preferably the degree of identity between a given nucleotide sequence and a nucleotide sequence which is a variant of said given nucleotide sequence will be at least 70%, preferably at least 75%, preferably at least 80%, more preferably at least 85%, even more preferably at least 90% or most preferably at least 95%, 96%, 97%, 98% or 99%. The degree of identity is preferably given for a region of at least about 30, at least about 50, at least about 70, at least about 90, at least about 100, at least about 150, at least about 200, at least about 250, at least about 300, or at least about 400 nucleotides. In preferred embodiments, the degree of identity is given for the entire length of the reference nucleotide sequence.

"Sequence identity" between two nucleotide sequences indicates the percentage of nucleotides that are identical between the sequences.

The term "percentage identity" is intended to denote a percentage of nucleotides which are identical between the two sequences to be compared, obtained after the best alignment, this percentage being purely statistical and the differences between the two sequences being distributed randomly and over their entire length. Sequence comparisons between two nucleotide sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Needleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

The present invention is further illustrated by the following figures and examples which are not be construed as limiting the scope of the invention.

### FIGURES

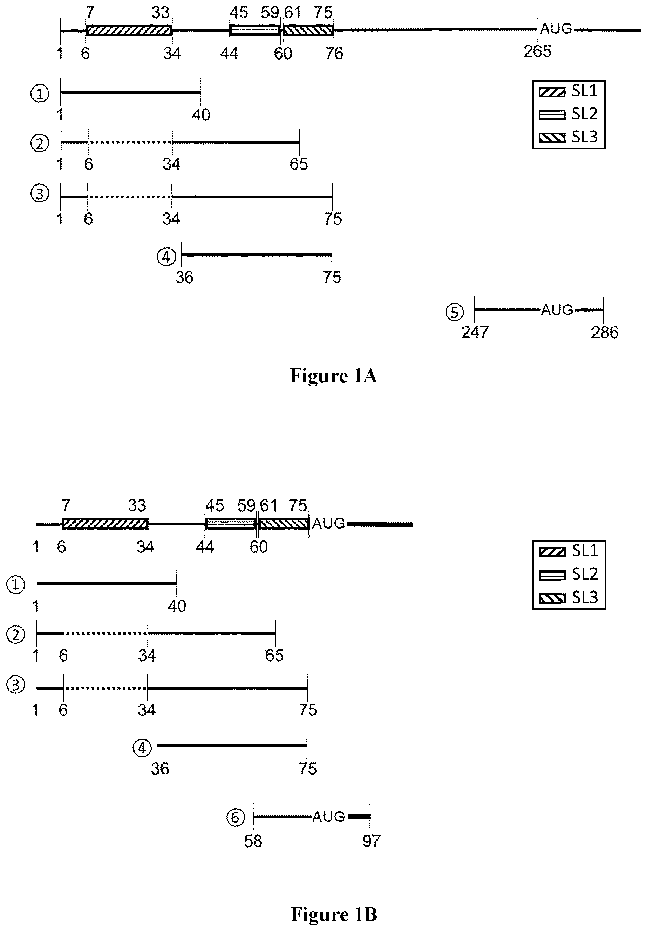

Figure 1A is a schematic representation of the 5'-UTR of the genome of SARS-CoV-2. The 5'-UTR is 265 nucleotides in length. Stem-loops (SL1-SL3) comprised within the 5'-UTR and the respective nucleotide positions are indicated, as well as the portions targeted by the polynucleotides of the present invention. Polynucleotide 1 is referred to herein also as "AS_1-40" (SEQ ID NO: 27), polynucleotide 2 is referred to herein also as "AS_1-65" (SEQ ID NO: 28, polynucleotide 3 is referred to herein also as "AS_1-75" (SEQ ID NO: 29), polynucleotide 4 is referred to herein also as "AS_36-75" (SEQ ID NO: 30), and polynucleotide 5 is referred to herein also as "AS_247-286" (SEQ ID NO: 31).
Figure 1B is a schematic representation of the 5'-leader of subgenomic RNAs (sg-mRNAs) of SARS-CoV-2. The 5'-leader is 75 nucleotides in length and comprises nucleotides 1-75 of the 5'-UTR sequence, including the TRS element at nucleotide positions 70 to 75. Stem-loops (SL1-SL3) comprised within the 5'-UTR and the respective nucleotide positions are indicated, as well as the portions targeted by the polynucleotides of the present invention. Polynucleotide 1 is referred to herein also as "AS_1-40" (SEQ ID NO: 27), polynucleotide 2 is referred to herein also as "AS_1-65" (SEQ ID NO: 28, polynucleotide 3 is referred to herein also as "AS_1-75" (SEQ ID NO: 29), polynucleotide 4 is referred to herein also as "AS_36-75" (SEQ ID NO: 30), and polynucleotide 6 is referred to herein also as "AS_58-97" (SEQ ID NO: 32).

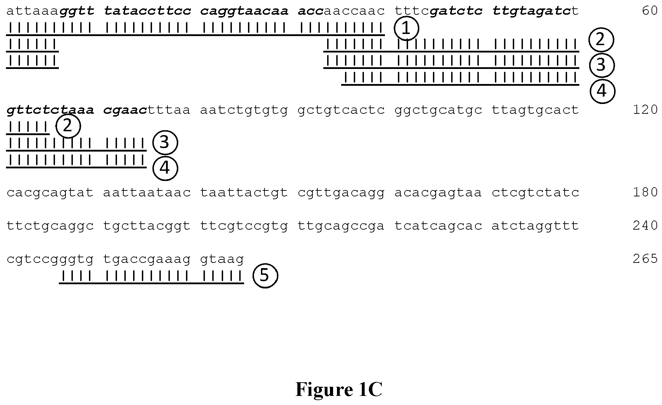

Figure 1C shows the nucleotide sequence of the 5'-UTR of SARS-CoV-2 (SEQ ID NO: 1) and the regions to which the polynucleotides 1 to 5 of the present invention can hybridize. For the polynucleotides the same numbers as in Figure 1A have been used. Within the 5'-UTR sequence (nts 1-265) the SL1 (nts 7-33), the SL2 (nts 45-59) and the SL3 (nts 61-75) are shown in cursive and bold letters.

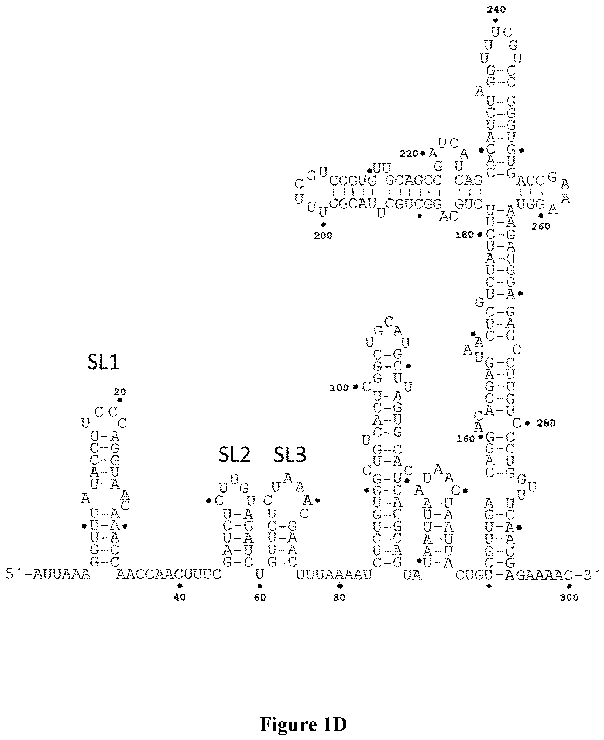

Figure 1D shows a secondary structure model of the 5'-UTR of SARS-CoV-2 (Madhugiri et al., Virology 517 (2018): 44-55; Miao et al., RNA Biol. 23 (2020): 1-10). Stem-loop 1 (SL1), stem-loop 2 (SL2) and stem-loop 3 (SL3) are indicated.

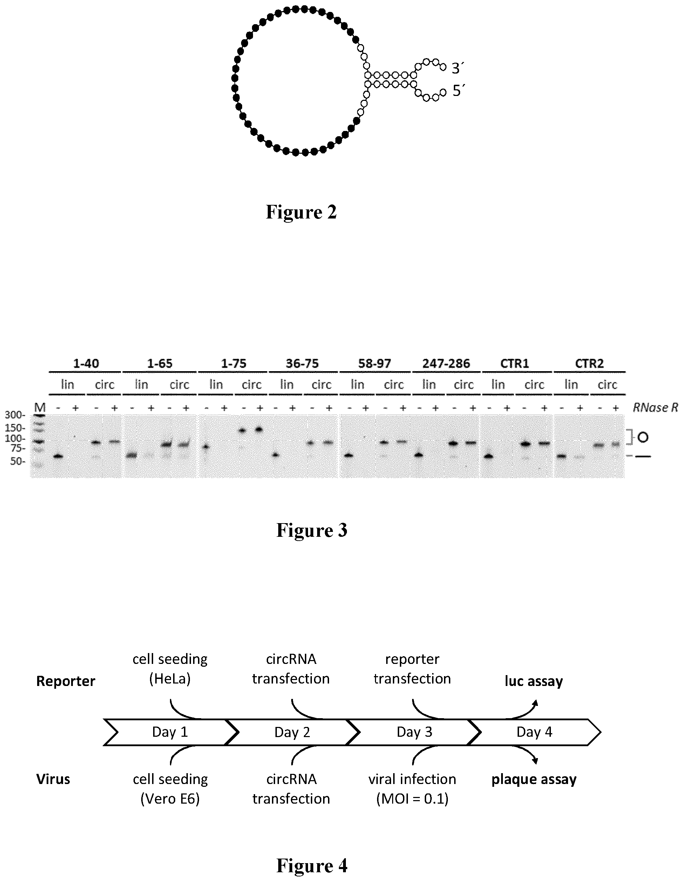

Figure 2 shows the design of circular polynucleotides of the present invention. For example, a circular RNA can be produced by *in vitro* T7 transcription and subsequent circularization by T4 RNA ligase joining the shown 5' and 3' ends together. Each circular polynucleotide is composed of a stem-loop with overhangs for efficient ligation (indicated as "○"), a short stretch of unrelated nucleotides (linker nucleotides) within the big circle for enhanced flexibility (indicated as "○"), and the nucleotides of the targeting region which are indicated as "•" in the big circle.
Figure 3 shows the results of circular RNA (circRNA) synthesis. RNase R treatment and aberrant electrophoretic migration confirm the circularity of the produced circular RNAs. Gel-purified linear and circular RNAs (lin/circ) were treated with RNase R, or left untreated (-/+), and analyzed by denaturing polyacrylamide electrophoresis and ethidium bromide staining. Mobilities of circular (o) and linear (-) forms are marked. *M*, DNA markers (sizes in bp).
Figure 4 shows the experimental workflow for luciferase reporter assays in HeLa cells (top), and viral infections followed by plaque assays in Vero E6 cells (bottom).

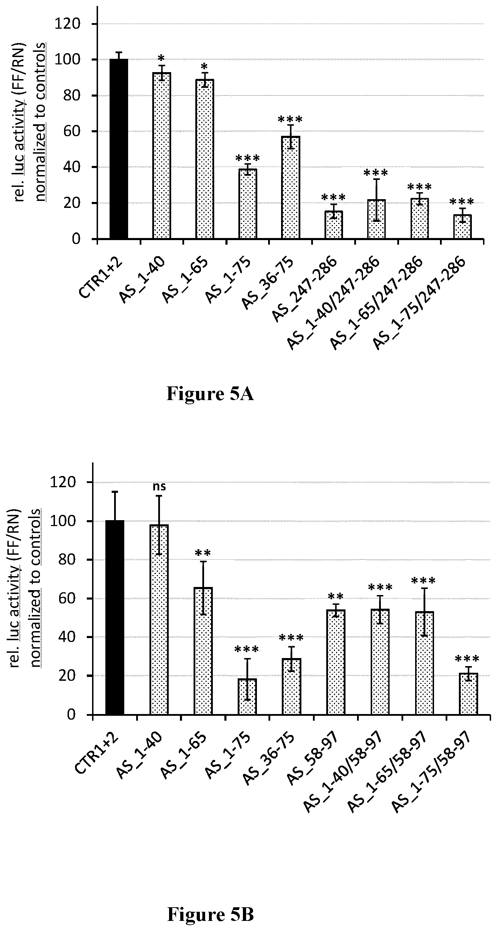

Figure 5A shows the translational repression of a SARS-CoV-2 5'-UTR reporter construct by circular RNAs (AS_1-40, AS_1-65, AS_1-75, AS_36-75, AS_247-286). The 5'-UTR reporter construct comprised the first 265 nucleotides of the SARS-CoV-2 genome including the ORF1a translation start codon and the first 24 nucleotides of ORF1a, fused in-frame with the luciferase ORF. HeLa cells were transfected with the respective circular RNA (as indicated below the diagram) or a combination thereof (e.g. AS_1-40/247-286). After 24 h, the 5-UTR-reporter construct was transfected, and relative luciferase activities (ratio of Firefly and Renilla expression) were measured, normalized to control circular RNAs CTR1 and 2 (mean and standard deviations of three replicates, p < 0.05*, p < 0.005**, p < 0.001***, ns =not significant, two-sided t-test).
Figure 5B shows the translational repression of a SARS-CoV-2 5'-leader reporter construct by circular RNAs (AS_1-40, AS_1-65, AS_1-75, AS_36-75, AS_58-97). The 5-leader-reporter construct comprised the first 75 nucleotides of the SARS-CoV-2 genome (including the TRS element), followed by 25 nucleotides with the AUG start codon and the 5'-terminal coding sequence of the S protein, fused in-frame with the luciferase ORF. HeLa cells were transfected with the respective circular RNA (as indicated below the diagram) or a combination thereof (*e.g.,* AS_1-40/58-97). After 24 h, the 5'-leader reporter construct was transfected, and relative luciferase activities (ratio of Firefly and Renilla expression) were measured, normalized to control circular RNAs CTR1 and 2 (mean and standard deviations of three replicates, p < 0.05*, p < 0.005**, p < 0.001***, ns =not significant, two-sided t-test).

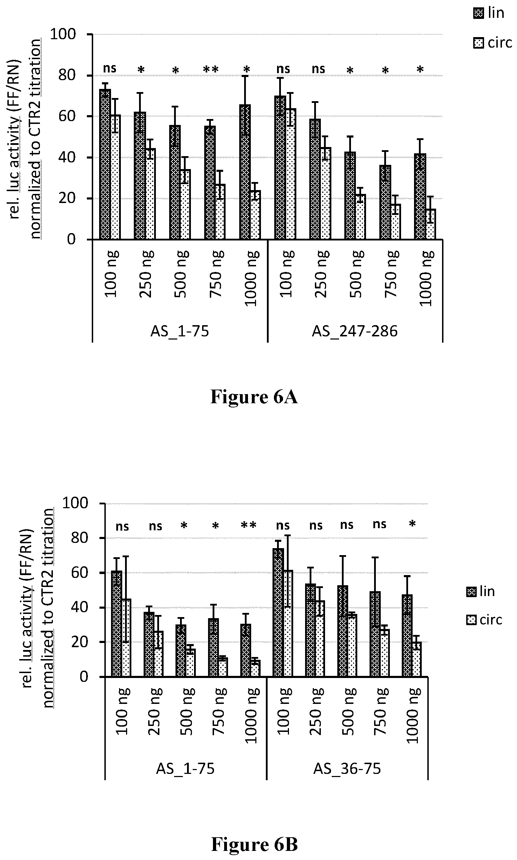

Figure 6A shows dosage dependent effects of linear versus circular RNAs on reporter expression. HeLa cells were transfected with increasing amounts (100 to 1000 ng per assay) of circular RNAs, or their linear counterparts. After 24 h, the 5'-UTR reporter construct was transfected, and relative luciferase activities (Firefly/Renilla expression ratios) were measured, normalized to control circular RNA CTR2 (mean and standard deviations of three replicates, p < 0.05*, p < 0.005**, p < 0.001***, ns =not significant, two-sided t-test).
Figure 6B shows dosage dependent effects of linear versus circular RNAs on reporter expression. HeLa cells were transfected with increasing amounts (100 to 1000 ng per assay) of circular RNAs, or their linear counterparts. After 24 h, the 5'-leader reporter construct was transfected, and relative luciferase activities (Firefly/Renilla expression ratios) were measured, normalized to control circular RNA CTR2 (mean and standard deviations of three replicates, p < 0.05*, p < 0.005**, p < 0.001***, ns =not significant, two-sided t-test).

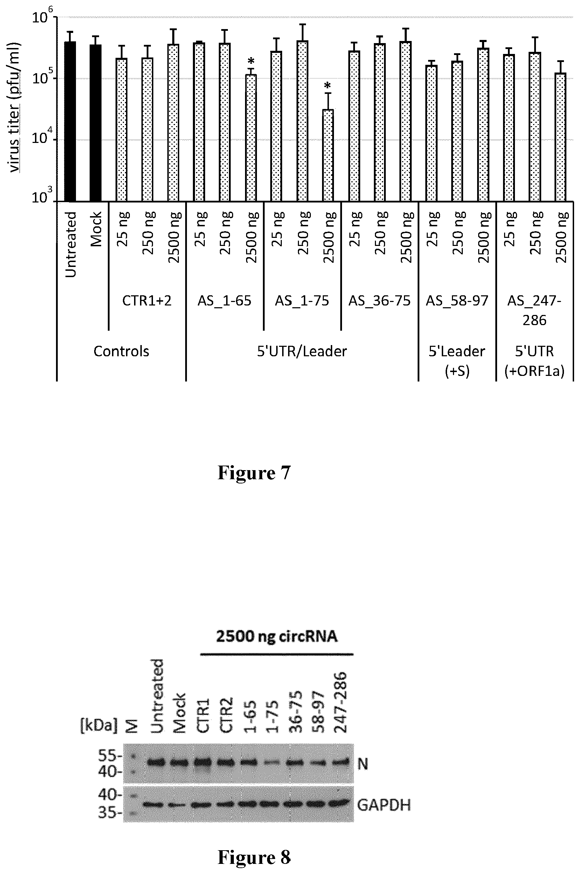

Figure 7 shows the inhibition of SARS-CoV-2 proliferation by circular RNAs of the present invention, namely AS_1-65, AS_1-75, AS_36-75, AS_58-97, AS_247-286 of Example 1. For the virus titer assays Vero E6 cells were transfected with increasing quantities of the circular RNAs (25, 250 and 2500 ng per assay). After 24 h, cells were infected by SARS-CoV-2 (MOI = 0.1). The effects on virus titers were measured by plaque assays 24 h post infection (mean and standard deviations of three experiments, p < 0.05*, ns =not significant, two-sided t-test). Untreated (without RNA and transfection reagent) and mock-treated cells (without RNA, but with transfection reagent) served as controls.
Figure 8 shows viral protein synthesis assays: Western blot analysis of the viral nucleocapsid protein (N) confirms reduction of the virus titer observed. Vero E6 cells transfected with 2500 ng of respective circular RNAs (AS_1-65, AS_1-75, AS_36-75, AS_58-97, AS_247-286) per assay were harvested, lysed and equal amounts of protein analyzed by Western blot, detecting the nucleocapsid protein as a marker for viral replication, and using GAPDH as loading control. *M*, protein markers (sizes in kDa).

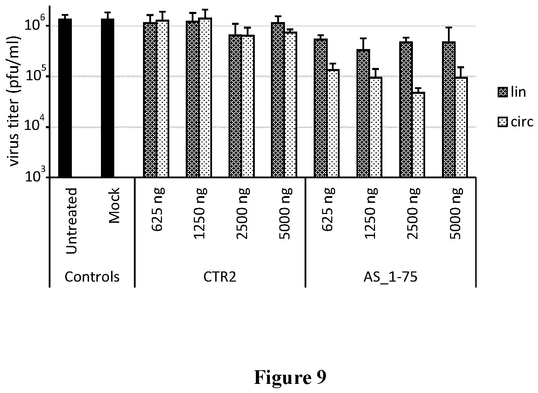

Figure 9 shows the dose dependence of antiviral effect of the circular RNA "AS_1-75" of Example 1, in comparison to its linear counterpart. Vero E6 cells were transfected with increasing amounts (625, 1250, 2500 and 5000 ng per assay) of circular AS_1-75, or of its linear counterpart, followed by viral infection (MOI = 0.1) after 24 h, and plaque assays of virus titer 24 h post infection (mean and standard deviations of three experiments, p < 0.05*, ns=not significant, two-sided t-test). As controls, untreated (without RNA and transfection reagent) and mock-treated cells (without RNA, but with transfection reagent) were used, as well as transfections with linear or circular control RNA (CTR2).

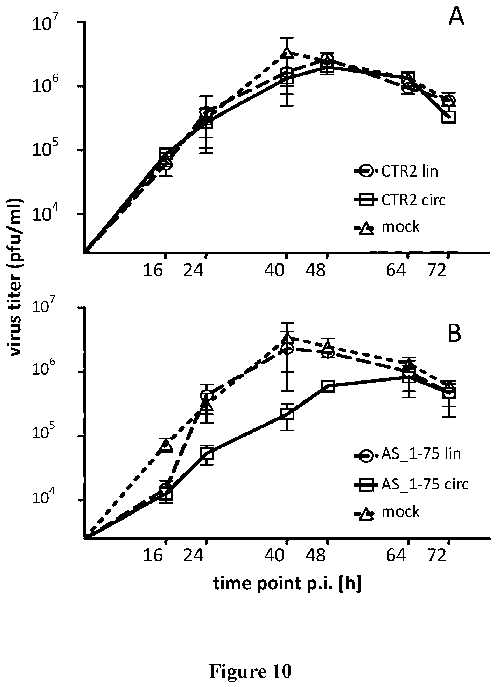

Figure 10 shows the durability of antiviral activity of AS-1-75 circRNA. Vero E6 cells were transfected with circRNA AS_1-75 or its linear counterpart (Figure 10B), followed by viral infection (MOI = 0.1) after 24 h, and plaque assays of virus titer at time points indicated (16 to 72 h post infection). As controls, untreated cells (without RNA and transfection reagent) and mock-treated cells (without RNA, but with transfection reagent) were used, as well as transfections with linear or circular control RNA (Figure 10A).

### EXAMPLES

### Example 1: In vitro transcription, circularization and purification of small antisense-RNAs

RNAs were transcribed from annealed DNA-oligonucleotide templates, using the HighScribe^{™} T7 high-yield RNA synthesis kit (NEB) in the presence of ATP, CTP, UTP, and GTP (each at 7.5 mM), GMP (30 mM GMP; Merck), and RNaseOut (Thermo Fisher Scientific) for 2 h at 37°C. The DNA template was digested by addition of RQ1 DNase (2 U per reaction, Promega), and incubation for 30 min at 37°C. Transcripts were purified using the Monarch RNA purification kit (NEB) and quantified by the Qubit^{™} RNA broad-range assay kit (Thermo Fisher Scientific).

For circularization, 60 µg transcribed RNA was incubated with 100 units of T4 RNA ligase (Thermo Fisher Scientific) in 1x T4 RNA ligase buffer, supplemented with 0.1 mg/ml BSA and RNaseOut (Thermo Fisher Scientific), overnight at 16°C in a final volume of 200 µl. RNA was recovered by phenol/chloroform extraction (Roth) and ethanol precipitation. RNA quality and circularization efficiency were verified by denaturing polyacrylamide gel electrophoresis and RNase R digestion (Biozym).

The *in vitro* synthesized small antisense-RNAs were gel purified as described for example in Breuer and Rossbach, Methods Protoc. 3 (2020): 42). To validate the circular conformation, 250 ng of gel-purified circular or linear RNA was incubated with or without 2 U of RNase R (Biozym; 30 min at 37°C). After digestion, 200 ng of RNAs were separated in a 10% denaturing polyacrylamide gel and visualized by ethidium bromide staining.

A series of six antisense circular RNAs (AS-circRNAs), two control sequences, and respective linear counterparts were produced:
AS_1-40 (SEQ ID NO: 27):
   GGGAGTAAGC*AAA***GTTGGTTGGTTTGTTACCTGGGAAGGTATAAACCTTTAAT***AAA*GCTTACAGTA
AS_1-65 (SEQ ID NO: 28):
   GGGAGTAAGC*AAA***AGAACAGATCTACAAGAGATCGAAAGTTGGTTaaTTTAAT***AAA*GCTTACAGTA
AS_1-75 (SEQ ID NO: 29): 
GGGAGTAAGCAAAGTTCGTTTAGAGAACAGATCTACAAGAGATCGAAAGTTGGTTaaTTTAATAAAGCTT
ACAGTA
AS_36-75 (SEQ ID NO: 30):
   GGGAGTAAGC*AAA***GTTCGTTTAGAGAACAGATCTACAAGAGATCGAAAGTTGG***AAA*GCTTACAGTA
AS_247-286 (SEQ ID NO: 31):
   GGGAGTAAGC*AAA***ACCAGGGACAAGGCTCTCCATCTTACCTTTCGGTCACACC***AAA*GCTTACAGTA
AS_58-97 (SEQ ID NO: 32):
   GGGAGTAAGC*AAA***TAAAACAAGAAAAACAAACATTGTTCGTTTAGAGAACAGA***AAA*GCTTACAGTA
CTR1 (SEQ ID NO: 33):
   GGGAGTAAGC*AAA***AGATGCGCACCGCACAGATGCGCACCACGCCACGCGTAGA***AAA*GCTTACAGTA
CTR2 (SEQ ID NO: 34):
   GGGAGTAAGC*AAA***AGTCAGTGAGTCAGTGTAATACGGGAGGATACCCGCTGTC***AAA*GCTTACAGTA

The six antisense RNAs between 66 and 76 nucleotides (nts) in length and with a targeting region of 40 to 50 nucleotides in length were designed to specifically target the SARS-CoV-2 5'-UTR regions. In particular, "AS_1-40" targets the 5'-terminal 40 nucleotides of the SARS-CoV-2 genome (and sgRNAs) including a region capable of forming a stem-loop 1 (SL1) as a secondary structure. "AS_1-65" targets 65 nucleotides at the 5' terminus, but omits the complete 27-nucleotide SL1 sequence. "AS_1-75" targets the same nucleotides as "AS_1-65" plus additional ten nucleotides, including the TRS element. "AS_36-75" targets the single-stranded region between SL1 and a region capable of forming a stem-loop 2 (SL2) in secondary structure, and regions capable of forming stem-loop 2 and stem-loop 3 (SL3) in secondary structure, including the TRS element. These four AS-circRNAs have in common that they can establish base-pairing interactions with both the genome RNA and the eight major sgRNAs (sgRNAs 2-9) produced in SARS-CoV-2-infected cells.

The antisense RNA "AS_247-286" targets the 3'-terminal region of the genomic 5'-UTR region and the first 21 nucleotides of ORF1a, while "AS_58-97" targets a region capable to form stem-loop 3 (SL3), the TRS element and the first 21 nucleotides of the S-protein ORF.

The two control sequences (CTR1 and CTR2) are nonspecific circular RNAs, each comprising a randomized sequence of 40 nucleotides instead of the SARS-CoV-2-specific nucleotide sequences.

Each circular RNA was designed such that the targeting sequence (shown in bold letters in the sequences shown in this example) was linked to a common short stem-loop comprising 6 perfect base-pairs (underlined letters in the above sequences) with two overhanging ends that were joined by ligation. The targeting region and the stem-loop are connected by two three-nucleotide linkers (shown in cursive letters in the sequences of this example), to allow a more flexible presentation of the targeting sequence.

For quality control and evidence of circularity, purified AS-circRNAs (as well as their linear counterparts) were characterized by denaturing PAGE (as shown in Figure 3). All of these relatively short circRNAs are RNase R resistant, in contrast to the corresponding linear RNAs, and the circular configuration results in slower mobility relative to the linear form, demonstrating circularity.

### Example 2: Transfection of AS-circRNAs and luciferase reporter assays

To functionally characterize the six antisense circular RNAs of Example 1, their ability to inhibit translation in two luciferase reporter systems (for a flowchart of analysis, see Figure 4) was tested.

Two luciferase reporter constructs were prepared. For these reporter constructs the 5'-UTR (nts 1-265) or the 5'-leader (nts 1-75) sequences of SARS-CoV-2 (NC_045512.2) was cloned into pcDNA5-CMV-FF (Medenbach J., et al. Cell 145 (2011): 902-913) containing the Firefly reporter ORF. Additionally, for the 5'-UTR construct, 24 nucleotides of the ORF1a sequence were included, and, for the 5'-leader construct, 25 nucleotides of the S (spike) ORF were included, followed in either reporter by the Firefly ORF.

HeLa cells were cultured in DME-medium supplemented with 10% FBS (Gibco) at 37°C and 5% CO₂. For luciferase reporter assays, 1x10⁵ cells were seeded per well (12-well plate). For RNA transfections, Lipofectamine 2000 was used (Thermo Fisher Scientific). For AS-circRNA screening, cells were transfected either with 1 µg of individual circular RNAs, or with a combination of two of them (0.5 µg of each). For titration experiments, different amounts (100, 250, 500, 750, 1000 ng) of circular or corresponding linear RNAs (CTR2 and AS_1-75 RNA) were used. Culture medium was always changed 1 h prior to and 4 h after transfection. After one day, cells were cotransfected with 50 ng of 5'-UTR or 5'-leader containing reporter plasmids, based on pcDNA5-CMV-FF Firefly plasmid DNA, together with 5 ng of pRL-SCV40 Renilla-reporter (Promega). 24 h post transfection, cells were washed three times with PBS (Gibco), and lysed in 250 µl Lysis-Juice (PJK). Luminescence was measured for Firefly and Renilla luciferase (Beetle- and Renilla-Juice kits, respectively; PJK), using a Centro LB 960 Luminometer (Berthold Technologies) and calculated in relation to appropriate controls (Figure 5).

The relative luciferase activities were calculated as a ratio of the Firefly and Renilla raw values, with three technical replicates per sample and a total of three independent biological replicates.

Comparing the effects of AS-circRNAs on the two reporters, 5'-UTR versus 5'-leader, corresponding effects were observed for the first four AS-circRNAs (AS_1-40, AS_1-65, AS_1-75, and AS_36-75): Addressing the very 5'-end, only in case of the longest version, AS_1-75, which excludes stem-loop 1, both reporters were strongly reduced in translation (to 39 and 18% residual activity, respectively). The two shorter AS-circRNAs (AS_1-40 and AS_1-65) showed only small or insignificant effects. The strong effect of AS_1-75 is not simply due to extended base-pairing, since the shorter AS_36-75 had an effect almost as strong as AS-1-75: reduction to 57 and 29%, respectively, for the two reporters. Moving from the 5'-end to the region overlapping the translation start codon, that is AS_247-286 and AS_58-97 for the 5'-UTR or 5'-leader reporters, respectively, also had strong effects on reporter activity (reduction to 15 and 54%, respectively). The use of AS-circRNAs (AS_247-286 and AS_58-97) targeting the AUG start codon region (of ORF1a and S, respectively) in combination with circRNAs targeting the 5'-terminal leader region did not further increase the inhibitory effect on reporter translation (see three combinations for each of the two reporters, Figure 5).

To address to question of whether the circular configuration of the AS-circRNA enhances the inhibitory activity, the two AS-circRNAs causing the most profound inhibitory effects on reporter activity were compared with their linear counterparts: AS_1-75 and AS_247-286, using the 5'-UTR reporter, and AS_1-75 and AS_36-75, using the 5'-leader reporter (Figure 6).

Using the same series of assays a dosage dependent effect for selected circRNAs was measured, with doses ranging between 100 and 1000 ng per assay. For each of the four setups, dosage dependent effects were observed, in particular for the circular configuration. Maximal effects were attained with 750 to 1000 ng of AScircRNA per assay, whereas the inhibitory effect with linear counterparts leveled off at 500 ng and more. The circRNAs were consistently more potent than their linear counterparts. At higher concentrations, the circRNA caused a more than 2-fold stronger reduction of reporter activity than the corresponding linear RNA. This superior inhibitory efficacy of the AS-circRNAs may be due to differential stabilities or intracellular localizations of the transfected RNAs, their intrinsic antisense activity, base-pairing potential, structural properties, or a combination thereof.

### Example 3: Viral infection and plaque assays

In order to analyze the effect of the sequences identified in the reporter assays of Example 2 on viral proliferation, the series of AS-circRNAs, except for AS_1-40, of Example 1 were transfected in Vero E6 cells, which are permissible for SARS-CoV-2 infection, and infectious virus progenies were produced.

Vero E6 cells were cultured in DME-medium supplemented with 10% FBS and 100 U/ml Penicillin-Streptomycin (Gibco) at 37°C and 5% CO₂. Cells were seeded with a density of 0.5 x 10⁵ per well (24-well plate). RNA transfections were done using Lipofectamine 2000 (Thermo Fisher Scientific). For AS-circRNA screening, cells were transfected either with increasing amounts (25, 250, and 2500 ng per assay (see Figure 7)) of individual circular RNAs, or their linear counterpart.

Culture medium was exchanged 1 h prior and 4 h after transfection. 24 h post transfection, cells were inoculated with SARS-CoV-2 at a multiplicity of infection (MOI) of 0.1 at 33°C. At 1h post infection, the inoculum was replaced with fresh medium.

Virus-containing supernatants were collected 24 h post infection, and virus titers were determined by plaque assays (Müller C., et al., Antiviral Res. 150 (2018): 123-129) to assess the antiviral effects of individual AS-circRNAs on viral replication in cell culture (for a flowchart, see Figure 4).

Compared to the control treatments (untreated cells, mock-infected cells, CTR1 and CTR2 at the three different doses), the three AS-circRNAs targeting the leader region of the 5'-UTR (AS_1-65, AS_1-75 and AS_36-75), which is present on both genomic and subgenomic RNAs, differentially affected virus titers: The strongest effect on viral proliferation was measured for AS_1-75, a reduction to 9% of control level, *i.e.,* indicated as "percent remaining" relative to mock treatment, consistent with the strong effect observed in the previous reporter assays (compare Figure 7 and Figure 5). The shortened version, AS_1-65, still had a moderate, but significant effect on virus titers (reduction to 33% compared to untreated control cells infected with SARS-CoV-2), whereas AS_36-75 had no significant effect on virus replication in cell culture (Figure 7). In contrast, AS_58-97, and AS_247-286 did not significantly reduce virus titers at circRNA amounts of up to 2500 ng.

### Example 4: Western blot

To obtain additional evidence for inhibition of viral replication, viral protein synthesis was measured by Western blotting, using a SARS-CoV-2 nucleocapsid protein (N)-specific antibody.

The viral protein accumulation was analyzed by Western blot of Vero E6 cell lysates, previously transfected with 2.5 µg of circRNAs (AS_1-65, AS_1-75, AS_36-75, AS_58-97 and AS_247-286 of Example 1) and infected with SARS-CoV-2 at a MOI of 0.1 pfu/cell. Total protein lysates obtained at 24 hpi were heat-denatured in SDS loading buffer (50 mM Tris-HCl, pH 6.8, 2% SDS, 10% glycerol, 2.5% 2-mercaptoethanol, and 0.05% bromophenol blue) at 95°C for 10 min. Following separation by SDS-polyacrylamide gel electrophoresis (PAGE; 10%), the proteins were blotted onto a nitrocellulose membrane (BioRad). Viral proteins were immunostained overnight with rabbit anti-SARS nucleocapsid protein (N, 200-401-A50, Rockland, 1:500) or mouse anti-GAPDH antibody (Sigma, 1:5000) and appropriate secondary antibodies.

The data shown in Figure 8 revealed a strongly reduced accumulation of viral N protein in cells treated with AS_1-75 circRNA, supporting the observed inhibitory effects of this particular circRNA on the production of infectious virus progeny.

### Example 5: Dose dependence of antiviral effect of AS-circRNA in comparison to its linear counterpart

Vero E6 cells were transfected with increasing amounts (625, 1250, 2500 and 5000 ng) of circRNA AS_1-75 of Example 1, or of its linear counterpart, followed by viral infection (MOI = 0.1) after 24 h, and plaque assays of virus titer 24 h post infection (mean and standard deviations of three experiments, p < 0.05*, ns=not significant, two-sided t-test). As controls, untreated (without RNA and transfection reagent) and mock-treated cells (without RNA, but with transfection reagent) were used, as well as transfections with linear or circular control RNA (CTR2 of Example 1). The virus titer of significantly affected samples is indicated as "percent remaining", relative to mock treatment.

As can be seen in Figure 9 and Table 6 below there is a dosage dependent strong antiviral effect of AS_1-75 circRNA.

**Table 6: Virus titer**

| Amount of AS_1-75 [ng] | Configuration of AS_1-75 | Virus titer of significantly affected samples, given as percent remaining, relative to mock treatment |
|---|---|---|
| 625 | linear | |
| 625 | circular | 10 |
| 1,250 | linear | 25 |
| 1,250 | circular | 7 |
| 2,500 | linear | 35 |
| 2,500 | circular | 4 |
| 5,000 | linear | |
| 5,000 | circular | 7 |

Applying circular AS_1-75 in an amount of 2,500 ng reduced the virus titer down to 4%. At an even higher dose (5000 ng), virus titers were reduced to 7%. In contrast, control AS-circRNA had no significant effect, as can be seen also in Table 7, below.

**Table 7:**

| Polynucleotide | Amount [ng] | linear vs. circular | linear compared to mock | circular compared to mock |
|---|---|---|---|---|
| AS_1-75 | 625 | * | ns | * |
| | 1,250 | ns | * | * |
| | 2,500 | * | * | * |
| | 5,000 | ns | ns | * |
| CTR2 | 625 | ns | ns | ns |
| | 1,250 | ns | ns | ns |
| | 2,500 | ns | ns | ns |
| | 5,000 | ns | ns | ns |

Comparing the effects of circular versus linear RNAs, for all quantities assayed the circRNA was clearly superior to the corresponding linear version, consistent with our results from reporter assays (see Example 3 and Figure 6).

### Example 6: Durability of AS-circRNA in comparison to its linear counterpart

In addition to determining the virus titer 24 h post-infection (cf. Example 5), it was tested whether the antiviral effect of AS_1-75 circRNA persisted for longer time periods. As can be seen in Figure 10, also, virus titers were determined at six time points post-infection, namely at 16h, 24h, 40 h, 48h, 64h and 72h, comparing the effects of AS_1-75 linear and circRNAs, as well as mock and negative-control RNA transfections. Whereas both linear and circular control RNA (CTR2), as well as mock transfection did not significantly differ in their development of virus titers over this time frame, AS_1-75 circRNA strongly reduced virus titers, particularly within the time window from 24 to 48 h. This antiviral effect was specific for the circular configuration and indicates durability of the effect over at least two days under these cell culture conditions.

The present invention is particularly directed to the following items:
1. A polynucleotide comprising a nucleotide sequence, which sequence comprises a targeting region comprising or consisting of a nucleotide sequence complementary to a 5'-untranslated region (5'-UTR) of a Coronavirus or to a continuous or discontinuous portion of said 5' untranslated region.
2. The polynucleotide of item 1, wherein the polynucleotide is a linear, preferably circular polynucleotide.
3. The polynucleotide of item 1 or 2, wherein the polynucleotide comprises at least one ribonucleotide.
4. The polynucleotide of any one of items 1 to 3, wherein the polynucleotide consists of ribonucleotides.
5. The polynucleotide of item 1 or 3, wherein the polynucleotide comprises at least one deoxyribonucleotide.
6. The polynucleotide of any one of items 1 to 3 or 5, wherein the polynucleotide comprises a mixture of ribonucleotides and deoxyribonucleotides.
7. The polynucleotide of any one of items 1 to 6, wherein the polynucleotide comprises at least one modified nucleotide.
8. The polynucleotide of any one of items 1 to 7, wherein the nucleotide sequence of the targeting region is complementary to at least one region of the 5'-UTR capable of forming a stem-loop.
9. The polynucleotide of item 8, wherein the region of the 5'-UTR capable of forming a stem-loop is selected from the group consisting of stem-loop 1, stem-loop 2 and stem-loop 3.
10. The polynucleotide of item 8 or 9, wherein the nucleotide sequence of the targeting region comprises or consists of a nucleotide sequence complementary to stem-loop 2 and/or stem-loop 3.
11. The polynucleotide of any one of items 8 to 10, wherein the nucleotide sequence of the targeting region does not comprise a nucleotide sequence complementary to stem-loop 1.
12. The polynucleotide of any one of items 8 to 10, wherein the nucleotide sequence of the targeting region comprises or consists of a nucleotide sequence complementary to stem-loop 1.
13. The polynucleotide of any one of items 1 to 12, wherein the nucleotide sequence of the 5'-untranslated region is depicted in SEQ ID NO: 1.
14. The polynucleotide of any one of items 1 to 13, wherein the nucleotide sequence of the targeting region is complementary to nucleotides 1 to 85 of the sequence depicted in SEQ ID NO: 1 or to a continuous or discontinuous portion of said sequence.
15. The polynucleotide of any one of items 1 to 14, wherein the nucleotide sequence of the targeting region is complementary to nucleotides 1 to 75 of the sequence depicted in SEQ ID NO: 1 or to a continuous or discontinuous portion of said sequence.
16. The polynucleotide of any one of items 1 to 13, wherein the nucleotide sequence of the targeting region is complementary to nucleotides 247 to 265 of the sequence depicted in SEQ ID NO: 1 or to a continuous or discontinuous portion of said sequence.
17. The polynucleotide of any one of items 1 to 15, wherein the nucleotide sequence of the targeting region is complementary to at least one continuous portion of the 5'-untranslated region, the continuous portion being selected from the group consisting of:
   (i) nucleotides 1 to 75 of the sequence depicted in SEQ ID NO: 1;
   (ii) nucleotides 1 to 65 of the sequence depicted in SEQ ID NO: 1;
   (iii) nucleotides 1 to 40 of the sequence depicted in SEQ ID NO: 1;
   (iv) nucleotides 36 to 75 of the sequence depicted in SEQ ID NO: 1;
   (v) nucleotides 45 to 75 of the sequence depicted in SEQ ID NO: 1;
   (vi) nucleotides 58 to 75 of the sequence depicted in SEQ ID NO: 1
   (vii) nucleotides 61 to 75 of the sequence depicted in SEQ ID NO: 1;
   (viii) nucleotides 70 to 75 of the sequence depicted in SEQ ID NO: 1;
   (ix) nucleotides 45 to 59 of the sequence depicted in SEQ ID NO: 1;
   (x) nucleotides 1 to 44 of the sequence depicted in SEQ ID NO: 1;
   (xi) nucleotides 34 to 44 of the sequence depicted in SEQ ID NO: 1;
   (xii) nucleotides 1 to 6 of the sequence depicted in SEQ ID NO: 1; and
   (xiii) nucleotides 7 to 33 of the sequence depicted in SEQ ID NO: 1.
18. The polynucleotide of any one of items 1 to 17, wherein the nucleotide sequence of the targeting region comprises at least two segments, each segment complementary to a nucleotide sequence of the 5'-UTR, wherein the complementary nucleotide sequences of the 5'-UTR are not continuous.
19. The polynucleotide of item 18, wherein
   (i) the first segment of the nucleotide sequence of targeting region is complementary to nucleotides 1 to 6 and the second segment is complementary to nucleotides 34 to 44 of the sequence depicted in SEQ ID NO: 1;
   (ii) the first segment of the nucleotide sequence of targeting region is complementary to nucleotides 45 to 59 and the second segment is complementary to nucleotides 61 to 75 of the sequence depicted in SEQ ID NO: 1;
   (iii) the first segment of the nucleotide sequence of targeting region is complementary to nucleotides 36 to 59 and the second segment is complementary to nucleotides 61 to 75 of the sequence depicted in SEQ ID NO: 1;
   (iv) the first segment of the nucleotide sequence of targeting region is complementary to nucleotides 1 to 6 and the second segment is complementary to nucleotides 34 to 59 of the sequence depicted in SEQ ID NO: 1; or
   (v) the first segment of the nucleotide sequence of targeting region is complementary to nucleotides 1 to 6, the second segment is complementary to nucleotides 34 to 59 and the third segment is complementary to nucleotides 61 to 75 of the sequence depicted in SEQ ID NO: 1.
20. The polynucleotide of item 18, wherein the nucleotide sequence of the targeting region comprises one or more nucleotides between the at least two segments, or the polynucleotide of item 19, wherein the nucleotide sequence of the targeting region comprises one or more nucleotides between the first segment and the second segment and/or between the second segment and the third segment.
21. The polynucleotide of item 20, wherein the one or more nucleotides between the two segments are two nucleotides.
22. The polynucleotide of item 21, wherein the two nucleotides are two adenine nucleotides.
23. The polynucleotide of item 20, wherein the nucleotide between the two segments is a single nucleotide.
24. The polynucleotide of any one of items 18 to 22, wherein two nucleotides are between the nucleotides of the first segment complementary to nucleotides 1 to 6 and the second segment complementary to nucleotides 34 to 44 of the sequence depicted in SEQ ID NO: 1, which two nucleotides are each not complementary to nucleotides 7 and 33, respectively, of the sequence depicted in SEQ ID NO: 1.
25. The polynucleotide of any one of items 18 to 24, wherein one nucleotide is between the nucleotides of the first segment complementary to nucleotides 45 to 59 and the second segment complementary to nucleotides 61 to 75 of the sequence depicted in SEQ ID NO: 1, which nucleotide is complementary or non-complementary to nucleotide 60 of the sequence depicted in SEQ ID NO: 1.
26. The polynucleotide of any one of items 1 to 25, wherein the nucleotide sequence of the targeting region is complementary to at least nucleotides 45 to 59 of the sequence depicted in SEQ ID NO: 1.
27. The polynucleotide of any one of items 1 to 25, wherein the nucleotide sequence of the targeting region is complementary to at least nucleotides 61 to 75 of the sequence depicted in SEQ ID NO: 1.
28. The polynucleotide of item 26 or 27, wherein the targeting region is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary.
29. The polynucleotide of item 26 or 28, wherein the nucleotide sequence of the targeting region complementary to at least nucleotides 45 to 59 of the sequence depicted in SEQ ID NO: 1 is flanked 3' by one or more additional nucleotides for targeting the nucleotide at position 44 up to at most to the nucleotide at position 1 of the sequence depicted in SEQ ID NO: 1.
30. The polynucleotide of item 29, wherein the nucleotide sequence of the targeting region complementary to at least nucleotides 45 to 59 of the sequence depicted in SEQ ID NO: 1 is flanked 3' by one or more additional nucleotides for targeting the nucleotide at position 44 up to at least to the nucleotide at position 34 of the sequence depicted in SEQ ID NO: 1.
31. The polynucleotide of item 26 or 29, wherein the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and one or more nucleotides at position 6 up to at most the nucleotide at position 1 of the sequence depicted in SEQ ID NO: 1.
32. The polynucleotide of item 31, wherein the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 2 of the sequence depicted in SEQ ID NO: 1.
33. The polynucleotide of item 31, wherein the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 3 of the sequence depicted in SEQ ID NO: 1.
34. The polynucleotide of item 31, wherein the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 4 of the sequence depicted in SEQ ID NO: 1.
35. The polynucleotide of item 31, wherein the nucleotide sequence of the targeting region complementary to at least nucleotides 45 to 59 of the sequence depicted in SEQ ID NO: 1 is flanked 3' by additional nucleotides for targeting the nucleotides at positions 44 to 34 and nucleotides at position 6 to 5 of the sequence depicted in SEQ ID NO: 1.
36. The polynucleotide of any one of items 31 to 35, wherein between the nucleotides for targeting the nucleotides at positions 44 to 34 and the nucleotides for targeting the nucleotides at position 6 up to at most the nucleotide at position 1 of the sequence depicted in SEQ ID NO: 1 a non-complementary sequence of two or more nucleotides is inserted.
37. The polynucleotide of item 36, wherein two nucleotides are inserted in between the said nucleotide sequences.
38. The polynucleotide of item 37, wherein two adenine nucleotides are inserted.
39. The polynucleotide of item 26 or any one of items 28 to 38, wherein the nucleotide sequence of the targeting region is depicted in any one of SEQ ID NO: 2 to SEQ ID: 9.
40. The polynucleotide of item 27, wherein the nucleotide sequence of the targeting region complementary to at least nucleotides 61 to 75 of the sequence depicted in SEQ ID NO: 1 is flanked 3' by one or more additional nucleotides for targeting the nucleotide at position 60 up to at most to the nucleotide at position 45 of the sequence depicted in SEQ ID NO: 1.
41. The polynucleotide of item 40, wherein the nucleotide sequence of the targeting region complementary to at least nucleotides 61 to 75 of the sequence depicted in SEQ ID NO: 1 is flanked 3' by one or more additional nucleotides for targeting the nucleotides at position 60 to 58 of the sequence depicted in SEQ ID NO: 1.
42. The polynucleotide of items 28 to 41, wherein the nucleotide sequence of the targeting region complementary to at least nucleotides 45 to 59 of the sequence depicted in SEQ ID NO: 1 is flanked 5' by one or more additional nucleotides for targeting the nucleotide at position 60 up to at most to the nucleotide at position 85 of the sequence depicted in SEQ ID NO: 1.
43. The polynucleotide of item 42, wherein the nucleotide sequence of the targeting region complementary to at least nucleotides 45 to 59 of the sequence depicted in SEQ ID NO: 1 is flanked 5' by one or more additional nucleotides for targeting the nucleotide at position 60 up to at most to the nucleotide at position 75 of the sequence depicted in SEQ ID NO: 1.
44. The polynucleotide of item 42 or 43, wherein the nucleotide sequence of the targeting region is depicted in any one of SEQ ID NO: 10 to SEQ ID: 20.
45. The polynucleotide of any one of items 1 to 44, wherein the nucleotide sequence of the targeting region is complementary to nucleotides 1 to 40 of the sequence depicted in SEQ ID NO: 1.
46. The polynucleotide of item 45, wherein the nucleotide sequence of the targeting region is depicted in SEQ ID NO: 24.
47. The polynucleotide of any one of items 1 to 44, wherein the nucleotide sequence of the targeting region is complementary to nucleotides 1 to 6 and 34 to 65 of the sequence depicted in SEQ ID NO: 1.
48. The polynucleotide of item 47, wherein the nucleotide sequence of the targeting region is depicted in SEQ ID NO: 21.
49. The polynucleotide of any one of items 1 to 44, wherein the nucleotide sequence of the targeting region is complementary to nucleotides 1 to 6 and 34 to 75 of the sequence depicted in SEQ ID NO: 1.
50. The polynucleotide of item 49, wherein the nucleotide sequence of the targeting region is depicted in SEQ ID NO: 22.
51. The polynucleotide of any one of items 1 to 44, wherein the nucleotide sequence of the targeting region is complementary to nucleotides 36 to 75 of the sequence depicted in SEQ ID NO: 1.
52. The polynucleotide of item 51, wherein the nucleotide sequence of the targeting region is depicted in SEQ ID NO: 23.
53. The polynucleotide of any one of items 1 to 44, wherein the nucleotide sequence of the targeting region is complementary to nucleotides 58 to 75 of the sequence depicted in SEQ ID NO: 1.
54. The polynucleotide of item 53, wherein the nucleotide sequence of the targeting region is depicted in SEQ ID NO: 26.
55. The polynucleotide of item 16, wherein the nucleotide sequence of the targeting region is depicted in SEQ ID NO: 25.
56. The polynucleotide of any one of items 45, 47, 49, 51, 53 or 55, wherein the nucleotide sequence of the targeting region has at least 70%, at least 75%, at least 80%, at least 85%, at least 90%, at least 95%, at least 97%, or at least 99% identity to the sequence depicted in SEQ ID NO: 21 to 26, respectively.
57. The polynucleotide of any one of items 1 to 56, wherein the nucleotide sequence of the targeting region is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% complementary to the 5'-untranslated region or a continuous or discontinuous portion thereof.
58. The polynucleotide of any one of items 1 to 57, wherein the nucleotide sequence of the targeting region is 100% complementary to the 5'-untranslated region or a continuous or discontinuous portion thereof.
59. The polynucleotide of any one of items 1 to 58, wherein the nucleotide sequence of the targeting region is at least 15 nucleotides, at least 20 nucleotides, at least 25 nucleotides, at least 30 nucleotides, at least 35 nucleotides, at least 40 nucleotides, or at least 45 nucleotides in length.
60. The polynucleotide of any one of items 1 to 59, wherein the nucleotide sequence of the targeting region is at most 300 nucleotides, at most 265 nucleotides, at most 250 nucleotides, at most 200 nucleotides, at most 150 nucleotides, at most 125 nucleotides, at most 100 nucleotides, at most 90 nucleotides, at most 80 nucleotides, at most 70 nucleotides, or at most 60 nucleotides in length.
61. The polynucleotide of any one of items 1 to 60, wherein the nucleotide sequence of the polynucleotide is depicted in SEQ ID NO: 27 to 32, respectively.
62. A pharmaceutical composition comprising one or more of the polynucleotides of any one of items 1 to 61, and a pharmaceutically acceptable carrier.
63. A pharmaceutical composition of item 62 for use as a medicament.
64. A polynucleotide of any one of items 1 to 61 or a pharmaceutical composition of item 62 for use in a method of treating or preventing a Coronavirus infection, said method comprising a step of administering to a patient in need thereof one or more of the polynucleotides or the pharmaceutical composition.
65. A method of treating or preventing a Coronavirus infection comprising administering to a patient in need thereof one or more of the polynucleotides of any one of items 1 to 61 or the pharmaceutical composition of item 62.
66. The method of item 65 or the polynucleotides or the pharmaceutical composition for use of item 64, wherein the one or more polynucleotides or the pharmaceutical composition is/are administered locally or systemically.
67. The method or the polynucleotides or the pharmaceutical composition for use of item 66, wherein the one or more polynucleotides or the pharmaceutical composition is/are administered parenterally.
68. The method or the polynucleotides or the pharmaceutical composition for use of any one of items 65 to 67, wherein the one or more polynucleotides or the pharmaceutical composition is/are administered alone or in combination with any other therapeutic agent.
69. The method of or the polynucleotides or the pharmaceutical composition for use of item 68, wherein the other agent is selected from a vaccine, an antiviral drug, a protease inhibitor, lipids or liposomes, or any combination thereof.
70. The method of or the polynucleotides or the pharmaceutical composition for use of any one of items 65 to 69, wherein the Coronavirus is SARS-CoV-2.
71. A method for targeting a nucleic acid comprising contacting the nucleic acid to be targeted with a polynucleotide of any one of items 1 to 61.
72. A method for forming a complex comprising a polynucleotide of any one of items 1 to 61, and a single-stranded nucleic acid, the method comprising a step of contacting the polynucleotide with the single-stranded nucleic acid.
73. The method of item 72, wherein the single-stranded nucleic acid is a nucleic acid comprising or consisting of ribonucleotides.
74. The method of item 72 or 73, wherein the single-stranded nucleic acid is the genome or an mRNA of a Coronavirus.
75. A method for interfering with genome expression and/or proliferation of a Coronavirus comprising contacting a polynucleotide of any one of items 1 to 61 with the nucleic acid of the pathogen.
76. The method of any one of items 65 to 75, wherein the method is an *in vitro* or an *in vivo* method.
77. The method of item 74 or 75, wherein the Coronavirus is SARS-CoV-2.

## Claims

1. A polynucleotide comprising a nucleotide sequence, which sequence comprises a targeting region comprising or consisting of a nucleotide sequence complementary to a 5'-untranslated region (5'-UTR) of a Coronavirus or to a continuous or discontinuous portion of said 5' untranslated region.

2. The polynucleotide of claim 1, wherein the polynucleotide is a linear, preferably circular polynucleotide.

3. The polynucleotide of claim 1 or 2, wherein the polynucleotide is **characterized by** at least one feature selected from the group consisting of:
(i) comprising at least one ribonucleotide or consisting of ribonucleotides;
(ii) comprising at least one deoxyribonucleotide;
(iii) comprising a mixture of ribonucleotides and deoxyribonucleotides; and
(iv) comprising at least one modified nucleotide.

4. The polynucleotide of any one of claims 1 to 3, wherein the nucleotide sequence of the targeting region is complementary to at least one region of the 5'-UTR capable of forming a stem-loop, wherein preferably the region of the 5'-UTR capable of forming a stem-loop is selected from the group consisting of stem-loop 1, stem-loop 2 and stem-loop 3,
more preferably wherein the nucleotide sequence of the targeting region comprises a nucleotide sequence complementary to stem-loop 2 and/or stem-loop 3, and/or does not comprise or comprises a nucleotide sequence complementary to stem-loop 1.

5. The polynucleotide of any one of claims 1 to 4, wherein the nucleotide sequence of the targeting region is complementary to at least one continuous portion of the 5'-untranslated region, the continuous portion being selected from the group consisting of:
(i) nucleotides 1 to 75 of the sequence depicted in SEQ ID NO: 1;
(ii) nucleotides 1 to 65 of the sequence depicted in SEQ ID NO: 1;
(iii) nucleotides 1 to 40 of the sequence depicted in SEQ ID NO: 1;
(iv) nucleotides 36 to 75 of the sequence depicted in SEQ ID NO: 1;
(v) nucleotides 45 to 75 of the sequence depicted in SEQ ID NO: 1;
(vi) nucleotides 58 to 75 of the sequence depicted in SEQ ID NO: 1
(vii) nucleotides 61 to 75 of the sequence depicted in SEQ ID NO: 1;
(viii) nucleotides 70 to 75 of the sequence depicted in SEQ ID NO: 1;
(ix) nucleotides 45 to 59 of the sequence depicted in SEQ ID NO: 1;
(x) nucleotides 1 to 44 of the sequence depicted in SEQ ID NO: 1;
(xi) nucleotides 34 to 44 of the sequence depicted in SEQ ID NO: 1;
(xii) nucleotides 1 to 6 of the sequence depicted in SEQ ID NO: 1;
(xiii) nucleotides 7 to 33 of the sequence depicted in SEQ ID NO: 1; and
(xiv) nucleotides 247 to 265 of the sequence depicted in SEQ ID NO: 1;
or wherein the nucleotide sequence of the targeting region comprises at least two segments, each segment complementary to a nucleotide sequence of the 5'-UTR, wherein the complementary nucleotide sequences of the 5'-UTR are not continuous, preferably wherein
(i) the first segment of the nucleotide sequence of targeting region is complementary to nucleotides 1 to 6 and the second segment is complementary to nucleotides 34 to 44 of the sequence depicted in SEQ ID NO: 1;
(ii) the first segment of the nucleotide sequence of targeting region is complementary to nucleotides 45 to 59 and the second segment is complementary to nucleotides 61 to 75 of the sequence depicted in SEQ ID NO: 1;
(iii) the first segment of the nucleotide sequence of targeting region is complementary to nucleotides 36 to 59 and the second segment is complementary to nucleotides 61 to 75 of the sequence depicted in SEQ ID NO: 1;
(iv) the first segment of the nucleotide sequence of targeting region is complementary to nucleotides 1 to 6 and the second segment is complementary to nucleotides 34 to 59 of the sequence depicted in SEQ ID NO: 1; or
(v) the first segment of the nucleotide sequence of targeting region is complementary to nucleotides 1 to 6, the second segment is complementary to nucleotides 34 to 59 and the third segment is complementary to nucleotides 61 to 75 of the sequence depicted in SEQ ID NO: 1.

6. The polynucleotide of claim 5, wherein the nucleotide sequence of the targeting region comprises one or more nucleotides between the at least two segments, or between the first segment and the second segment and/or between the second segment and the third segment,
preferably wherein the one or more nucleotides between the two segments are two nucleotides, more preferably wherein two nucleotides are between the nucleotides of the first segment complementary to nucleotides 1 to 6 and the second segment complementary to nucleotides 34 to 44 of the sequence depicted in SEQ ID NO: 1, which two nucleotides are each not complementary to nucleotides 7 and 33, respectively, of the sequence depicted in SEQ ID NO: 1, or
preferably wherein the nucleotide between the two segments is a single nucleotide, more preferably wherein one nucleotide is between the nucleotides of the first segment complementary to nucleotides 45 to 59 and the second segment complementary to nucleotides 61 to 75 of the sequence depicted in SEQ ID NO: 1, which nucleotide is complementary or non-complementary to nucleotide 60 of the sequence depicted in SEQ ID NO: 1.

7. The polynucleotide of any one of claims 1 to 6, wherein the nucleotide sequence of the targeting region is complementary to at least nucleotides 45 to 59 of the sequence depicted in SEQ ID NO: 1,
preferably wherein the targeting region is flanked on one or both ends by a flanking region, wherein each flanking region comprises one or more additional nucleotides, and each additional nucleotide is complementary or non-complementary to the corresponding nucleotide of the nucleotides flanking the portion of the 5'-UTR to which the nucleotide sequence of the targeting region is complementary,
more preferably, wherein the nucleotide sequence of the targeting region complementary to at least nucleotides 45 to 59 of the sequence depicted in SEQ ID NO: 1 is
(i) flanked 3' by one or more additional nucleotides for targeting the nucleotide at position 44 up to at most to the nucleotide at position 1 of the sequence depicted in SEQ ID NO: 1, or for targeting the nucleotides at positions 44 to 34 and one or more nucleotides at position 6 up to at most the nucleotide at position 1 of the sequence depicted in SEQ ID NO: 1, more preferably wherein the nucleotide sequence of the targeting region is depicted in any one of SEQ ID NO: 2 to SEQ ID: 9; and/or is
(ii) flanked 5' by one or more additional nucleotides for targeting the nucleotide at position 60 up to at most to the nucleotide at position 85 of the sequence depicted in SEQ ID NO: 1, preferably for targeting the nucleotide at position 60 up to at most to the nucleotide at position 75 of the sequence depicted in SEQ ID NO: 1, more preferably wherein the nucleotide sequence of the targeting region is depicted in any one of SEQ ID NO: 10 to SEQ ID: 20.

8. The polynucleotide of any one of claims 1 to 7, wherein
(i) the nucleotide sequence of the targeting region is complementary to nucleotides 1 to 6 and 34 to 65 of the sequence depicted in SEQ ID NO: 1, preferably wherein the nucleotide sequence of the targeting region is depicted in SEQ ID NO: 21, or
(ii) the nucleotide sequence of the targeting region is complementary to nucleotides 1 to 6 and 34 to 75 of the sequence depicted in SEQ ID NO: 1, preferably wherein the nucleotide sequence of the targeting region is depicted in SEQ ID NO: 22, or
(iii) the nucleotide sequence of the targeting region is complementary to nucleotides 36 to 75 of the sequence depicted in SEQ ID NO: 1, preferably wherein the nucleotide sequence of the targeting region is depicted in SEQ ID NO: 23, or
(iv) the nucleotide sequence of the targeting region is complementary to nucleotides 58 to 75 of the sequence depicted in SEQ ID NO: 1, preferably wherein the nucleotide sequence of the targeting region is depicted in SEQ ID NO: 26, or
(v) the nucleotide sequence of the targeting region is complementary to nucleotides 247 to 265 of the sequence depicted in SEQ ID NO: 1, preferably is depicted in SEQ ID NO: 25, or
(vi) the nucleotide sequence of the targeting region has at least 70%, at least 75%, at least 80%, at least 85%, at least 90%, at least 95%, at least 97%, or at least 99% identity to the sequence depicted in SEQ ID NO: 21 to 23 or 25 to 26, respectively.

9. The polynucleotide of any one of claims 1 to 8, wherein the nucleotide sequence of the targeting region is at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% complementary to the 5'-untranslated region or a continuous or discontinuous portion thereof, and/or wherein the nucleotide sequence of the targeting region is at least 15 nucleotides, at least 20 nucleotides, at least 25 nucleotides, at least 30 nucleotides, at least 35 nucleotides, at least 40 nucleotides, or at least 45 nucleotides in length, and/or wherein the nucleotide sequence of the targeting region is at most 300 nucleotides, at most 265 nucleotides, at most 250 nucleotides, at most 200 nucleotides, at most 150 nucleotides, at most 125 nucleotides, at most 100 nucleotides, at most 90 nucleotides, at most 80 nucleotides, at most 70 nucleotides, or at most 60 nucleotides in length, and/or
wherein the nucleotide sequence of the polynucleotide is depicted in SEQ ID NO: 27 to 32, respectively.

10. A pharmaceutical composition comprising one or more of the polynucleotides of any one of claims 1 to 9, and a pharmaceutically acceptable carrier.

11. A pharmaceutical composition of claim 10 for use as a medicament.

12. A polynucleotide of any one of claims 1 to 9 or a pharmaceutical composition of claim 10 for use in a method of treating or preventing a Coronavirus infection, preferably a SARS-CoV-2 infection, said method comprising a step of administering to a patient in need thereof one or more of the polynucleotides or the pharmaceutical composition.

13. A method for targeting a nucleic acid comprising contacting the nucleic acid to be targeted with a polynucleotide of any one of claims 1 to 9.

14. A method for forming a complex comprising a polynucleotide of any one of claims 1 to 9, and a single-stranded nucleic acid, the method comprising a step of contacting the polynucleotide with the single-stranded nucleic acid, preferably wherein the single-stranded nucleic acid is a nucleic acid comprising or consisting of ribonucleotides and/or preferably wherein the single-stranded nucleic acid is the genome or an mRNA of a Coronavirus, more preferably the genome or an mRNA of SARS-CoV-2.

15. A method for interfering with genome expression and/or proliferation of a Coronavirus, preferably of SARS-CoV-2, comprising contacting a polynucleotide of any one of claims 1 to 9 with the nucleic acid of the pathogen.


SEQUENCE LISTING

<110>  Justus-Liebig-Universität Gießen

<120>  CIRCULAR NUCLEIC ACIDS AND USES THEREOF FOR INTERFERING WITH
       GENOME EXPRESSION AND PROLIFERATION OF CORONAVIRUSES

<130>  TM 1110

<160>  35

<170>  PatentIn version 3.5

<210>  1
<211>  265
<212>  DNA
<213>  Severe acute respiratory syndrome coronavirus 2 isolate Wuhan-Hu-1;
5′-UTR

<400>  1
attaaaggtt tataccttcc caggtaacaa accaaccaac tttcgatctc ttgtagatct      60

gttctctaaa cgaactttaa aatctgtgtg gctgtcactc ggctgcatgc ttagtgcact     120

cacgcagtat aattaataac taattactgt cgttgacagg acacgagtaa ctcgtctatc     180

ttctgcaggc tgcttacggt ttcgtccgtg ttgcagccga tcatcagcac atctaggttt     240

cgtccgggtg tgaccgaaag gtaag                                           265


<210>  2
<211>  15
<212>  DNA
<213>  Artificial Sequence

<220>
<223>  Targeting region for SL2

<400>  2
gatctacaag agatc                                                       15


<210>  3
<211>  26
<212>  DNA
<213>  Artificial Sequence

<220>
<223>  Targeting region for SL2 3′ elongated

<400>  3
gatctacaag agatcgaaag ttggtt                                           26


<210>  4
<211>  29
<212>  DNA
<213>  Artificial Sequence

<220>
<223>  Targeting region for SL2 3′ elongated

<400>  4
gatctacaag agatcgaaag ttggtthdt                                          29


<210>  5
<211>  30
<212>  DNA
<213>  Artificial Sequence

<220>
<223>  Targeting region for SL2 3′ elongated

<400>  5
gatctacaag agatcgaaag ttggtthdtt                                         30


<210>  6
<211>  31
<212>  DNA
<213>  Artificial Sequence

<220>
<223>  Targeting region for SL2 3′ elongated

<400>  6
gatctacaag agatcgaaag ttggtthdtt t                                       31


<210>  7
<211>  32
<212>  DNA
<213>  Artificial Sequence

<220>
<223>  Targeting region for SL2 3′ elongated

<400>  7
gatctacaag agatcgaaag ttggtthdtt ta                                      32


<210>  8
<211>  33
<212>  DNA
<213>  Artificial sequence

<220>
<223>  Targeting region for SL2 3′ elongated

<400>  8
gatctacaag agatcgaaag ttggtthdtt taa                                     33


<210>  9
<211>  34
<212>  DNA
<213>  Artificial Sequence

<220>
<223>  Targeting region for SL2 3′ elongated

<400>  9
gatctacaag agatcgaaag ttggtthdtt taat                                    34

<210>  10
<211>  21
<212>  DNA
<213>  Artificial Sequence

<220>
<223>  Targeting region for SL2 5′ elongated

<400>  10
agaacagatc tacaagagat c                                                21


<210>  11
<211>  22
<212>  DNA
<213>  Artificial Sequence

<220>
<223>  Targeting region for SL2 5′ elongated

<400>  11
gagaacagat ctacaagaga tc                                               22


<210>  12
<211>  23
<212>  DNA
<213>  Artificial Sequence

<220>
<223>  Targeting region for SL2 5′ elongated

<400>  12
agagaacaga tctacaagag atc                                              23


<210>  13
<211>  24
<212>  DNA
<213>  Artificial Sequence

<220>
<223>  Targeting region for SL2 5′ elongated

<400>  13
tagagaacag atctacaaga gatc                                             24


<210>  14
<211>  25
<212>  DNA
<213>  Artificial Sequence

<220>
<223>  Targeting region for SL2 5′ elongated

<400>  14
ttagagaaca gatctacaag agatc                                            25


<210>  15
<211>  26

<212>  DNA
<213>  Artificial Sequence

<220>
<223>  Targeting region for SL2 5′ elongated

<400>  15
tttagagaac agatctacaa gagatc                                          26


<210>  16
<211>  27
<212>  DNA
<213>  Artificial Sequence

<220>
<223>  Targeting region for SL2 5′ elongated

<400>  16
gtttagagaa cagatctaca agagatc                                         27


<210>  17
<211>  28
<212>  DNA
<213>  Artificial Sequence

<220>
<223>  Targeting region for SL2 5′ elongated

<400>  17
cgtttagaga acagatctac aagagatc                                        28


<210>  18
<211>  29
<212>  DNA
<213>  Artificial Sequence

<220>
<223>  Targeting region for SL2 5′ elongated

<400>  18
tcgtttagag aacagatcta caagagatc                                       29


<210>  19
<211>  30
<212>  DNA
<213>  Artificial Sequence

<220>
<223>  Targeting region for SL2 5′ elongated

<400>  19
ttcgtttaga gaacagatct acaagagatc                                      30


<210>  20
<211>  31
<212>  DNA
<213>  Artificial Sequence

<220>
<223>  Targeting region for SL2 5′ elongated

<400>  20
gttcgtttag agaacagatc tacaagagat c                                    31


<210>  21
<211>  40
<212>  DNA
<213>  Artificial Sequence

<220>
<223>  Targeting region

<400>  21
agaacagatc tacaagagat cgaaagttgg ttaatttaat                           40


<210>  22
<211>  50
<212>  DNA
<213>  Artificial Sequence

<220>
<223>  Targeting region

<400>  22
gttcgtttag agaacagatc tacaagagat cgaaagttgg ttaatttaat                50


<210>  23
<211>  40
<212>  DNA
<213>  Artificial Sequence

<220>
<223>  Targeting region

<400>  23
gttcgtttag agaacagatc tacaagagat cgaaagttgg                           40


<210>  24
<211>  40
<212>  DNA
<213>  Artificial Sequence

<220>
<223>  Targeting region

<400>  24
gttggttggt ttgttacctg ggaaggtata aacctttaat                           40


<210>  25
<211>  19
<212>  DNA
<213>  Artificial Sequence

<220>
<223>  Targeting region

<400>  25
cttacctttc ggtcacacc                                                    19


<210>  26
<211>  18
<212>  DNA
<213>  Artificial Sequence

<220>
<223>  Targeting region

<400>  26
gttcgtttag agaacaga                                                     18


<210>  27
<211>  66
<212>  DNA
<213>  Artificial Sequence

<220>
<223>  Circularizable polynucleotide

<400>  27
gggagtaagc aaagttggtt ggtttgttac ctgggaaggt ataaaccttt aataaagctt      60

acagta                                                                  66


<210>  28
<211>  66
<212>  DNA
<213>  Artificial Sequence

<220>
<223>  Circularizable polynucleotide

<400>  28
gggagtaagc aaaagaacag atctacaaga gatcgaaagt tggttaattt aataaagctt      60

acagta                                                                  66


<210>  29
<211>  76
<212>  DNA
<213>  Artificial Sequence

<220>
<223>  Circularizable polynucleotide

<400>  29
gggagtaagc aaagttcgtt tagagaacag atctacaaga gatcgaaagt tggttaattt      60

aataaagctt acagta                                                       76


<210>  30
<211>  66
<212>  DNA
<213>  Artificial Sequence

<220>
<223>  Circularizable polynucleotide

<400>  30
gggagtaagc aaagttcgtt tagagaacag atctacaaga gatcgaaagt tggaaagctt      60

acagta                                                                  66


<210>  31
<211>  66
<212>  DNA
<213>  Artificial Sequence

<220>
<223>  Circularizable polynucleotide

<400>  31
gggagtaagc aaaaccaggg acaaggctct ccatcttacc tttcggtcac accaaagctt      60

acagta                                                                  66


<210>  32
<211>  66
<212>  DNA
<213>  Artificial Sequence

<220>
<223>  Circularizable polynucleotide

<400>  32
gggagtaagc aaataaaaca agaaaaacaa acattgttcg tttagagaac agaaaagctt      60

acagta                                                                  66


<210>  33
<211>  66
<212>  DNA
<213>  Artificial Sequence

<220>
<223>  Circularizable polynucleotide, CTR1

<400>  33
gggagtaagc aaaagatgcg caccgcacag atgcgcacca cgccacgcgt agaaaagctt      60

acagta                                                                  66


<210>  34
<211>  66
<212>  DNA
<213>  Artificial Sequence

<220>
<223>  Circularizable polynucleotide, CTR2

<400>  34
gggagtaagc aaaagtcagt gagtcagtgt aatacgggag gatacccgct gtcaaagctt      60

acagta                                                                  66

<210>  35
<211>  29903
<212>  DNA
<213>  Severe acute respiratory syndrome coronavirus 2 isolate Wuhan-Hu-1

<400>  35
   attaaaggtt tataccttcc caggtaacaa accaaccaac tttcgatctc ttgtagatct      60
   gttctctaaa cgaactttaa aatctgtgtg gctgtcactc ggctgcatgc ttagtgcact     120
   cacgcagtat aattaataac taattactgt cgttgacagg acacgagtaa ctcgtctatc     180
   ttctgcaggc tgcttacggt ttcgtccgtg ttgcagccga tcatcagcac atctaggttt     240
   cgtccgggtg tgaccgaaag gtaagatgga gagccttgtc cctggtttca acgagaaaac     300
   acacgtccaa ctcagtttgc ctgttttaca ggttcgcgac gtgctcgtac gtggctttgg     360
   agactccgtg gaggaggtct tatcagaggc acgtcaacat cttaaagatg gcacttgtgg     420
   cttagtagaa gttgaaaaag gcgttttgcc tcaacttgaa cagccctatg tgttcatcaa     480
   acgttcggat gctcgaactg cacctcatgg tcatgttatg gttgagctgg tagcagaact     540
   cgaaggcatt cagtacggtc gtagtggtga gacacttggt gtccttgtcc ctcatgtggg     600
   cgaaatacca gtggcttacc gcaaggttct tcttcgtaag aacggtaata aaggagctgg     660
   tggccatagt tacggcgccg atctaaagtc atttgactta ggcgacgagc ttggcactga     720
   tccttatgaa gattttcaag aaaactggaa cactaaacat agcagtggtg ttacccgtga     780
   actcatgcgt gagcttaacg gaggggcata cactcgctat gtcgataaca acttctgtgg     840
   ccctgatggc taccctcttg agtgcattaa agaccttcta gcacgtgctg gtaaagcttc     900
   atgcactttg tccgaacaac tggactttat tgacactaag aggggtgtat actgctgccg     960
   tgaacatgag catgaaattg cttggtacac ggaacgttct gaaaagagct atgaattgca    1020
   gacacctttt gaaattaaat tggcaaagaa atttgacacc ttcaatgggg aatgtccaaa    1080
   ttttgtattt cccttaaatt ccataatcaa gactattcaa ccaagggttg aaaagaaaaa    1140
   gcttgatggc tttatgggta gaattcgatc tgtctatcca gttgcgtcac caaatgaatg    1200
   caaccaaatg tgcctttcaa ctctcatgaa gtgtgatcat tgtggtgaaa cttcatggca    1260
   gacgggcgat tttgttaaag ccacttgcga attttgtggc actgagaatt tgactaaaga    1320
   aggtgccact acttgtggtt acttacccca aaatgctgtt gttaaaattt attgtccagc    1380
   atgtcacaat tcagaagtag gacctgagca tagtcttgcc gaataccata atgaatctgg    1440
   cttgaaaacc attcttcgta agggtggtcg cactattgcc tttggaggct gtgtgttctc    1500
   ttatgttggt tgccataaca agtgtgccta ttgggttcca cgtgctagcg ctaacatagg    1560
   ttgtaaccat acaggtgttg ttggagaagg ttccgaaggt cttaatgaca accttcttga    1620
   aatactccaa aaagagaaag tcaacatcaa tattgttggt gactttaaac ttaatgaaga    1680
   gatcgccatt attttggcat ctttttctgc ttccacaagt gcttttgtgg aaactgtgaa 1740
   aggtttggat tataaagcat tcaaacaaat tgttgaatcc tgtggtaatt ttaaagttac 1800
   aaaaggaaaa gctaaaaaag gtgcctggaa tattggtgaa cagaaatcaa tactgagtcc 1860
   tctttatgca tttgcatcag aggctgctcg tgttgtacga tcaattttct cccgcactct 1920
   tgaaactgct caaaattctg tgcgtgtttt acagaaggcc gctataacaa tactagatgg 1980
   aatttcacag tattcactga gactcattga tgctatgatg ttcacatctg atttggctac 2040
   taacaatcta gttgtaatgg cctacattac aggtggtgtt gttcagttga cttcgcagtg 2100
   gctaactaac atctttggca ctgtttatga aaaactcaaa cccgtccttg attggcttga 2160
   agagaagttt aaggaaggtg tagagtttct tagagacggt tgggaaattg ttaaatttat 2220
   ctcaacctgt gcttgtgaaa ttgtcggtgg acaaattgtc acctgtgcaa aggaaattaa 2280
   ggagagtgtt cagacattct ttaagcttgt aaataaattt ttggctttgt gtgctgactc 2340
   tatcattatt ggtggagcta aacttaaagc cttgaattta ggtgaaacat ttgtcacgca 2400
   ctcaaaggga ttgtacagaa agtgtgttaa atccagagaa gaaactggcc tactcatgcc 2460
   tctaaaagcc ccaaaagaaa ttatcttctt agagggagaa acacttccca cagaagtgtt 2520
   aacagaggaa gttgtcttga aaactggtga tttacaacca ttagaacaac ctactagtga 2580
   agctgttgaa gctccattgg ttggtacacc agtttgtatt aacgggctta tgttgctcga 2640
   aatcaaagac acagaaaagt actgtgccct tgcacctaat atgatggtaa caaacaatac 2700
   cttcacactc aaaggcggtg caccaacaaa ggttactttt ggtgatgaca ctgtgataga 2760
   agtgcaaggt tacaagagtg tgaatatcac ttttgaactt gatgaaagga ttgataaagt 2820
   acttaatgag aagtgctctg cctatacagt tgaactcggt acagaagtaa atgagttcgc 2880
   ctgtgttgtg gcagatgctg tcataaaaac tttgcaacca gtatctgaat tacttacacc 2940
   actgggcatt gatttagatg agtggagtat ggctacatac tacttatttg atgagtctgg 3000
   tgagtttaaa ttggcttcac atatgtattg ttctttctac cctccagatg aggatgaaga 3060
   agaaggtgat tgtgaagaag aagagtttga gccatcaact caatatgagt atggtactga 3120
   agatgattac caaggtaaac ctttggaatt tggtgccact tctgctgctc ttcaacctga 3180
   agaagagcaa gaagaagatt ggttagatga tgatagtcaa caaactgttg gtcaacaaga 3240
   cggcagtgag gacaatcaga caactactat tcaaacaatt gttgaggttc aacctcaatt 3300
   agagatggaa cttacaccag ttgttcagac tattgaagtg aatagtttta gtggttattt 3360
   aaaacttact gacaatgtat acattaaaaa tgcagacatt gtggaagaag ctaaaaaggt 3420
   aaaaccaaca gtggttgtta atgcagccaa tgtttacctt aaacatggag gaggtgttgc 3480
   aggagcctta aataaggcta ctaacaatgc catgcaagtt gaatctgatg attacatagc 3540
   tactaatgga ccacttaaag tgggtggtag ttgtgtttta agcggacaca atcttgctaa 3600
   acactgtctt catgttgtcg gcccaaatgt taacaaaggt gaagacattc aacttcttaa 3660
   gagtgcttat gaaaatttta atcagcacga agttctactt gcaccattat tatcagctgg 3720
   tatttttggt gctgacccta tacattcttt aagagtttgt gtagatactg ttcgcacaaa 3780
   tgtctactta gctgtctttg ataaaaatct ctatgacaaa cttgtttcaa gctttttgga 3840
   aatgaagagt gaaaagcaag ttgaacaaaa gatcgctgag attcctaaag aggaagttaa 3900
   gccatttata actgaaagta aaccttcagt tgaacagaga aaacaagatg ataagaaaat 3960
   caaagcttgt gttgaagaag ttacaacaac tctggaagaa actaagttcc tcacagaaaa 4020
   cttgttactt tatattgaca ttaatggcaa tcttcatcca gattctgcca ctcttgttag 4080
   tgacattgac atcactttct taaagaaaga tgctccatat atagtgggtg atgttgttca 4140
   agagggtgtt ttaactgctg tggttatacc tactaaaaag gctggtggca ctactgaaat 4200
   gctagcgaaa gctttgagaa aagtgccaac agacaattat ataaccactt acccgggtca 4260
   gggtttaaat ggttacactg tagaggaggc aaagacagtg cttaaaaagt gtaaaagtgc 4320
   cttttacatt ctaccatcta ttatctctaa tgagaagcaa gaaattcttg gaactgtttc 4380
   ttggaatttg cgagaaatgc ttgcacatgc agaagaaaca cgcaaattaa tgcctgtctg 4440
   tgtggaaact aaagccatag tttcaactat acagcgtaaa tataagggta ttaaaataca 4500
   agagggtgtg gttgattatg gtgctagatt ttacttttac accagtaaaa caactgtagc 4560
   gtcacttatc aacacactta acgatctaaa tgaaactctt gttacaatgc cacttggcta 4620
   tgtaacacat ggcttaaatt tggaagaagc tgctcggtat atgagatctc tcaaagtgcc 4680
   agctacagtt tctgtttctt cacctgatgc tgttacagcg tataatggtt atcttacttc 4740
   ttcttctaaa acacctgaag aacattttat tgaaaccatc tcacttgctg gttcctataa 4800
   agattggtcc tattctggac aatctacaca actaggtata gaatttctta agagaggtga 4860
   taaaagtgta tattacacta gtaatcctac cacattccac ctagatggtg aagttatcac 4920
   ctttgacaat cttaagacac ttctttcttt gagagaagtg aggactatta aggtgtttac 4980
   aacagtagac aacattaacc tccacacgca agttgtggac atgtcaatga catatggaca 5040
   acagtttggt ccaacttatt tggatggagc tgatgttact aaaataaaac ctcataattc 5100
   acatgaaggt aaaacatttt atgttttacc taatgatgac actctacgtg ttgaggcttt 5160
   tgagtactac cacacaactg atcctagttt tctgggtagg tacatgtcag cattaaatca 5220
   cactaaaaag tggaaatacc cacaagttaa tggtttaact tctattaaat gggcagataa 5280
   caactgttat cttgccactg cattgttaac actccaacaa atagagttga agtttaatcc 5340
   acctgctcta caagatgctt attacagagc aagggctggt gaagctgcta acttttgtgc 5400
   acttatctta gcctactgta ataagacagt aggtgagtta ggtgatgtta gagaaacaat 5460
   gagttacttg tttcaacatg ccaatttaga ttcttgcaaa agagtcttga acgtggtgtg 5520
   taaaacttgt ggacaacagc agacaaccct taagggtgta gaagctgtta tgtacatggg 5580
   cacactttct tatgaacaat ttaagaaagg tgttcagata ccttgtacgt gtggtaaaca 5640
   agctacaaaa tatctagtac aacaggagtc accttttgtt atgatgtcag caccacctgc 5700
   tcagtatgaa cttaagcatg gtacatttac ttgtgctagt gagtacactg gtaattacca 5760
   gtgtggtcac tataaacata taacttctaa agaaactttg tattgcatag acggtgcttt 5820
   acttacaaag tcctcagaat acaaaggtcc tattacggat gttttctaca aagaaaacag 5880
   ttacacaaca accataaaac cagttactta taaattggat ggtgttgttt gtacagaaat 5940
   tgaccctaag ttggacaatt attataagaa agacaattct tatttcacag agcaaccaat 6000
   tgatcttgta ccaaaccaac catatccaaa cgcaagcttc gataatttta agtttgtatg 6060
   tgataatatc aaatttgctg atgatttaaa ccagttaact ggttataaga aacctgcttc 6120
   aagagagctt aaagttacat ttttccctga cttaaatggt gatgtggtgg ctattgatta 6180
   taaacactac acaccctctt ttaagaaagg agctaaattg ttacataaac ctattgtttg 6240
   gcatgttaac aatgcaacta ataaagccac gtataaacca aatacctggt gtatacgttg 6300
   tctttggagc acaaaaccag ttgaaacatc aaattcgttt gatgtactga agtcagagga 6360
   cgcgcaggga atggataatc ttgcctgcga agatctaaaa ccagtctctg aagaagtagt 6420
   ggaaaatcct accatacaga aagacgttct tgagtgtaat gtgaaaacta ccgaagttgt 6480
   aggagacatt atacttaaac cagcaaataa tagtttaaaa attacagaag aggttggcca 6540
   cacagatcta atggctgctt atgtagacaa ttctagtctt actattaaga aacctaatga 6600
   attatctaga gtattaggtt tgaaaaccct tgctactcat ggtttagctg ctgttaatag 6660
   tgtcccttgg gatactatag ctaattatgc taagcctttt cttaacaaag ttgttagtac 6720
   aactactaac atagttacac ggtgtttaaa ccgtgtttgt actaattata tgccttattt 6780
   ctttacttta ttgctacaat tgtgtacttt tactagaagt acaaattcta gaattaaagc 6840
   atctatgccg actactatag caaagaatac tgttaagagt gtcggtaaat tttgtctaga 6900
   ggcttcattt aattatttga agtcacctaa tttttctaaa ctgataaata ttataatttg 6960
   gtttttacta ttaagtgttt gcctaggttc tttaatctac tcaaccgctg ctttaggtgt 7020
   tttaatgtct aatttaggca tgccttctta ctgtactggt tacagagaag gctatttgaa 7080
   ctctactaat gtcactattg caacctactg tactggttct ataccttgta gtgtttgtct 7140
   tagtggttta gattctttag acacctatcc ttctttagaa actatacaaa ttaccatttc 7200
   atcttttaaa tgggatttaa ctgcttttgg cttagttgca gagtggtttt tggcatatat 7260
   tcttttcact aggtttttct atgtacttgg attggctgca atcatgcaat tgtttttcag 7320
   ctattttgca gtacatttta ttagtaattc ttggcttatg tggttaataa ttaatcttgt 7380
   acaaatggcc ccgatttcag ctatggttag aatgtacatc ttctttgcat cattttatta 7440
   tgtatggaaa agttatgtgc atgttgtaga cggttgtaat tcatcaactt gtatgatgtg 7500
   ttacaaacgt aatagagcaa caagagtcga atgtacaact attgttaatg gtgttagaag 7560
   gtccttttat gtctatgcta atggaggtaa aggcttttgc aaactacaca attggaattg 7620
   tgttaattgt gatacattct gtgctggtag tacatttatt agtgatgaag ttgcgagaga 7680
   cttgtcacta cagtttaaaa gaccaataaa tcctactgac cagtcttctt acatcgttga 7740
   tagtgttaca gtgaagaatg gttccatcca tctttacttt gataaagctg gtcaaaagac 7800
   ttatgaaaga cattctctct ctcattttgt taacttagac aacctgagag ctaataacac 7860
   taaaggttca ttgcctatta atgttatagt ttttgatggt aaatcaaaat gtgaagaatc 7920
   atctgcaaaa tcagcgtctg tttactacag tcagcttatg tgtcaaccta tactgttact 7980
   agatcaggca ttagtgtctg atgttggtga tagtgcggaa gttgcagtta aaatgtttga 8040
   tgcttacgtt aatacgtttt catcaacttt taacgtacca atggaaaaac tcaaaacact 8100
   agttgcaact gcagaagctg aacttgcaaa gaatgtgtcc ttagacaatg tcttatctac 8160
   ttttatttca gcagctcggc aagggtttgt tgattcagat gtagaaacta aagatgttgt 8220
   tgaatgtctt aaattgtcac atcaatctga catagaagtt actggcgata gttgtaataa 8280
   ctatatgctc acctataaca aagttgaaaa catgacaccc cgtgaccttg gtgcttgtat 8340
   tgactgtagt gcgcgtcata ttaatgcgca ggtagcaaaa agtcacaaca ttgctttgat 8400
   atggaacgtt aaagatttca tgtcattgtc tgaacaacta cgaaaacaaa tacgtagtgc 8460
   tgctaaaaag aataacttac cttttaagtt gacatgtgca actactagac aagttgttaa 8520
   tgttgtaaca acaaagatag cacttaaggg tggtaaaatt gttaataatt ggttgaagca 8580
   gttaattaaa gttacacttg tgttcctttt tgttgctgct attttctatt taataacacc 8640
   tgttcatgtc atgtctaaac atactgactt ttcaagtgaa atcataggat acaaggctat 8700
   tgatggtggt gtcactcgtg acatagcatc tacagatact tgttttgcta acaaacatgc 8760
   tgattttgac acatggttta gccagcgtgg tggtagttat actaatgaca aagcttgccc 8820
   attgattgct gcagtcataa caagagaagt gggttttgtc gtgcctggtt tgcctggcac 8880
   gatattacgc acaactaatg gtgacttttt gcatttctta cctagagttt ttagtgcagt 8940
   tggtaacatc tgttacacac catcaaaact tatagagtac actgactttg caacatcagc 9000
   ttgtgttttg gctgctgaat gtacaatttt taaagatgct tctggtaagc cagtaccata 9060
   ttgttatgat accaatgtac tagaaggttc tgttgcttat gaaagtttac gccctgacac 9120
   acgttatgtg ctcatggatg gctctattat tcaatttcct aacacctacc ttgaaggttc 9180
   tgttagagtg gtaacaactt ttgattctga gtactgtagg cacggcactt gtgaaagatc 9240
   agaagctggt gtttgtgtat ctactagtgg tagatgggta cttaacaatg attattacag 9300
   atctttacca ggagttttct gtggtgtaga tgctgtaaat ttacttacta atatgtttac 9360
   accactaatt caacctattg gtgctttgga catatcagca tctatagtag ctggtggtat 9420
   tgtagctatc gtagtaacat gccttgccta ctattttatg aggtttagaa gagcttttgg 9480
   tgaatacagt catgtagttg cctttaatac tttactattc cttatgtcat tcactgtact 9540
   ctgtttaaca ccagtttact cattcttacc tggtgtttat tctgttattt acttgtactt 9600
   gacattttat cttactaatg atgtttcttt tttagcacat attcagtgga tggttatgtt 9660
   cacaccttta gtacctttct ggataacaat tgcttatatc atttgtattt ccacaaagca 9720
   tttctattgg ttctttagta attacctaaa gagacgtgta gtctttaatg gtgtttcctt 9780
   tagtactttt gaagaagctg cgctgtgcac ctttttgtta aataaagaaa tgtatctaaa 9840
   gttgcgtagt gatgtgctat tacctcttac gcaatataat agatacttag ctctttataa 9900
   taagtacaag tattttagtg gagcaatgga tacaactagc tacagagaag ctgcttgttg 9960
   tcatctcgca aaggctctca atgacttcag taactcaggt tctgatgttc tttaccaacc 10020
   accacaaacc tctatcacct cagctgtttt gcagagtggt tttagaaaaa tggcattccc 10080
   atctggtaaa gttgagggtt gtatggtaca agtaacttgt ggtacaacta cacttaacgg 10140
   tctttggctt gatgacgtag tttactgtcc aagacatgtg atctgcacct ctgaagacat 10200
   gcttaaccct aattatgaag atttactcat tcgtaagtct aatcataatt tcttggtaca 10260
   ggctggtaat gttcaactca gggttattgg acattctatg caaaattgtg tacttaagct 10320
   taaggttgat acagccaatc ctaagacacc taagtataag tttgttcgca ttcaaccagg 10380
   acagactttt tcagtgttag cttgttacaa tggttcacca tctggtgttt accaatgtgc 10440
   tatgaggccc aatttcacta ttaagggttc attccttaat ggttcatgtg gtagtgttgg 10500
   ttttaacata gattatgact gtgtctcttt ttgttacatg caccatatgg aattaccaac 10560
   tggagttcat gctggcacag acttagaagg taacttttat ggaccttttg ttgacaggca 10620
   aacagcacaa gcagctggta cggacacaac tattacagtt aatgttttag cttggttgta 10680
   cgctgctgtt ataaatggag acaggtggtt tctcaatcga tttaccacaa ctcttaatga 10740
   ctttaacctt gtggctatga agtacaatta tgaacctcta acacaagacc atgttgacat 10800
   actaggacct ctttctgctc aaactggaat tgccgtttta gatatgtgtg cttcattaaa 10860
   agaattactg caaaatggta tgaatggacg taccatattg ggtagtgctt tattagaaga 10920
   tgaatttaca ccttttgatg ttgttagaca atgctcaggt gttactttcc aaagtgcagt 10980
   gaaaagaaca atcaagggta cacaccactg gttgttactc acaattttga cttcactttt 11040
   agttttagtc cagagtactc aatggtcttt gttctttttt ttgtatgaaa atgccttttt 11100
   accttttgct atgggtatta ttgctatgtc tgcttttgca atgatgtttg tcaaacataa 11160
   gcatgcattt ctctgtttgt ttttgttacc ttctcttgcc actgtagctt attttaatat 11220
   ggtctatatg cctgctagtt gggtgatgcg tattatgaca tggttggata tggttgatac 11280
   tagtttgtct ggttttaagc taaaagactg tgttatgtat gcatcagctg tagtgttact 11340
   aatccttatg acagcaagaa ctgtgtatga tgatggtgct aggagagtgt ggacacttat 11400
   gaatgtcttg acactcgttt ataaagttta ttatggtaat gctttagatc aagccatttc 11460
   catgtgggct cttataatct ctgttacttc taactactca ggtgtagtta caactgtcat 11520
   gtttttggcc agaggtattg tttttatgtg tgttgagtat tgccctattt tcttcataac 11580
   tggtaataca cttcagtgta taatgctagt ttattgtttc ttaggctatt tttgtacttg 11640
   ttactttggc ctcttttgtt tactcaaccg ctactttaga ctgactcttg gtgtttatga 11700
   ttacttagtt tctacacagg agtttagata tatgaattca cagggactac tcccacccaa 11760
   gaatagcata gatgccttca aactcaacat taaattgttg ggtgttggtg gcaaaccttg 11820
   tatcaaagta gccactgtac agtctaaaat gtcagatgta aagtgcacat cagtagtctt 11880
   actctcagtt ttgcaacaac tcagagtaga atcatcatct aaattgtggg ctcaatgtgt 11940
   ccagttacac aatgacattc tcttagctaa agatactact gaagcctttg aaaaaatggt 12000
   ttcactactt tctgttttgc tttccatgca gggtgctgta gacataaaca agctttgtga 12060
   agaaatgctg gacaacaggg caaccttaca agctatagcc tcagagttta gttcccttcc 12120
   atcatatgca gcttttgcta ctgctcaaga agcttatgag caggctgttg ctaatggtga 12180
   ttctgaagtt gttcttaaaa agttgaagaa gtctttgaat gtggctaaat ctgaatttga 12240
   ccgtgatgca gccatgcaac gtaagttgga aaagatggct gatcaagcta tgacccaaat 12300
   gtataaacag gctagatctg aggacaagag ggcaaaagtt actagtgcta tgcagacaat 12360
   gcttttcact atgcttagaa agttggataa tgatgcactc aacaacatta tcaacaatgc 12420
   aagagatggt tgtgttccct tgaacataat acctcttaca acagcagcca aactaatggt 12480
   tgtcatacca gactataaca catataaaaa tacgtgtgat ggtacaacat ttacttatgc 12540
   atcagcattg tgggaaatcc aacaggttgt agatgcagat agtaaaattg ttcaacttag 12600
   tgaaattagt atggacaatt cacctaattt agcatggcct cttattgtaa cagctttaag 12660
   ggccaattct gctgtcaaat tacagaataa tgagcttagt cctgttgcac tacgacagat 12720
   gtcttgtgct gccggtacta cacaaactgc ttgcactgat gacaatgcgt tagcttacta 12780
   caacacaaca aagggaggta ggtttgtact tgcactgtta tccgatttac aggatttgaa 12840
   atgggctaga ttccctaaga gtgatggaac tggtactatc tatacagaac tggaaccacc 12900
   ttgtaggttt gttacagaca cacctaaagg tcctaaagtg aagtatttat actttattaa 12960
   aggattaaac aacctaaata gaggtatggt acttggtagt ttagctgcca cagtacgtct 13020
   acaagctggt aatgcaacag aagtgcctgc caattcaact gtattatctt tctgtgcttt 13080
   tgctgtagat gctgctaaag cttacaaaga ttatctagct agtgggggac aaccaatcac 13140
   taattgtgtt aagatgttgt gtacacacac tggtactggt caggcaataa cagttacacc 13200
   ggaagccaat atggatcaag aatcctttgg tggtgcatcg tgttgtctgt actgccgttg 13260
   ccacatagat catccaaatc ctaaaggatt ttgtgactta aaaggtaagt atgtacaaat 13320
   acctacaact tgtgctaatg accctgtggg ttttacactt aaaaacacag tctgtaccgt 13380
   ctgcggtatg tggaaaggtt atggctgtag ttgtgatcaa ctccgcgaac ccatgcttca 13440
   gtcagctgat gcacaatcgt ttttaaacgg gtttgcggtg taagtgcagc ccgtcttaca 13500
   ccgtgcggca caggcactag tactgatgtc gtatacaggg cttttgacat ctacaatgat 13560
   aaagtagctg gttttgctaa attcctaaaa actaattgtt gtcgcttcca agaaaaggac 13620
   gaagatgaca atttaattga ttcttacttt gtagttaaga gacacacttt ctctaactac 13680
   caacatgaag aaacaattta taatttactt aaggattgtc cagctgttgc taaacatgac 13740
   ttctttaagt ttagaataga cggtgacatg gtaccacata tatcacgtca acgtcttact 13800
   aaatacacaa tggcagacct cgtctatgct ttaaggcatt ttgatgaagg taattgtgac 13860
   acattaaaag aaatacttgt cacatacaat tgttgtgatg atgattattt caataaaaag 13920
   gactggtatg attttgtaga aaacccagat atattacgcg tatacgccaa cttaggtgaa 13980
   cgtgtacgcc aagctttgtt aaaaacagta caattctgtg atgccatgcg aaatgctggt 14040
   attgttggtg tactgacatt agataatcaa gatctcaatg gtaactggta tgatttcggt 14100
   gatttcatac aaaccacgcc aggtagtgga gttcctgttg tagattctta ttattcattg 14160
   ttaatgccta tattaacctt gaccagggct ttaactgcag agtcacatgt tgacactgac 14220
   ttaacaaagc cttacattaa gtgggatttg ttaaaatatg acttcacgga agagaggtta 14280
   aaactctttg accgttattt taaatattgg gatcagacat accacccaaa ttgtgttaac 14340
   tgtttggatg acagatgcat tctgcattgt gcaaacttta atgttttatt ctctacagtg 14400
   ttcccaccta caagttttgg accactagtg agaaaaatat ttgttgatgg tgttccattt 14460
   gtagtttcaa ctggatacca cttcagagag ctaggtgttg tacataatca ggatgtaaac 14520
   ttacatagct ctagacttag ttttaaggaa ttacttgtgt atgctgctga ccctgctatg 14580
   cacgctgctt ctggtaatct attactagat aaacgcacta cgtgcttttc agtagctgca 14640
   cttactaaca atgttgcttt tcaaactgtc aaacccggta attttaacaa agacttctat 14700
   gactttgctg tgtctaaggg tttctttaag gaaggaagtt ctgttgaatt aaaacacttc 14760
   ttctttgctc aggatggtaa tgctgctatc agcgattatg actactatcg ttataatcta 14820
   ccaacaatgt gtgatatcag acaactacta tttgtagttg aagttgttga taagtacttt 14880
   gattgttacg atggtggctg tattaatgct aaccaagtca tcgtcaacaa cctagacaaa 14940
   tcagctggtt ttccatttaa taaatggggt aaggctagac tttattatga ttcaatgagt 15000
   tatgaggatc aagatgcact tttcgcatat acaaaacgta atgtcatccc tactataact 15060
   caaatgaatc ttaagtatgc cattagtgca aagaatagag ctcgcaccgt agctggtgtc 15120
   tctatctgta gtactatgac caatagacag tttcatcaaa aattattgaa atcaatagcc 15180
   gccactagag gagctactgt agtaattgga acaagcaaat tctatggtgg ttggcacaac 15240
   atgttaaaaa ctgtttatag tgatgtagaa aaccctcacc ttatgggttg ggattatcct 15300
   aaatgtgata gagccatgcc taacatgctt agaattatgg cctcacttgt tcttgctcgc 15360
   aaacatacaa cgtgttgtag cttgtcacac cgtttctata gattagctaa tgagtgtgct 15420
   caagtattga gtgaaatggt catgtgtggc ggttcactat atgttaaacc aggtggaacc 15480
   tcatcaggag atgccacaac tgcttatgct aatagtgttt ttaacatttg tcaagctgtc 15540
   acggccaatg ttaatgcact tttatctact gatggtaaca aaattgccga taagtatgtc 15600
   cgcaatttac aacacagact ttatgagtgt ctctatagaa atagagatgt tgacacagac 15660
   tttgtgaatg agttttacgc atatttgcgt aaacatttct caatgatgat actctctgac 15720
   gatgctgttg tgtgtttcaa tagcacttat gcatctcaag gtctagtggc tagcataaag 15780
   aactttaagt cagttcttta ttatcaaaac aatgttttta tgtctgaagc aaaatgttgg 15840
   actgagactg accttactaa aggacctcat gaattttgct ctcaacatac aatgctagtt 15900
   aaacagggtg atgattatgt gtaccttcct tacccagatc catcaagaat cctaggggcc 15960
   ggctgttttg tagatgatat cgtaaaaaca gatggtacac ttatgattga acggttcgtg 16020
   tctttagcta tagatgctta cccacttact aaacatccta atcaggagta tgctgatgtc 16080
   tttcatttgt acttacaata cataagaaag ctacatgatg agttaacagg acacatgtta 16140
   gacatgtatt ctgttatgct tactaatgat aacacttcaa ggtattggga acctgagttt 16200
   tatgaggcta tgtacacacc gcatacagtc ttacaggctg ttggggcttg tgttctttgc 16260
   aattcacaga cttcattaag atgtggtgct tgcatacgta gaccattctt atgttgtaaa 16320
   tgctgttacg accatgtcat atcaacatca cataaattag tcttgtctgt taatccgtat 16380
   gtttgcaatg ctccaggttg tgatgtcaca gatgtgactc aactttactt aggaggtatg 16440
   agctattatt gtaaatcaca taaaccaccc attagttttc cattgtgtgc taatggacaa 16500
   gtttttggtt tatataaaaa tacatgtgtt ggtagcgata atgttactga ctttaatgca 16560
   attgcaacat gtgactggac aaatgctggt gattacattt tagctaacac ctgtactgaa 16620
   agactcaagc tttttgcagc agaaacgctc aaagctactg aggagacatt taaactgtct 16680
   tatggtattg ctactgtacg tgaagtgctg tctgacagag aattacatct ttcatgggaa 16740
   gttggtaaac ctagaccacc acttaaccga aattatgtct ttactggtta tcgtgtaact 16800
   aaaaacagta aagtacaaat aggagagtac acctttgaaa aaggtgacta tggtgatgct 16860
   gttgtttacc gaggtacaac aacttacaaa ttaaatgttg gtgattattt tgtgctgaca 16920
   tcacatacag taatgccatt aagtgcacct acactagtgc cacaagagca ctatgttaga 16980
   attactggct tatacccaac actcaatatc tcagatgagt tttctagcaa tgttgcaaat 17040
   tatcaaaagg ttggtatgca aaagtattct acactccagg gaccacctgg tactggtaag 17100
   agtcattttg ctattggcct agctctctac tacccttctg ctcgcatagt gtatacagct 17160
   tgctctcatg ccgctgttga tgcactatgt gagaaggcat taaaatattt gcctatagat 17220
   aaatgtagta gaattatacc tgcacgtgct cgtgtagagt gttttgataa attcaaagtg 17280
   aattcaacat tagaacagta tgtcttttgt actgtaaatg cattgcctga gacgacagca 17340
   gatatagttg tctttgatga aatttcaatg gccacaaatt atgatttgag tgttgtcaat 17400
   gccagattac gtgctaagca ctatgtgtac attggcgacc ctgctcaatt acctgcacca 17460
   cgcacattgc taactaaggg cacactagaa ccagaatatt tcaattcagt gtgtagactt 17520
   atgaaaacta taggtccaga catgttcctc ggaacttgtc ggcgttgtcc tgctgaaatt 17580
   gttgacactg tgagtgcttt ggtttatgat aataagctta aagcacataa agacaaatca 17640
   gctcaatgct ttaaaatgtt ttataagggt gttatcacgc atgatgtttc atctgcaatt 17700
   aacaggccac aaataggcgt ggtaagagaa ttccttacac gtaaccctgc ttggagaaaa 17760
   gctgtcttta tttcacctta taattcacag aatgctgtag cctcaaagat tttgggacta 17820
   ccaactcaaa ctgttgattc atcacagggc tcagaatatg actatgtcat attcactcaa 17880
   accactgaaa cagctcactc ttgtaatgta aacagattta atgttgctat taccagagca 17940
   aaagtaggca tactttgcat aatgtctgat agagaccttt atgacaagtt gcaatttaca 18000
   agtcttgaaa ttccacgtag gaatgtggca actttacaag ctgaaaatgt aacaggactc 18060
   tttaaagatt gtagtaaggt aatcactggg ttacatccta cacaggcacc tacacacctc 18120
   agtgttgaca ctaaattcaa aactgaaggt ttatgtgttg acatacctgg catacctaag 18180
   gacatgacct atagaagact catctctatg atgggtttta aaatgaatta tcaagttaat 18240
   ggttacccta acatgtttat cacccgcgaa gaagctataa gacatgtacg tgcatggatt 18300
   ggcttcgatg tcgaggggtg tcatgctact agagaagctg ttggtaccaa tttaccttta 18360
   cagctaggtt tttctacagg tgttaaccta gttgctgtac ctacaggtta tgttgataca 18420
   cctaataata cagatttttc cagagttagt gctaaaccac cgcctggaga tcaatttaaa 18480
   cacctcatac cacttatgta caaaggactt ccttggaatg tagtgcgtat aaagattgta 18540
   caaatgttaa gtgacacact taaaaatctc tctgacagag tcgtatttgt cttatgggca 18600
   catggctttg agttgacatc tatgaagtat tttgtgaaaa taggacctga gcgcacctgt 18660
   tgtctatgtg atagacgtgc cacatgcttt tccactgctt cagacactta tgcctgttgg 18720
   catcattcta ttggatttga ttacgtctat aatccgttta tgattgatgt tcaacaatgg 18780
   ggttttacag gtaacctaca aagcaaccat gatctgtatt gtcaagtcca tggtaatgca 18840
   catgtagcta gttgtgatgc aatcatgact aggtgtctag ctgtccacga gtgctttgtt 18900
   aagcgtgttg actggactat tgaatatcct ataattggtg atgaactgaa gattaatgcg 18960
   gcttgtagaa aggttcaaca catggttgtt aaagctgcat tattagcaga caaattccca 19020
   gttcttcacg acattggtaa ccctaaagct attaagtgtg tacctcaagc tgatgtagaa 19080
   tggaagttct atgatgcaca gccttgtagt gacaaagctt ataaaataga agaattattc 19140
   tattcttatg ccacacattc tgacaaattc acagatggtg tatgcctatt ttggaattgc 19200
   aatgtcgata gatatcctgc taattccatt gtttgtagat ttgacactag agtgctatct 19260
   aaccttaact tgcctggttg tgatggtggc agtttgtatg taaataaaca tgcattccac 19320
   acaccagctt ttgataaaag tgcttttgtt aatttaaaac aattaccatt tttctattac 19380
   tctgacagtc catgtgagtc tcatggaaaa caagtagtgt cagatataga ttatgtacca 19440
   ctaaagtctg ctacgtgtat aacacgttgc aatttaggtg gtgctgtctg tagacatcat 19500
   gctaatgagt acagattgta tctcgatgct tataacatga tgatctcagc tggctttagc 19560
   ttgtgggttt acaaacaatt tgatacttat aacctctgga acacttttac aagacttcag 19620
   agtttagaaa atgtggcttt taatgttgta aataagggac actttgatgg acaacagggt 19680
   gaagtaccag tttctatcat taataacact gtttacacaa aagttgatgg tgttgatgta 19740
   gaattgtttg aaaataaaac aacattacct gttaatgtag catttgagct ttgggctaag 19800
   cgcaacatta aaccagtacc agaggtgaaa atactcaata atttgggtgt ggacattgct 19860
   gctaatactg tgatctggga ctacaaaaga gatgctccag cacatatatc tactattggt 19920
   gtttgttcta tgactgacat agccaagaaa ccaactgaaa cgatttgtgc accactcact 19980
   gtcttttttg atggtagagt tgatggtcaa gtagacttat ttagaaatgc ccgtaatggt 20040
   gttcttatta cagaaggtag tgttaaaggt ttacaaccat ctgtaggtcc caaacaagct 20100
   agtcttaatg gagtcacatt aattggagaa gccgtaaaaa cacagttcaa ttattataag 20160
   aaagttgatg gtgttgtcca acaattacct gaaacttact ttactcagag tagaaattta 20220
   caagaattta aacccaggag tcaaatggaa attgatttct tagaattagc tatggatgaa 20280
   ttcattgaac ggtataaatt agaaggctat gccttcgaac atatcgttta tggagatttt 20340
   agtcatagtc agttaggtgg tttacatcta ctgattggac tagctaaacg ttttaaggaa 20400
   tcaccttttg aattagaaga ttttattcct atggacagta cagttaaaaa ctatttcata 20460
   acagatgcgc aaacaggttc atctaagtgt gtgtgttctg ttattgattt attacttgat 20520
   gattttgttg aaataataaa atcccaagat ttatctgtag tttctaaggt tgtcaaagtg 20580
   actattgact atacagaaat ttcatttatg ctttggtgta aagatggcca tgtagaaaca 20640
   ttttacccaa aattacaatc tagtcaagcg tggcaaccgg gtgttgctat gcctaatctt 20700
   tacaaaatgc aaagaatgct attagaaaag tgtgaccttc aaaattatgg tgatagtgca 20760
   acattaccta aaggcataat gatgaatgtc gcaaaatata ctcaactgtg tcaatattta 20820
   aacacattaa cattagctgt accctataat atgagagtta tacattttgg tgctggttct 20880
   gataaaggag ttgcaccagg tacagctgtt ttaagacagt ggttgcctac gggtacgctg 20940
   cttgtcgatt cagatcttaa tgactttgtc tctgatgcag attcaacttt gattggtgat 21000
   tgtgcaactg tacatacagc taataaatgg gatctcatta ttagtgatat gtacgaccct 21060
   aagactaaaa atgttacaaa agaaaatgac tctaaagagg gttttttcac ttacatttgt 21120
   gggtttatac aacaaaagct agctcttgga ggttccgtgg ctataaagat aacagaacat 21180
   tcttggaatg ctgatcttta taagctcatg ggacacttcg catggtggac agcctttgtt 21240
   actaatgtga atgcgtcatc atctgaagca tttttaattg gatgtaatta tcttggcaaa 21300
   ccacgcgaac aaatagatgg ttatgtcatg catgcaaatt acatattttg gaggaataca 21360
   aatccaattc agttgtcttc ctattcttta tttgacatga gtaaatttcc ccttaaatta 21420
   aggggtactg ctgttatgtc tttaaaagaa ggtcaaatca atgatatgat tttatctctt 21480
   cttagtaaag gtagacttat aattagagaa aacaacagag ttgttatttc tagtgatgtt 21540
   cttgttaaca actaaacgaa caatgtttgt ttttcttgtt ttattgccac tagtctctag 21600
   tcagtgtgtt aatcttacaa ccagaactca attaccccct gcatacacta attctttcac 21660
   acgtggtgtt tattaccctg acaaagtttt cagatcctca gttttacatt caactcagga 21720
   cttgttctta cctttctttt ccaatgttac ttggttccat gctatacatg tctctgggac 21780
   caatggtact aagaggtttg ataaccctgt cctaccattt aatgatggtg tttattttgc 21840
   ttccactgag aagtctaaca taataagagg ctggattttt ggtactactt tagattcgaa 21900
   gacccagtcc ctacttattg ttaataacgc tactaatgtt gttattaaag tctgtgaatt 21960
   tcaattttgt aatgatccat ttttgggtgt ttattaccac aaaaacaaca aaagttggat 22020
   ggaaagtgag ttcagagttt attctagtgc gaataattgc acttttgaat atgtctctca 22080
   gccttttctt atggaccttg aaggaaaaca gggtaatttc aaaaatctta gggaatttgt 22140
   gtttaagaat attgatggtt attttaaaat atattctaag cacacgccta ttaatttagt 22200
   gcgtgatctc cctcagggtt tttcggcttt agaaccattg gtagatttgc caataggtat 22260
   taacatcact aggtttcaaa ctttacttgc tttacataga agttatttga ctcctggtga 22320
   ttcttcttca ggttggacag ctggtgctgc agcttattat gtgggttatc ttcaacctag 22380
   gacttttcta ttaaaatata atgaaaatgg aaccattaca gatgctgtag actgtgcact 22440
   tgaccctctc tcagaaacaa agtgtacgtt gaaatccttc actgtagaaa aaggaatcta 22500
   tcaaacttct aactttagag tccaaccaac agaatctatt gttagatttc ctaatattac 22560
   aaacttgtgc ccttttggtg aagtttttaa cgccaccaga tttgcatctg tttatgcttg 22620
   gaacaggaag agaatcagca actgtgttgc tgattattct gtcctatata attccgcatc 22680
   attttccact tttaagtgtt atggagtgtc tcctactaaa ttaaatgatc tctgctttac 22740
   taatgtctat gcagattcat ttgtaattag aggtgatgaa gtcagacaaa tcgctccagg 22800
   gcaaactgga aagattgctg attataatta taaattacca gatgatttta caggctgcgt 22860
   tatagcttgg aattctaaca atcttgattc taaggttggt ggtaattata attacctgta 22920
   tagattgttt aggaagtcta atctcaaacc ttttgagaga gatatttcaa ctgaaatcta 22980
   tcaggccggt agcacacctt gtaatggtgt tgaaggtttt aattgttact ttcctttaca 23040
   atcatatggt ttccaaccca ctaatggtgt tggttaccaa ccatacagag tagtagtact 23100
   ttcttttgaa cttctacatg caccagcaac tgtttgtgga cctaaaaagt ctactaattt 23160
   ggttaaaaac aaatgtgtca atttcaactt caatggttta acaggcacag gtgttcttac 23220
   tgagtctaac aaaaagtttc tgcctttcca acaatttggc agagacattg ctgacactac 23280
   tgatgctgtc cgtgatccac agacacttga gattcttgac attacaccat gttcttttgg 23340
   tggtgtcagt gttataacac caggaacaaa tacttctaac caggttgctg ttctttatca 23400
   ggatgttaac tgcacagaag tccctgttgc tattcatgca gatcaactta ctcctacttg 23460
   gcgtgtttat tctacaggtt ctaatgtttt tcaaacacgt gcaggctgtt taataggggc 23520
   tgaacatgtc aacaactcat atgagtgtga catacccatt ggtgcaggta tatgcgctag 23580
   ttatcagact cagactaatt ctcctcggcg ggcacgtagt gtagctagtc aatccatcat 23640
   tgcctacact atgtcacttg gtgcagaaaa ttcagttgct tactctaata actctattgc 23700
   catacccaca aattttacta ttagtgttac cacagaaatt ctaccagtgt ctatgaccaa 23760
   gacatcagta gattgtacaa tgtacatttg tggtgattca actgaatgca gcaatctttt 23820
   gttgcaatat ggcagttttt gtacacaatt aaaccgtgct ttaactggaa tagctgttga 23880
   acaagacaaa aacacccaag aagtttttgc acaagtcaaa caaatttaca aaacaccacc 23940
   aattaaagat tttggtggtt ttaatttttc acaaatatta ccagatccat caaaaccaag 24000
   caagaggtca tttattgaag atctactttt caacaaagtg acacttgcag atgctggctt 24060
   catcaaacaa tatggtgatt gccttggtga tattgctgct agagacctca tttgtgcaca 24120
   aaagtttaac ggccttactg ttttgccacc tttgctcaca gatgaaatga ttgctcaata 24180
   cacttctgca ctgttagcgg gtacaatcac ttctggttgg acctttggtg caggtgctgc 24240
   attacaaata ccatttgcta tgcaaatggc ttataggttt aatggtattg gagttacaca 24300
   gaatgttctc tatgagaacc aaaaattgat tgccaaccaa tttaatagtg ctattggcaa 24360
   aattcaagac tcactttctt ccacagcaag tgcacttgga aaacttcaag atgtggtcaa 24420
   ccaaaatgca caagctttaa acacgcttgt taaacaactt agctccaatt ttggtgcaat 24480
   ttcaagtgtt ttaaatgata tcctttcacg tcttgacaaa gttgaggctg aagtgcaaat 24540
   tgataggttg atcacaggca gacttcaaag tttgcagaca tatgtgactc aacaattaat 24600
   tagagctgca gaaatcagag cttctgctaa tcttgctgct actaaaatgt cagagtgtgt 24660
   acttggacaa tcaaaaagag ttgatttttg tggaaagggc tatcatctta tgtccttccc 24720
   tcagtcagca cctcatggtg tagtcttctt gcatgtgact tatgtccctg cacaagaaaa 24780
   gaacttcaca actgctcctg ccatttgtca tgatggaaaa gcacactttc ctcgtgaagg 24840
   tgtctttgtt tcaaatggca cacactggtt tgtaacacaa aggaattttt atgaaccaca 24900
   aatcattact acagacaaca catttgtgtc tggtaactgt gatgttgtaa taggaattgt 24960
   caacaacaca gtttatgatc ctttgcaacc tgaattagac tcattcaagg aggagttaga 25020
   taaatatttt aagaatcata catcaccaga tgttgattta ggtgacatct ctggcattaa 25080
   tgcttcagtt gtaaacattc aaaaagaaat tgaccgcctc aatgaggttg ccaagaattt 25140
   aaatgaatct ctcatcgatc tccaagaact tggaaagtat gagcagtata taaaatggcc 25200
   atggtacatt tggctaggtt ttatagctgg cttgattgcc atagtaatgg tgacaattat 25260
   gctttgctgt atgaccagtt gctgtagttg tctcaagggc tgttgttctt gtggatcctg 25320
   ctgcaaattt gatgaagacg actctgagcc agtgctcaaa ggagtcaaat tacattacac 25380
   ataaacgaac ttatggattt gtttatgaga atcttcacaa ttggaactgt aactttgaag 25440
   caaggtgaaa tcaaggatgc tactccttca gattttgttc gcgctactgc aacgataccg 25500
   atacaagcct cactcccttt cggatggctt attgttggcg ttgcacttct tgctgttttt 25560
   cagagcgctt ccaaaatcat aaccctcaaa aagagatggc aactagcact ctccaagggt 25620
   gttcactttg tttgcaactt gctgttgttg tttgtaacag tttactcaca ccttttgctc 25680
   gttgctgctg gccttgaagc cccttttctc tatctttatg ctttagtcta cttcttgcag 25740
   agtataaact ttgtaagaat aataatgagg ctttggcttt gctggaaatg ccgttccaaa 25800
   aacccattac tttatgatgc caactatttt ctttgctggc atactaattg ttacgactat 25860
   tgtatacctt acaatagtgt aacttcttca attgtcatta cttcaggtga tggcacaaca 25920
   agtcctattt ctgaacatga ctaccagatt ggtggttata ctgaaaaatg ggaatctgga 25980
   gtaaaagact gtgttgtatt acacagttac ttcacttcag actattacca gctgtactca 26040
   actcaattga gtacagacac tggtgttgaa catgttacct tcttcatcta caataaaatt 26100
   gttgatgagc ctgaagaaca tgtccaaatt cacacaatcg acggttcatc cggagttgtt 26160
   aatccagtaa tggaaccaat ttatgatgaa ccgacgacga ctactagcgt gcctttgtaa 26220
   gcacaagctg atgagtacga acttatgtac tcattcgttt cggaagagac aggtacgtta 26280
   atagttaata gcgtacttct ttttcttgct ttcgtggtat tcttgctagt tacactagcc 26340
   atccttactg cgcttcgatt gtgtgcgtac tgctgcaata ttgttaacgt gagtcttgta 26400
   aaaccttctt tttacgttta ctctcgtgtt aaaaatctga attcttctag agttcctgat 26460
   cttctggtct aaacgaacta aatattatat tagtttttct gtttggaact ttaattttag 26520
   ccatggcaga ttccaacggt actattaccg ttgaagagct taaaaagctc cttgaacaat 26580
   ggaacctagt aataggtttc ctattcctta catggatttg tcttctacaa tttgcctatg 26640
   ccaacaggaa taggtttttg tatataatta agttaatttt cctctggctg ttatggccag 26700
   taactttagc ttgttttgtg cttgctgctg tttacagaat aaattggatc accggtggaa 26760
   ttgctatcgc aatggcttgt cttgtaggct tgatgtggct cagctacttc attgcttctt 26820
   tcagactgtt tgcgcgtacg cgttccatgt ggtcattcaa tccagaaact aacattcttc 26880
   tcaacgtgcc actccatggc actattctga ccagaccgct tctagaaagt gaactcgtaa 26940
   tcggagctgt gatccttcgt ggacatcttc gtattgctgg acaccatcta ggacgctgtg 27000
   acatcaagga cctgcctaaa gaaatcactg ttgctacatc acgaacgctt tcttattaca 27060
   aattgggagc ttcgcagcgt gtagcaggtg actcaggttt tgctgcatac agtcgctaca 27120
   ggattggcaa ctataaatta aacacagacc attccagtag cagtgacaat attgctttgc 27180
   ttgtacagta agtgacaaca gatgtttcat ctcgttgact ttcaggttac tatagcagag 27240
   atattactaa ttattatgag gacttttaaa gtttccattt ggaatcttga ttacatcata 27300
   aacctcataa ttaaaaattt atctaagtca ctaactgaga ataaatattc tcaattagat 27360
   gaagagcaac caatggagat tgattaaacg aacatgaaaa ttattctttt cttggcactg 27420
   ataacactcg ctacttgtga gctttatcac taccaagagt gtgttagagg tacaacagta 27480
   cttttaaaag aaccttgctc ttctggaaca tacgaggcaa attcaccatt tcatcctcta 27540
   gctgataaca aatttgcact gacttgcttt agcactcaat ttgcttttgc ttgtcctgac 27600
   ggcgtaaaac acgtctatca gttacgtgcc agatcagttt cacctaaact gttcatcaga 27660
   caagaggaag ttcaagaact ttactctcca atttttctta ttgttgcggc aatagtgttt 27720
   ataacacttt gcttcacact caaaagaaag acagaatgat tgaactttca ttaattgact 27780
   tctatttgtg ctttttagcc tttctgctat tccttgtttt aattatgctt attatctttt 27840
   ggttctcact tgaactgcaa gatcataatg aaacttgtca cgcctaaacg aacatgaaat 27900
   ttcttgtttt cttaggaatc atcacaactg tagctgcatt tcaccaagaa tgtagtttac 27960
   agtcatgtac tcaacatcaa ccatatgtag ttgatgaccc gtgtcctatt cacttctatt 28020
   ctaaatggta tattagagta ggagctagaa aatcagcacc tttaattgaa ttgtgcgtgg 28080
   atgaggctgg ttctaaatca cccattcagt acatcgatat cggtaattat acagtttcct 28140
   gtttaccttt tacaattaat tgccaggaac ctaaattggg tagtcttgta gtgcgttgtt 28200
   cgttctatga agacttttta gagtatcatg acgttcgtgt tgttttagat ttcatctaaa 28260
   cgaacaaact aaaatgtctg ataatggacc ccaaaatcag cgaaatgcac cccgcattac 28320
   gtttggtgga ccctcagatt caactggcag taaccagaat ggagaacgca gtggggcgcg 28380
   atcaaaacaa cgtcggcccc aaggtttacc caataatact gcgtcttggt tcaccgctct 28440
   cactcaacat ggcaaggaag accttaaatt ccctcgagga caaggcgttc caattaacac 28500
   caatagcagt ccagatgacc aaattggcta ctaccgaaga gctaccagac gaattcgtgg 28560
   tggtgacggt aaaatgaaag atctcagtcc aagatggtat ttctactacc taggaactgg 28620
   gccagaagct ggacttccct atggtgctaa caaagacggc atcatatggg ttgcaactga 28680
   gggagccttg aatacaccaa aagatcacat tggcacccgc aatcctgcta acaatgctgc 28740
   aatcgtgcta caacttcctc aaggaacaac attgccaaaa ggcttctacg cagaagggag 28800
   cagaggcggc agtcaagcct cttctcgttc ctcatcacgt agtcgcaaca gttcaagaaa 28860
   ttcaactcca ggcagcagta ggggaacttc tcctgctaga atggctggca atggcggtga 28920
   tgctgctctt gctttgctgc tgcttgacag attgaaccag cttgagagca aaatgtctgg 28980
   taaaggccaa caacaacaag gccaaactgt cactaagaaa tctgctgctg aggcttctaa 29040
   gaagcctcgg caaaaacgta ctgccactaa agcatacaat gtaacacaag ctttcggcag 29100
   acgtggtcca gaacaaaccc aaggaaattt tggggaccag gaactaatca gacaaggaac 29160
   tgattacaaa cattggccgc aaattgcaca atttgccccc agcgcttcag cgttcttcgg 29220
   aatgtcgcgc attggcatgg aagtcacacc ttcgggaacg tggttgacct acacaggtgc 29280
   catcaaattg gatgacaaag atccaaattt caaagatcaa gtcattttgc tgaataagca 29340
   tattgacgca tacaaaacat tcccaccaac agagcctaaa aaggacaaaa agaagaaggc 29400
   tgatgaaact caagccttac cgcagagaca gaagaaacag caaactgtga ctcttcttcc 29460
   tgctgcagat ttggatgatt tctccaaaca attgcaacaa tccatgagca gtgctgactc 29520
   aactcaggcc taaactcatg cagaccacac aaggcagatg ggctatataa acgttttcgc 29580
   ttttccgttt acgatatata gtctactctt gtgcagaatg aattctcgta actacatagc 29640
   acaagtagat gtagttaact ttaatctcac atagcaatct ttaatcagtg tgtaacatta 29700
   gggaggactt gaaagagcca ccacattttc accgaggcca cgcggagtac gatcgagtgt 29760
   acagtgaaca atgctaggga gagctgccta tatggaagag ccctaatgtg taaaattaat 29820
   tttagtagtg ctatccccat gtgattttaa tagcttctta ggagaatgac aaaaaaaaaa 29880
   aaaaaaaaaa aaaaaaaaaa aaa 29903